# EUROPEAN PATENT APPLICATION

(11) **EP 4 488 292 A1**
(43) Date of publication of application: **08.01.2025**
(21) Application number: 23760185.1
(22) Date of filing: 27.02.2023
(51) Int. Cl.: C07K 16/28, A61K 31/713, A61K 35/12, A61K 38/02, A61K 39/395, A61K 45/00, A61K 48/00, A61P 25/00, A61P 25/28, A61P 37/04, A61P 43/00, C07K 14/705, C07K 16/46, C12N 1/15, C12N 1/19, C12N 1/21, C12N 5/10, C12N 15/06, C12N 15/13, C12N 15/63, C12P 21/08

(54) **THERAPEUTIC AGENT FOR NEURODEGENERATIVE DISORDER**

(30) Priority: 28.02.2022 JP 2022029825; 28.04.2022 JP 2022075095
(71) Applicant: Teijin Pharma Limited, Tokyo 100-0013 (JP); University Public Corporation Osaka, Osaka-shi, Osaka 545-0051 (JP)
(72) Inventor: EGUCHI, Hiroshi, Tokyo 100-0013 (JP); TOMIYAMA, Takami, Osaka-shi, Osaka 558-8585 (JP); UMEDA, Tomohiro, Osaka-shi, Osaka 558-8585 (JP)
(74) Representative: Carpmaels & Ransford LLP
(86) International application number: PCT/JP2023/007143
(87) International publication number: WO 2023/163187

(57) **Abstract**

The present invention provides: an anti-gpNMB antibody that binds to gpNMB and affects the same and that has an effect of such as removal of dysfunctional microglia; and a use application of the anti-gpNMB antibody. The anti-gpNMB antibody specifically binds to at least one site in a region from a PMEL-CAF-like domain to a PKD domain of gpNMB.

## Description

### TECHNICAL FIELD

The present invention relates to an anti-gpNMB antibody that has the functions of decreasing the number of mal-functional microglia in a subject and/or removing amyloid β oligomers in a subject and/or increasing the number of synapses in a subject and/or restoring cognitive functions, and a pharmaceutical drug comprising an ingredient that has such functions.

### BACKGROUND ART

### 1. Neurodegenerative diseases

Neurodegenerative diseases are progressive neurological disorders in which the accumulation of toxic proteins (proteins that cannot be degraded intracellularly due to multimerization or aggregate formation caused by structural changes) in nerve cells results in impaired neurotransmission and intracellular clearance functions of nerve cells, as well as nerve cell death, leading to cognitive decline, memory impairment, mental impairment, and motor function impairment. Neurodegenerative diseases have no fundamental therapies, and drugs that alleviate the symptoms associated with the diseases are used. Most of these "neurodegenerative diseases" are "central nervous system degenerative diseases."

Examples classified as neurodegenerative diseases include (1) tauopathies (diseases in which Tau accumulates and causes neurodegeneration): specifically, Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclear paralysis (PSP), globular glial tauopathy (GGT), argyrophilic grain dementia (AGD) (argyrophilic grain disease ), age-related Tau astroglial tauopathy (ARTAG), familial British dementia (FBD), familial Danish dementia (FDD), FTDP17, multisystem tauopathy with dementia (MSTD), neurofibrillary tangle dementia, neurofibrillary tangle with calcinosis (DNTC), and white matter tauopathy with globular glial inclusion (WMT-GGI); and (2) neurodegenerative diseases other than tauopathies: specifically, Parkinson's disease (accumulation of αSyn), Huntington's disease (accumulation of mutant huntingtin), spinal cord cerebellum degeneration (accumulation of polyglutamic acid protein), hereditary spinal cord cerebellar ataxia (accumulation of polyglutamic acid protein), spinobulbar muscular atrophy (accumulation of polyglutamic acid protein), Lewy body dementia (accumulation of αSyn), Creutzfeldt-Jakob disease (abnormal prion protein accumulation ), amyotrophic lateral sclerosis (ALS) (accumulation of SOD or TDP43), and frontotemporal lobar degeneration (accumulation of FTLD-TDP, FTLD-FUS:TDP43, or FUS).

### 2. Alzheimer's disease (Alzheimer-type dementia)

Dementia is recognized as a major problem in the global aging population. According to a study conducted by Alzheimer's Disease International, the number of patients worldwide with dementia is projected to increase to approximately 76 million by 2030 and then to 139 million by 2050.

According to the World Health Organization (WHO), about 60 to 70% of all dementias are reported to be Alzheimer's disease (https://www.who.int/news-room/fact-sheets/detail/dementia). Two major pathologies have been reported in Alzheimer's disease: senile plaques (amyloid deposition) and neurofibrillary changes (tau accumulation).

Because patients with familial Alzheimer's disease have genetic mutations that are associated with increased production of amyloid, research is being conducted on antibodies and vaccines against amyloid beta (Aβ), a component of amyloid. Various anti-Aβ antibodies that eliminate amyloid deposition (Non-Patent Literature 1) and drugs targeting Aβ oligomers (Non-Patent Literature 2), which are considered highly toxic, are already in clinical development, and Aducanumab (Non-Patent Literature 3) received conditional approval from the FDA in 2021. However, these drugs against amyloid are reported to exhibit only limited efficacies and also involve high risks of side effects.

Tau, another component of neurofibrillary tangles, is also attracting attention as a drug discovery target, as recent brain imaging analysis has confirmed a correlation between Tau accumulation and clinical symptoms and disease progression. Clinical trials of anti-Tau antibodies against various epitopes are currently underway (Non-Patent Literature 4).

Other techniques to efficiently transfer antibodies into the brain for the treatment of Alzheimer's disease includes antibodies against membrane proteins expressed in the blood-brain barrier (BBB), such as transferrin receptors, or antibodies that can efficiently pass through the BBB by adding peptides with affinity to the constant region or by introducing amino acid mutations (Non-Patent Literature 5).

However, there are currently no effective drugs for inhibiting or improving declined cognitive function, and the creation of effective Alzheimer's disease drugs is a pressing issue worldwide.

### 3. Microglia

Microglia belong to the glial cell family, which also includes astrocytes and oligodendrocytes. However, unlike astrocytes and oligodendrocytes, microglia originate from progenitor cells that develop in the embryonic yolk sac. Microglia are macrophage-like cells in the brain and are thought to act as immunocompetent cells in the central nervous system, for example, to remove toxic proteins such as amyloid-β and aggregated Tau in the brain. It has also been reported that microglia can act in a neuroprotective or reparative manner in the event of neurological disorders. Furthermore, microglia have been shown to play an important role in the maintenance of neural networks, such as synaptic pruning. On the other hand, activated microglia release inflammatory cytokines and induce inflammation in the brain. Recent advances in genetic analysis technology have led to reports of the existence of various microglial subtypes, and the relationship of each microglial subtype to diseases is an issue for future research.

With regard to the relationship between microglia and Alzheimer's pathology, it was reported that degenerated microglia are associated with tau accumulation in immunohistochemistry using Alzheimer's disease patient brains (Non-Patent Literature 6). There is also a report which identified a subtype of dysfunctional microglia in Alzheimer's disease by immunohistology (single cell histology) (Non-Patent Literature 7). However, it is unclear how dysfunctional microglia modify diseases. In addition, although dysfunctional microglia are generally identified by their morphology, some researchers refer to dysfunctional microglia-like microglia as "degenerating microglia" or "aging microglia" (Non-Patent Literature 8), and the meaning of these terms have not been necessarily settled.

Brain imaging analysis of Alzheimer's disease patients has also been conducted using ligands for TSPO (translocator protein), whose expression increases when microglia are activated, and it was reported that the signal detected by TSPO-PET (Positron Emission (TSPO-PET) is higher in patients with Alzheimer's disease (Non-Patent Literature 9) and correlates with tau and amyloid pathology (Non-Patent Literature 10). However, research on the specificity of these TSPO-PET ligands for activated microglia is still in its infancy (Non-Patent Literature 11), and no common view has yet been reached.

Recently, GWAS (Genome Wide Association Study) analysis of Alzheimer's disease (Non-Patent Literature 12) revealed that many disease-related genes are expressed in microglia. Therefore, research and development of therapeutic agents for Alzheimer's disease targeting microglia is underway. For example, humans with a loss of function (LOF) gene mutation (R47H), which causes loss of function of TREM2, are at increased risk of developing Alzheimer's disease (Non-Patent Literature 13). Currently, clinical trials are underway against anti-TREM2 agonist antibodies (Non-Patent Literature 14) and anti-CD33 inhibitory antibodies (Non-Patent Literature 15) that have the ability to activate microglia (NCT04592874, NCT03822208), with the hope that activating microglia will promote clearance of toxic proteins such as Aβ and tau as candidate antibody drugs for microglia targeting. On the other hand, anti-Sema4D antibodies (Non-Patent Literature 16), which inhibit activated microglia in terms of suppressing gliosis, are also in clinical trials (NCT04381468).

Recently, scRNAseq (single-cell RNA sequencing) analysis of microglia in mouse models of Alzheimer's disease has revealed the existence of microglial subtypes that appear in association with Alzheimer's pathology. Such microglial subtypes include DAM (Disease Associated Microglia, Non-Patent Literature 17), ARM (Activated Response Microglia, Non-Patent Literature 18), and MGnD (microglial neurodegenerative phenotype, Non-Patent Literature 19). However, it is still unclear how these disease-associated microglia modify Alzheimer's disease, i.e., how they exert their effects, since there is no way to specifically regulate these DAMs and other disease-associated microglia. Furthermore, it is unknown which of the disease-specific microglia subtypes that have been identified suppress the pathogenesis and which subtypes enhance the pathogenesis. It is also unclear which dysfunctional microglia are classified or included within which subtypes, since mRNA expression levels do not directly provide information on whether cells accumulate denatured proteins or not.

### 4. spNMB

gpNMB (Glycoprotein nonmetastatic melanoma protein B) (also known as Osteoactivin, DC-HIL, Non-Patent Literature 20) is a single transmembrane glycoprotein and has as many as 12 glycosylation sites in humans. gpNMB is normally expressed in intracellular organelles (endoplasmic reticulum, lysosomes, Golgi apparatus, melanosomes), etc. (Non-Patent Literature 21). However, gpNMB has also been reported to be expressed on the plasma membrane when overexpressed (Non-Patent Literature 22). It has also been reported that gpNMB is expressed in cancer cells (Non-Patent Literature 23), and that KLD (kringle-like domain) is important for cell growth and that intracellular ITIM motifs induce signaling (Non-Patent Literature 24).

gpNMB has a region homologous to the CAF (Core Amyloid Fragment) region of the family protein PMEL (Premelanosome Protein) (Non-Patent Literature 25) (hereinafter referred to as the amyloid core domain) and PKD (Polycystic Kidney Disease homologous) domain. The PKD domain has a β-sandwich structure formed by a β-sheet consisting of three strands and a β-sheet consisting of four strands that fold together. It was named the PKD domain because it is a characteristic structure possessed by polycystin-1, the gene responsible for polycystic kidney disease (Non-Patent Literature 26).

gpNMB is important for the function of phagosomes and lysosomes (Non-Patent Literature 27), as it has been reported that lysosome function is impaired in gpNMB-deficient mice (Non-Patent Literature 28). Abnormalities of the iris and the development of glaucoma have been reported in mice lacking gpNMB (Non-Patent Literature 29), and humans lacking gpNMB have been reported to have cutaneous amyloidosis (Non-Patent Literature 30).

It is known that gpNMB is cleaved by membrane proteases such as ADAM10 and released as soluble gpNMB (Non-Patent Literature 31), and soluble gpNMB has been reported to be involved in neuroprotection (Non-Patent Literature 32), anti-inflammation (Non-Patent Literature 21) and cell proliferation (Non-Patent Literature 33). It has also been reported that neural function was improved in transgenic mice overexpressing soluble gpNMB (extracellular region gpNMB) (Non-Patent Literature 34), and cognitive function was increased in an Alzheimer's disease model mouse (APP/PS1) when autophagy was enhanced by using lentiviral vectors to express gpNMB (Non-Patent Literature 35).

On the other hand, it has been reported that administration of a mouse gpNMB-specific vaccine peptide with some amino acid sequences of mouse gpNMB reduces aged vascular endothelial cells and fibroblasts, improves insulin resistance and atherosclerosis in a high-fat diet load model mouse, and extends survival in mice with accelerated aging. It has also been reported that antibody-dependent cellular cytotoxicity (ADCC) activity is important in this process, and gpNMB partial peptide sequences that are effective in removing senescent cells have been disclosed (Patent Literature 1, Non-Patent Literature 36). Likewise, it has been reported that administration of a polyclonal antibody against mouse gpNMB in a high-fat diet-loaded mouse model improved obesity and insulin resistance by inhibiting soluble gpNMB produced by the liver (Non-Patent Literature 37).

Glembatumumab (Patent Literature 2), an anti-human gpNMB-specific human antibody, is known as an anti-gpNMB antibody. Glembatumumab Vedotin, a conjugate of Glembatumumab and an anticancer drug, advanced to Phase II trials as a potential cancer drug, but its development was discontinued. Another anti-gpNMB antibody that binds to gpNMB expressed on the surface of cancer cells and treats cancer with immunotoxins has also been disclosed (Patent Literature 3). There is also a report on the acquisition of another anti-human gpNMB-specific antibody, which describes diagnostic and therapeutic applications against human-derived cancer cells (Non-Patent Literature 38). Anti-rat gpNMB-specific antibodies specific for activated rat microglia have also been reported (Non-Patent Literature 39). It has also been reported that anti-mouse DC-HIL specific antibodies put signals into the cells (Non-Patent Literature 40).

With regard to gpNMB expression in Alzheimer's disease (AD), immunohistochemistry and RNA expression analysis and cerebrospinal fluid concentrations have been reported. Regarding immunohistochemistry, accumulation of gpNMB-expressing microglia in ApoE-positive Aβ plaques was reported in the brains of AD patients (Non-Patent Literature 41).

Regarding RNA expression, scRNAseq analysis of AppNL-G-F mice showed that microglia subtypes were classified into Activated Response Microglia (ARM), which are tissue repair genes such as gpNMB, etc., and increased TREM2 production and decreased CD33 production have been reported (Non-Patent Literature 18). There has also been a report that gpNMB expression is upregulated in MGnD in 5XFAD, a mouse model of Alzheimer's disease, suggesting that it may have a neuroprotective role (Non-Patent Literature 19). On the other hand, it has been reported that scRNAseq analysis of Alzheimer's disease model mice showed the expression of gpNMB in mal-functional microglia (Non-Patent Literature 42). There has also been a report that snRNAseq (single-nucleus RNA sequencing) using Alzheimer's disease patient brain samples showed the expression of ApoE4, TREM2, ITGAX, GPNMB, FLT1, SPP1, etc., in activated microglia with phagocytic ability, which are thought to work to remove toxic proteins (Non-Patent Literature 43). The association of type 1 microglia with Alzheimer's disease and the possible two-fold function of gpNMBs have also been reported (Non-Patent Literature 44). However, many microglial subtypes are known to exist, as shown in Non-Patent Literature 18 and Non-Patent Literature 43.

Furthermore, it has been reported that gpNMB is involved in autophagy function, reduces Aβ deposition in AD model mice (APP-PS1), and improves cognitive function as demonstrated by Morris water maze test (Non-Patent Literature 44).

Regarding the relationship between soluble gpNMB concentrations in cerebrospinal fluid and Alzheimer's disease, some reports (Non-Patent Literature 19) indicate that the concentrations are increased in AD, while others (Non-Patent Literature 45) indicate that there is no relationship.

### 5. Alzheimer's disease model mouse

Various mouse models of Alzheimer's disease have been developed and are currently used in research (Non-Patent Literature 46). One representative model mouse is the 5XFAD mouse (Non-Patent Literature 47), which produces Aβ oligomers, which are considered the most toxic form of amyloid-β, and also accumulates Aβ oligomers in neurons. The APPosk mouse (Non-Patent Literature 48) is another model mouse that accumulates Aβ oligomers in neurons, like the 5XFAD model mouse, and has been reported to improve pathology, restore synapse number, and improve cognitive function after intranasal administration of rifampicin (Non-Patent Literature 49). However, there have been no reports of clearance of intracellularly accumulated Aβ oligomers by antibodies such as anti-Aβ antibodies. There have also been no reports of antibodies that showed recovery of the number of synapses in neurons, an indicator of neuronal function and neurotransmission.

Tg2576 is a Tg mouse that overexpresses the Swedish mutant (KM670/671NL) somatic gene of the APP (Amyloid precursor protein, isoform 695) gene. Tg2576 is a model mouse for AD that has been reported to exhibit amyloid plaques (senile plaques in the brain of Alzheimer's disease patients) at about 9 months of age (Non-Patent Literature 50), and is used for general purpose studies of amyloid pathology.

Tau264 is a Tg mouse expressing human Tau. Although it does not exhibit Tau pathology by itself, it has been reported that its crossbreeding with APPosk mice induces phosphorylated tau accumulation (Non-Patent Literature 51), and is considered a model mouse with pathology similar to human Alzheimer's disease in that it exhibits phosphorylation and accumulation of wild-type Tau without mutations.

### 6. Evaluation of nerve function

Recovery of neural functions such as spatial reference function and memory functions is generally evaluated in vivo using behavioral tests, such as the Morris water maze test, the Y-maze, and novel substance search.

In vitro evaluation of the number of neurons is commonly carried out by immunohistochemical staining such as NeuN staining, which is an indicator of the number of neurons, and synaptophysin staining, which is an indicator of the number of synapses.

In many mouse models of Alzheimer's disease, evaluation is performed in a state that does not lead to neuronal cell death, and therefore, evaluation of neural functions is carried out by analysis of the number of synapses using synaptophysin staining.

Many papers have reported a correlation between synaptophysin levels in vitro and neurological functions in vivo (Non-Patent Literature 53, Non-Patent Literature 54, and Non-Patent Literature 55), and quantification of synaptophysin protein by synaptophysin staining or other means can be used to assess recovery of neurological functions.

### LIST OF CITATIONS

### Patent Literature

[Patent Literature 1] WO2021/020047 A
[Patent Literature 2] JP6334496 A
[Patent Literature 3] WO2007/053718 A

### Non-Patent Literature

[Non-Patent Literature 1] Avgerinos et al., Ageing Res. Rev., (2021), 68:101339
[Non-Patent Literature 2] Tolar et al., Int. J. Mol. Sci. (2021), 22[12]:6355
[Non-Patent Literature 3] Jeremic et al., Ageing Res. Rev., (2021), 72:101496
[Non-Patent Literature 4] Ji et al., Drugs, (2021), 81[10]:1135-1152
[Non-Patent Literature 5] Pardridge et al., Pharmaceuticals (2022), 15[3], Internet <https://doi.org/10.3390/ph 15010003>
[Non-Patent Literature 6] Streit et al., Acta Neuropathol., (2009), 118[4]:475-85
[Non-Patent Literature 7] Swanson et al., Acta Neuropathol. Commun., (2020), 8:170
[Non-Patent Literature 8] Shahidehpour et al., Neurobiol. Aging., (2021), 99:19-27
[Non-Patent Literature 9] Malpetti et al., Brain, (2020), 143:1588-1602
[Non-Patent Literature 10] Dani et al., Brain, (2018), 141[9]:2740-2754
[Non-Patent Literature 11] Gouilly et al., Eur. J. Neurosci., (2022), doi: 10.1111/ejn. 15613
[Non-Patent Literature 12] Jansen et al., Nat. Genet., (2019), 51[3]:404-413
[Non-Patent Literature 13] Wang et al., Cell, (2015), 160[6]:1061-1071
[Non-Patent Literature 14] Wang et al., J. Exp. Med., (2020), .217[9]:e20200785
[Non-Patent Literature 15] Griciuc et al., Curr. Opin. Neurol., (2021), 34[2]:228-236
[Non-Patent Literature 16] Mao et al., Int. J. Mol. Sci., (2021), 22:9465
[Non-Patent Literature 17] Keren-Shaul et al., Cell, (2017), 169:1276-1290
[Non-Patent Literature 18] Frigerio et al., Cell Rep., (2019), .27[4]:1293-1306
[Non-Patent Literature 19] Huettenrauch et al., Acta Neuropathol. Commun., (2018), 6:108
[Non-Patent Literature 20] Shikano et al., J. Biol. Chem., (2001), 276[11]:8125-8134
[Non-Patent Literature 21] Ripoll et al., J. Immunol., (2007), 178:6557-6566
[Non-Patent Literature 22] Tse et al., Clin. Cancer Res., (2006), 12[4]:1373-1382
[Non-Patent Literature 23] Maric et al., OncoTargets Ther., (2013), 6:839-852
[Non-Patent Literature 24] Xie et al., Cancer Sci., (2019), 110[7]:2237-2246
[Non-Patent Literature 25] Hee et al., Scientific Reports, (2017), 7:44064
[Non-Patent Literature 26] Bycroft et al., EMBO J., (1999), 18[2]:297-305
[Non-Patent Literature 27] Li et al., FASEB J., (2010), 24[12]:4767-4781
[Non-Patent Literature 28] Robinet et al., Scientific Reports, (2021), 11:10249
[Non-Patent Literature 29] Anderson et al., Nat. Genet., (2002), 30:81-85
[Non-Patent Literature 30] Yang et al., Am. J. Hum. Genet., (2018), 102[2]:219-232
[Non-Patent Literature 31] Rose et al., PLoS One, (2010), 5[8]:e12093
[Non-Patent Literature 32] Nakano et al., Neuroscience, (2014), 277:123-131
[Non-Patent Literature 33] Wang et al., Cancer Sci.. (2021), 112:4187-4197
[Non-Patent Literature 34] Murata et al., J. Neurochem., (2015), 132:583-594
[Non-Patent Literature 35] Zhu et al., Neurosci. Lett., (2022), 767:136300
[Non-Patent Literature 36] Suda et al., Nat. Aging, (2021), 1:1117-1126
[Non-Patent Literature 37] Gong et al., Nat. Metab., (2019), 1[5]:570-583
[Non-Patent Literature 38] FBRI LLC, "ALZFORUM: NETWORKING FOR A CURE", "RESEARCH MODELS", [online], (1996), [Seached in 2022], Internet <https://www.alzforum.org/research-models/alzheimers-disease>
[Non-Patent Literature 39] Zhang et al., Monoclon. Antibodies Immunodiagn. Immunother., (2013), .32[4]:265-269
[Non-Patent Literature 40] Kawahara et al., Glia, (2016), 64[11]:1938-1961
[Non-Patent Literature 41] Chung et al., J. Immunol., (2019), 183[5]:5190-5198
[Non-Patent Literature 42] Satoh et al., Intractable Rare Dis. Res., (2019), 8[2]:120-128
[Non-Patent Literature 43] Krasemann et al., Immunity, (2017), 47:566-581
[Non-Patent Literature 44] Gerrits et al., Acta Neuropathol., (2021), 141:681-696
[Non-Patent Literature 45] Zhu et al., Neuroscience Letters, (2022), 767:136300
[Non-Patent Literature 46] Kawahara, Monthly "Cell," (2022), 54[2]:110-114
[Non-Patent Literature 47] Aichholzer et al., Alzheimers Res. Ther., (2021), 13:94
[Non-Patent Literature 48] Oakley et al., J. Neurosci., (2006), 26[40]:10129-10140
[Non-Patent Literature 49] Umeda et al., J. Neurosci. Res., (2011), 89:1031-1042
[Non-Patent Literature 50] Umeda et al., Front. Neurosci., (2021), 15:763476
[Non-Patent Literature 51] Hsiao et al., Science, (1996), 274[5284]:99-102
[Non-Patent Literature 52] Umeda et al., Acta Neuropathol., (2014), 127:685-698
[Non-Patent Literature 53] Shi et al., Front. Pharmacol., (2021), 12:794458
[Non-Patent Literature 54] Umeda et al., Front. Neurosci., (2021), 15:763476
[Non-Patent Literature 55] Hafez et al., Neuroscience, (2012), 223:465-472
[Non-Patent Literature 56] EMBL-EBI, "AlphaFold Protein Structure Database", "Transmembrane glycoprotein NMB", [online], (2021), [Seached in 2022], Internet <https://alphafold.ebi.ac.uk/entry/Q99P91 >
[Non-Patent Literature 57] Woollacott et al., J. Neuroinflammation, (2020), 17:234
[Non-Patent Literature 58] Kushwaha et al., Ann. Neurosci., (2018), 25:223-233
[Non-Patent Literature 59] Swanson et al., Acta Neuropathol. Commun., (2020), 8:170
[Non-Patent Literature 60] Quek et al., J. Neuroinflammation, (2022), 19:58
[Non-Patent Literature 61] Boi et al., Int. J. Mol. Sci., (2020), 21[22]:8535
[Non-Patent Literature 62] Ishijima et al., Science Progress, (2021), 104[4]:1-21
[Non-Patent Literature 63] Cai et al., Bioengineered, (2021), 12[2]:11390-11398
[Non-Patent Literature 64] Paasila et al., Brain Pathology, (2019), 29:726-740
[Non-Patent Literature 65] Hajj et al., J. Neuroinflammation, (2019), 16:87
[Non-Patent Literature 66] Stratoulias et al., EMBO J., (2019), 38:e101997
[Non-Patent Literature 67] Anderson et al., Pigment Cell Melanoma Res. (2013), 26[4]:470-486

### SUMMARY OF INVENTION

### PROBLEM TO BE SOLVED BY THE INVENTION

There are some drugs that improve symptoms of neurodegenerative diseases, but there have been no therapeutic drugs. On the other hand, scRNAseq and other methods have revealed the existence of disease-associated microglia that appear specifically in neurodegenerative diseases, but it is unclear as to which microglia are responsible for disease progression. It is also unclear as to whether the identification and specific elimination of the disease-causing mal-functional microglia would lead to therapeutic effects such as elimination of toxic proteins in neurons and restoration of neuronal function.

In this context, as described in the BACKGROUND ART section above, although gpNMB is one of the genes expressed in mal-functional microglia that are involved in autophagosome and lysosome function, it is unclear as to whether gpNMB-high microglia are involved in the pathogenesis of the disease or not, or even whether they are pathogenic or protective.

As mentioned above, the presence of disease-associated microglia has been reported in several mouse models of Alzheimer's disease. However, it was unclear as to how disease-associated microglia modulate the disease, since there was no way to specifically regulate disease-associated microglia.

Although it has been reported that gpNMBs expressed in disease-associated microglia act in a neuroprotective manner and that they are expressed in mal-functional microglia, as described above, there have been no reports of anti-gpNMB monoclonal antibodies that bind with high affinity under physiological conditions in vivo, which can be administered to animal models of diseases to evaluate their efficacy. Furthermore, there have been no report that a high-affinity anti-gpNMB monoclonal antibodies, which can act in the brain, was administered to a mouse model of Alzheimer's disease to analyze its effects.

gpNMB is a glycoprotein rich in glycans, with 12 sites of glycosylation in humans. Therefore, it is not easy to create monoclonal antibodies that can recognize gpNMBs expressed on cells, and as mentioned above, the clinical trials conducted so far were those for glembatumumab vedotin, a conjugate of glembatumumab and an anticancer drug, have only been conducted for cancer types that highly express gpNMBs (melanoma, breast cancer, etc.). Because glenvatumumab is a human gpNMB-specific antibody, it was not possible to evaluate its effect on endogenous gpNMB in mouse or rat disease models.

On the other hand, there have been reports of improvement of obesity and insulin resistance in high-fat diet-loaded mouse models by inhibition of liver-expressed soluble gpNMB with an anti-mouse gpNMB polyclonal antibody (Non-Patent Literature 38) and improvement of atherosclerosis and insulin resistance and prolongation of survival in accelerated aging model mice by removal of senescent cells with mouse gpNMB specific vaccine peptide (Patent Literature 35 and Non-Patent Literature 36).

With regard to the production of anti-gpNMB antibodies with mouse-human cross-reactivity for the purpose of developing a therapeutic agent for Alzheimer's disease, there have been no reports to date as mentioned above, since gpNMB has a very large number of glycosylation sites, as low a homology of as about 70% between mouse and human, and a complex conformation (Non-Patent Literature 56), rendering the attempt to obtain antibodies that recognize the conformation in vivo very challenging. Furthermore, because the modification of glycans differs depending on the cells and tissues in which they are expressed, it was assumed to be extremely difficult to obtain antibodies that bind specifically to gpNMBs expressed in microglia, and even more difficult to create anti-gpNMB antibodies that bind to gpNMBs expressed on the cell membrane and have mouse/human cross-reactivity.

The present invention has been made in view of the above issues, and an objective of the present invention is to provide a means to specifically regulate mal-functional microglia, and also to provide a pharmaceutical drug based on a new mechanism of action using such means.

### MEANS TO SOLVE THE PROBLEM

The present inventors had confirmed the presence of microglia with intracellular accumulation of Aβ, Tau, or α-synuclein in mouse models of Alzheimer's disease and Parkinson's disease.

There has also been a report of a method for scoring the level of degeneration (dysfunction) based on the ramified structure Non-Patent Literature 57), where these microglia had a reduced ramified structure in morphology and were evaluated as degenerated. In addition, accumulation of Lipofuscin, which is observed in senescent cells, was also observed. These findings suggest that these microglia are mal-functional microglia that are unable to degrade aggregation proteins. It has been proposed that such mal-functional microglia accumulating aggregate proteins lose their neuroprotective effects and phagocytosis, leading to neurodegenerative pathologies (Non-Patent Literature 58 and Non-Patent Literature 59).

The present inventors have also found that most of these mal-functional microglia are gpNMB-positive, i.e., cells that are double-stained by immunohistochemistry with anti-gpNMB and anti-Iba1 antibodies. For example, based on the immunohistological analysis of the brain of APPosk mice, the inventors found that gpNMB-positive cells with intracellular Aβ accumulation were anti-gpNMB and anti-Iba1 antibody-positive cells, i.e., gpNMB-high expressing microglia. The term "mal-functional microglia" herein refers to a cell that is double-stained by immunohistochemistry with anti-gpNMB and anti-Iba1 antibodies.

Although researchers do not always agree on what is meant by "mal-functional microglia" as mentioned above, the term "mal-functional microglia" as used herein refers to microglia that have at least one of the following characteristics.
(1) Their phagocytic and/or toxic proteolytic functions are reduced or abnormally enhanced.
(2) They release proinflammatory cytokines that may progress diseases (e.g., inflammation in the brain).
(3) They have reduced ramified structures compared to normal microglia.

Among these, the first characteristic (1) can be confirmed by the fact that a variety of aggregate proteins are known to be subject to phagocytosis, which vary by disease, e.g., Aβ, tau, alpha-synuclein, Lipofuscin, L-Ferritin, TDP43, SOD, polyglutamate proteins, and their accumulation is observed in the microglia (Non-Patent Literature 60 and Non-Patent Literature 61). Specifically, this characteristic can be identified by staining with Iba1, a marker for microglia, as well as by detection with antibodies against aggregating proteins (e.g., anti-Aβ or anti-tau antibodies) or by autofluorescence (lipofuscin). The second characteristic (2) can be confirmed by the detection of the cytokines concerned (Non-Patent Literature 62 and Non-Patent Literature 63). The third characteristic (3) can be confirmed by immunohistochemical staining (Non-Patent Literature 64) and morphological observation using electron microscopy (Non-Patent Literature 65) (Non-Patent Literature 66).

On the other hand, the term "normal microglia" used herein refers to microglia that do not have any of the aforementioned characteristics of mal-functional microglia and are working to maintain homeostasis in the body.

Through intensive investigations in view of the above background, the present inventors have succeeded in obtaining anti-gpNMB antibodies that specifically binds to at least one site in a region from a PMEL-CAF-like domain to a PKD domain of human gpNMB, and have found that these antibodies have unexpected functions, e.g., the effects of decreasing the number of mal-functional microglia in a subject and/or removing amyloid β oligomers in a subject and/or increasing the number of synapses in a subject and/or restoring cognitive functions, and can be used for a variety of applications based on these functions, e.g., for treating or preventing neurodegenerative disease in a subject. The present inventors have also found that using any means to reduce the number of dysfunctional microglia, such as antibodies makes it possible to achieve unexpected effects, e.g., the effects of decreasing the number of mal-functional microglia in a subject and/or removing amyloid β oligomers in a subject and/or increasing the number of synapses in a subject and/or restoring cognitive functions, and even the unexpected effect of treating or preventing various neurodegenerative diseases. Based on these findings, the inventors have completed the following inventions.

Specifically, aspects of the present invention include the following.
[Aspect 1] An anti-gpNMB antibody or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B).
[Aspect 2] The anti-gpNMB antibody or its fragment or a derivative thereof according to claim 1, which binds specifically to a region containing amino acid residue(s) D287 and/or H301 of human gpNMB having the amino acid sequence defined in SEQ ID NO 209.
[Aspect 3] The anti-gpNMB antibody or its fragment or a derivative thereof according to claim 1 or 2, which binds specifically to a region containing amino acid residue(s) R214 and/or R215 of human gpNMB having the amino acid sequence defined in SEQ ID NO 209.
[Aspect 4] The anti-gpNMB antibody or its fragment or a derivative thereof according to claim 2 or 3, which also binds specifically to one or more regions selected from of a region containing amino acid residue(s) K257 and/or D258, a region containing amino acid residue(s) H268 and/or D269, a region containing an amino acid residue K282, a region containing an amino acid residue K316, and a region containing amino acid residue(s) H216 and/or R218, of human gpNMB having the amino acid sequence defined in SEQ ID NO 209.
[Aspect 5] The anti-gpNMB antibody or its fragment or a derivative thereof according to any one of claims 1 to 4, which also binds specifically to at least one site in a region from a PMEL-CAF-like domain to a PKD domain of mouse gpNMB.
[Aspect 6] The anti-gpNMB antibody or its fragment or a derivative thereof according to any one of claims 1 to 5, having one or more activities selected from: an activity to reduce the number of mal-functional microglia; an activity to remove amyloid beta oligomers; an activity to increase the number of synapses; and an activity to restore cognitive function.
[Aspect 7] The anti-gpNMB antibody or its fragment or a derivative thereof according to any one of claims 1 to 6, which is a monoclonal antibody or its fragment or a derivative thereof.
[Aspect 8] The anti-gpNMB antibody or its fragment or a derivative thereof according to any one of claims 1 to 7, which comprises at least a heavy chain variable region comprising:
   (1) as a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 1,
      as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 3, and
      as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably identity) of 66.6 % or higher, 75.0 % or higher, 83.3 % or higher, or 91.6 % or higher to SEQ ID NO 5, or
   (2) as a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 17,
      as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 19, and
      as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 21, or
   (3) as a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 33,
      as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 35, and
      as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 37, or
   (4) as a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 49,
      as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 51, and
      as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably identity) of 62.5 % or higher, 68.7 % or higher, 75.0 % or higher, 81.2 % or higher, 87.5 % or higher, or 93.7 % or higher to SEQ ID NO 53, or
   (5) as a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 65,
      as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 67, and
      as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably identity) of 64.2 % or higher, 71.4 % or higher, 78.5 % or higher, 85.7 % or higher, or 92.8 % or higher to SEQ ID NO 69, or
   (6) as a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 81,
      as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably identity) of 71.4 % or higher or 85.7 % or higher to SEQ ID NO 83, and
      as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 85, or
   (7) as a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 97,
      as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 99, and
      as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably identity) of 60.0 % or higher or 80.0 % or higher to SEQ ID NO 101, or
   (8) as a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 113,
      as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably identity) of 71.4 % or higher or 85.7 % or higher to SEQ ID NO 115, and
      as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 117, or
   (9) as a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 129,
      as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably identity) of 71.4 % or higher or 85.7 % or higher to SEQ ID NO 131, and
      as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 133, or
   (10) as a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 145,
      as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 147, and
      as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 149, or
   (11) as a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 161,
      as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably identity) of 71.4 % or higher or 85.7 % or higher to SEQ ID NO 163, and
      as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 165,
   (12) as a CDR-H1 sequence, the amino acid sequence defined in SEQ ID NO 287, or an amino acid sequence derived from SEQ ID NO 287 via substitution, deletion, or insertion of any one, two, or three amino acid residues, or an amino acid sequence having a homology (preferably identity) of 62.5 % or higher, 75.0 % or higher, 87.5 % or higher, or 90.0 % or higher to SEQ ID NO 287,
      as a CDR-H2 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably identity) of 71.4 % or higher or 85.7 % or higher to SEQ ID NO 289, and
      as a CDR-H3 sequence, the amino acid sequence defined in SEQ ID NO 291, or an amino acid sequence derived from SEQ ID NO 291 via substitution, deletion, or insertion of any one or two amino acid residues, or an amino acid sequence having a homology (preferably identity) of 66.6 % or higher or 83.3 % or higher to SEQ ID NO 291, or
   (13) as a CDR-H1 sequence, the amino acid sequence defined in SEQ ID NO 17, or an amino acid sequence derived from SEQ ID NO 17 via substitution, deletion, or insertion of
      any one, two, three, four, five, or six amino acid residues other than asparagine at position 32 and tryptophan at position 33,
      as a CDR-H2 sequence, the amino acid sequence defined in SEQ ID NO 19, or an amino acid sequence derived from SEQ ID NO 19 via substitution, deletion, or insertion of any one, two, three, four, or five amino acid residues other than aspartic acid at position 55, phenylalanine at position 57, and threonine at position 58, and
      as a CDR-H3 sequence, the amino acid sequence defined in SEQ ID NO 21, or an amino acid sequence derived from SEQ ID NO 21 via substitution, deletion, or insertion of any one, two, three, four, five, six, seven, eight, nine, ten, eleven, or twelve amino acid residues arginine at position 98, glycine at position 100, and glycine at position 109.
[Aspect 9] The anti-gpNMB antibody or its fragment or a derivative thereof according to any one of claims 1 to 7, which comprises at least a heavy chain variable region comprising:
   (1) the amino acid sequence defined in SEQ ID NO 13, or an amino acid sequence having a homology of 60 % or higher to SEQ ID NO 13, or
   (2) the amino acid sequence defined in SEQ ID NO 29, or an amino acid sequence having a homology of 60 % or higher to SEQ ID NO 29, or
   (3) the amino acid sequence defined in SEQ ID NO 45, or an amino acid sequence having a homology of 60 % or higher to SEQ ID NO 45, or
   (4) the amino acid sequence defined in SEQ ID NO 61, or an amino acid sequence having a homology of 60 % or higher to SEQ ID NO 61, or
   (5) the amino acid sequence defined in SEQ ID NO 77, or an amino acid sequence having a homology of 60 % or higher to SEQ ID NO 77, or
   (6) the amino acid sequence defined in SEQ ID NO 93, or an amino acid sequence having a homology of 60 % or higher to SEQ ID NO 93, or
   (7) the amino acid sequence defined in SEQ ID NO 109, or an amino acid sequence having a homology of 60 % or higher to SEQ ID NO 109, or
   (8) the amino acid sequence defined in SEQ ID NO 125, or an amino acid sequence having a homology of 60 % or higher to SEQ ID NO 125, or
   (9) the amino acid sequence defined in SEQ ID NO 141, or an amino acid sequence having a homology of 60 % or higher to SEQ ID NO 141, or
   (10) the amino acid sequence defined in SEQ ID NO 157, or an amino acid sequence having a homology of 60 % or higher to SEQ ID NO 157, or
   (11) the amino acid sequence defined in SEQ ID NO 173, or an amino acid sequence having a homology of 60 % or higher to SEQ ID NO 173, or
   (12) the amino acid sequence defined in SEQ ID NO 299, or an amino acid sequence having a homology of 60 % or higher to SEQ ID NO 299.
[Aspect 10] The anti-gpNMB antibody or its fragment or a derivative thereof according to claim 8 or 9, wherein the heavy chain variable region comprises, as a framework sequence, a framework sequence of any class of human immunoglobulin.
[Aspect 11] The anti-gpNMB antibody or its fragment or a derivative thereof according to any one of claims 8 to 10, which further comprises a heavy chain constant region having an amino acid sequence of a heavy chain constant region of any class of human immunoglobulin.
[Aspect 12] The anti-gpNMB antibody or its fragment or a derivative thereof according to any one of claims 1 to 11, which comprises at least a light chain variable region comprising:
   (1) as a CDR-L1, the amino acid sequence defined in SEQ ID NO 7, or an amino acid sequence derived from SEQ ID NO 7 via substitution, deletion, or insertion of any one or two amino acid residues, or an amino acid sequence having a homology (preferably identity) of 66.6 % or higher or 83.3 % or higher to SEQ ID NO 7,
      as a CDR-L2, the amino acid sequence defined in SEQ ID NO 9, or an amino acid sequence derived from SEQ ID NO 9 via substitution, deletion, or insertion of any one amino acid residue, or an amino acid sequence having a homology (preferably identity) of 66.6 % or higher to SEQ ID NO 9, and
      as a CDR-L3, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 11, or
   (2) as a CDR-L1, the amino acid sequence defined in SEQ ID NO 23, or an amino acid sequence derived from SEQ ID NO 23 via substitution, deletion, or insertion of any one or two amino acid residues, or an amino acid sequence having a homology (preferably identity) of 60.0 % or higher or 80.0 % or higher to SEQ ID NO 23,
      as a CDR-L2, the amino acid sequence defined in SEQ ID NO 25, or an amino acid sequence derived from SEQ ID NO 25 via substitution, deletion, or insertion of any one amino acid residue, or an amino acid sequence having a homology (preferably identity) of 66.6 % or higher to SEQ ID NO 25, and
      as a CDR-L3, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably identity) of 66.6 % or higher, 77.7 % or higher, or 88.8 % or higher, or
   (3) as a CDR-L1, the amino acid sequence defined in SEQ ID NO 39, or an amino acid sequence derived from SEQ ID NO 39 via substitution, deletion, or insertion of any one or two amino acid residues, or an amino acid sequence having a homology (preferably identity) of 60.0 % or higher or 80.0 % or higher to SEQ ID NO 39,
      as a CDR-L2, the amino acid sequence defined in SEQ ID NO 41, or an amino acid sequence derived from SEQ ID NO 41 via substitution, deletion, or insertion of any one amino acid residue, or an amino acid sequence having a homology (preferably identity) of 66.6 % or higher to SEQ ID NO 41, and
      as a CDR-L3, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 43, or
   (4) as a CDR-L1, the amino acid sequence defined in SEQ ID NO 55, or an amino acid sequence derived from SEQ ID NO 55 via substitution, deletion, or insertion of any one or two amino acid residues, or an amino acid sequence having a homology (preferably identity) of 66.6 % or higher or 83.3 % or higher to SEQ ID NO 55,
      as a CDR-L2, the amino acid sequence defined in SEQ ID NO 57, or an amino acid sequence derived from SEQ ID NO 57 via substitution, deletion, or insertion of any one amino acid residue, or an amino acid sequence having a homology (preferably identity) of 66.6 % or higher to SEQ ID NO 57, and
      as a CDR-L3, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 59, or
   (5) as a CDR-L1, the amino acid sequence defined in SEQ ID NO 71, or an amino acid sequence derived from SEQ ID NO 71 via substitution, deletion, or insertion of any one or two amino acid residues, or an amino acid sequence having a homology (preferably identity) of 60.0 % or higher or 80.0 % or higher to SEQ ID NO 71,
      as a CDR-L2, the amino acid sequence defined in SEQ ID NO 73, or an amino acid sequence derived from SEQ ID NO 73 via substitution, deletion, or insertion of any one amino acid residue, or an amino acid sequence having a homology (preferably identity) of 66.6 % or higher to SEQ ID NO 73, and
      as a CDR-L3, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 75, or
   (6) as a CDR-L1, the amino acid sequence defined in SEQ ID NO 87, or an amino acid sequence derived from SEQ ID NO 87 via substitution, deletion, or insertion of any one or two amino acid residues, or an amino acid sequence having a homology (preferably identity) of 66.6 % or higher or 83.3 % or higher to SEQ ID NO 87,
      as a CDR-L2, the amino acid sequence defined in SEQ ID NO 89, or an amino acid sequence derived from SEQ ID NO 89 via substitution, deletion, or insertion of any one amino acid residue, or an amino acid sequence having a homology (preferably identity) of 66.6 % or higher to SEQ ID NO 89, and
      as a CDR-L3, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 91, or
   (7) as a CDR-L1, the amino acid sequence defined in SEQ ID NO 103, or an amino acid sequence derived from SEQ ID NO 103 via substitution, deletion, or insertion of any one or two amino acid residues, or an amino acid sequence having a homology (preferably identity) of 66.6 % or higher or 83.3 % or higher to SEQ ID NO 103,
      as a CDR-L2, the amino acid sequence defined in SEQ ID NO 105, or an amino acid sequence derived from SEQ ID NO 105 via substitution, deletion, or insertion of any one amino acid residue, or an amino acid sequence having a homology (preferably identity) of 66.6 % or higher to SEQ ID NO 105, and
      as a CDR-L3, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 107, or
   (8) as a CDR-L1, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 119,
      as a CDR-L2, the amino acid sequence defined in SEQ ID NO 121, or an amino acid sequence derived from SEQ ID NO 121 via substitution, deletion, or insertion of any one amino acid residue, or an amino acid sequence having a homology (preferably identity) of 66.6 % or higher to SEQ ID NO 121, and
      as a CDR-L3, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 123, or
   (9) as a CDR-L1, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 135,
      as a CDR-L2, the amino acid sequence defined in SEQ ID NO 137, or an amino acid sequence derived from SEQ ID NO 137 via substitution, deletion, or insertion of any one amino acid residue, or an amino acid sequence having a homology (preferably identity) of 66.6 % or higher to SEQ ID NO 137, and
      as a CDR-L3, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 139, or
   (10) as a CDR-L1, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 151,
      as a CDR-L2, the amino acid sequence defined in SEQ ID NO 153, or an amino acid sequence derived from SEQ ID NO 153 via substitution, deletion, or insertion of any one amino acid residue, or an amino acid sequence having a homology (preferably identity) of 66.6 % or higher to SEQ ID NO 153, and
      as a CDR-L3, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 155, or
   (11) as a CDR-L1, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 167,
      as a CDR-L2, the amino acid sequence defined in SEQ ID NO 169, or an amino acid sequence derived from SEQ ID NO 169 via substitution, deletion, or insertion of any one amino acid residue, or an amino acid sequence having a homology (preferably identity) of 66.6 % or higher to SEQ ID NO 169, and
      as a CDR-L3, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 171, or
   (12) as a CDR-L1, the amino acid sequence defined in SEQ ID NO 293, or an amino acid sequence derived from SEQ ID NO 293 via substitution, deletion, or insertion of any one, two, three, or four amino acid residues, or an amino acid sequence having a homology (preferably identity) of 64.2 % or higher, 71.4 % or higher, 81.8 % or higher or 90.9 % or higher to SEQ ID NO 293,
      as a CDR-L2, the amino acid sequence defined in SEQ ID NO 295, or an amino acid sequence derived from SEQ ID NO 295 via substitution, deletion, or insertion of any one amino acid residue, or an amino acid sequence having a homology (preferably identity) of 66.6 % or higher to SEQ ID NO 295, and
      as a CDR-L3, the amino acid sequence defined in SEQ ID NO 297, or an amino acid sequence derived from SEQ ID NO 297 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 297, or
   (13) as a CDR-L1, the amino acid sequence defined in SEQ ID NO 23, or an amino acid sequence derived from SEQ ID NO 23 via substitution, deletion, or insertion of any one, two, or three amino acid residues other than isoleucine at position 29 and tyrosine at position 31,
      as a CDR-L2, the amino acid sequence defined in SEQ ID NO 25, or an amino acid sequence derived from SEQ ID NO 25 via substitution, deletion, or insertion of any one or two amino acid residues other than threonine at No. 50, and
      as a CDR-L3, the amino acid sequence defined in SEQ ID NO 27, or an amino acid sequence derived from SEQ ID NO 27 via substitution, deletion, or insertion of any one, two, three, or four amino acid residues other than histidine at position 88, glutamine at position 89, tryptophan at position 90, serine at position 92, and tyrosine at position 93.
[Aspect 13] The anti-gpNMB antibody or its fragment or a derivative thereof according to any one of claims 1 to 11, which comprises at least a light chain variable region comprising:
   (1) the amino acid sequence defined in SEQ ID NO 15, or an amino acid sequence having a homology of 60 % or higher to SEQ ID NO 15, or
   (2) the amino acid sequence defined in SEQ ID NO 31, or an amino acid sequence having a homology of 60 % or higher to SEQ ID NO 31, or
   (3) the amino acid sequence defined in SEQ ID NO 47, or an amino acid sequence having a homology of 60 % or higher to SEQ ID NO 47, or
   (4) the amino acid sequence defined in SEQ ID NO 63, or an amino acid sequence having a homology of 60 % or higher to SEQ ID NO 63, or
   (5) the amino acid sequence defined in SEQ ID NO 79, or an amino acid sequence having a homology of 60 % or higher to SEQ ID NO 79, or
   (6) the amino acid sequence defined in SEQ ID NO 95, or an amino acid sequence having a homology of 60 % or higher to SEQ ID NO 95, or
   (7) the amino acid sequence defined in SEQ ID NO 111, or an amino acid sequence having a homology of 60 % or higher to SEQ ID NO 111, or
   (8) the amino acid sequence defined in SEQ ID NO 127, or an amino acid sequence having a homology of 60 % or higher to SEQ ID NO 127, or
   (9) the amino acid sequence defined in SEQ ID NO 143, or an amino acid sequence having a homology of 60 % or higher to SEQ ID NO 143, or
   (10) the amino acid sequence defined in SEQ ID NO 159, or an amino acid sequence having a homology of 60 % or higher to SEQ ID NO 159, or
   (11) the amino acid sequence defined in SEQ ID NO 175, or an amino acid sequence having a homology of 60 % or higher to SEQ ID NO 175, or
   (12) the amino acid sequence defined in SEQ ID NO 301, or an amino acid sequence having a homology of 60 % or higher to SEQ ID NO 301.
[Aspect 14] The anti-gpNMB antibody or its fragment or a derivative thereof according to claim 12 or 13, wherein the light chain variable region comprises, as a framework sequence, a framework sequence of any class of human immunoglobulin.
[Aspect 15] The anti-gpNMB antibody or its fragment or a derivative thereof according to any one of claims 12 to 14, which further comprises a light chain constant region having an amino acid sequence of a light chain constant region of any class of human immunoglobulin.
[Aspect 16] The anti-gpNMB antibody or its fragment or a derivative thereof according to any one of claims 1 to 15, which is a Fab, scFv, Diabody, Nanobody, VHH, bispecific antibody, or multispecific antibody, or a derivative thereof.
[Aspect 17] An anti-gpNMB antibody or its fragment or a derivative thereof which binds to at least one site in a region from a PMEL-CAF-like domain to a PKD domain competitively with an anti-gpNMB antibody or its fragment or a derivative thereof according to any one of claims 1 to 16.
[Aspect 18] A nucleic acid molecule comprising a polynucleotide sequence encoding an anti-gpNMB antibody or its fragment or a derivative thereof according to any one of claims 1 to 17.
[Aspect 19] A cloning vector or expression vector carrying at least one nucleic acid molecule according to claim 18.
[Aspect 20] A recombinant cell transformed with a vector according to claim 19.
[Aspect 21] A method for producing an anti-gpNMB antibody or its fragment or a derivative thereof according to any one of claims 1 to 17, comprising culturing a recombinant cell according to claim 20.
[Aspect 22] A method for producing an anti-gpNMB antibody or its fragment or a derivative thereof according to any one of claims 1 to 17, comprising: administering to an animal a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209; and collecting an antibody or its fragment or a derivative thereof produced in the body of the animal.
[Aspect 23] A vaccine having an activity to stimulate the production of an anti-gpNMB antibody or its fragment or a derivative thereof according to any one of claims 1 to 17, comprising a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209. [Aspect 24] A pharmaceutical composition comprising, as an active ingredient, one or more selected from the group consisting of an anti-gpNMB antibody or its fragment or a derivative thereof according to any one of claims 1 to 17, a nucleic acid molecule according to claim 18, a vector according to claim 19, and a recombinant cell according to claim 20.
[Aspect 25] The pharmaceutical composition according to claim 24, for use in decreasing the number of mal-functional microglia in a subject and/or removing amyloid β oligomers in a subject and/or increasing the number of synapses in a subject and/or treating or preventing neurodegenerative disease in a subject.
[Aspect 26] A pharmaceutical composition for use in removing amyloid β oligomers in a subject and/or increasing the number of synapses in a subject and/or treating or preventing neurodegenerative disease in a subject, comprising, as an active ingredient, an agent for decreasing the number of mal-functional microglia.
[Aspect 27] The pharmaceutical composition according to claim 25 or 26, wherein the neurodegenerative disease is Alzheimer's disease.
[Aspect 28] The pharmaceutical composition according to claim 27, further comprising a second active ingredient.
[Aspect 29] The pharmaceutical composition according to claim 28, wherein the second active ingredient is one or more selected from an anti-Tau antibody, anti-amyloid β antibody, anti-CD33 antibody, anti-semaphorin 4D antibody, anti-TNFα antibody, anti-sortilin antibody, anti-galactose-specific lectin (galectin) 3 antibody, and anti-TREM2 (triggering receptor expressed on myeloid cells 2) antibody.
[Aspect 30] The pharmaceutical composition according to claim 28, wherein the second active ingredient is a vaccine comprising a full-length or partial-length polypeptide of one or more proteins selected from Tau, amyloid β, CD33, semaphorin 4D, TNFα, sortilin, galactose-specific lectin (galectin) 3, and TREM2 (triggering receptor expressed on myeloid cells 2), or a nucleic acid encoding the polypeptide.

The term "pharmaceutical drug" used herein is to be interpreted as encompassing both the concepts of "therapeutic agent" and "prophylactic agent," unless it is inconsistent with the context.

### EFFECT OF THE INVENTION

The anti-gpNMB antibodies of the present invention can exhibit extremely unexpected effects, e.g., the effects of decreasing the number of mal-functional microglia in a subject and/or removing amyloid β oligomers in a subject and/or increasing the number of synapses in a subject and/or restoring cognitive functions, by specifically binding to at least one site in a region from a PMEL-CAF-like domain to a PKD domain of human gpNMB. The use of such anti-gpNMB antibodies will also provide pharmaceutical drugs for treating or preventing various neurodegenerative diseases based on new mechanisms of action.

### BRIEF EXPLANATION OF FIGURES

[Figure 1] Figure 1 shows an amino acid sequence alignment of human gpNMB (isotype a) and mouse gpNMB, in which the region at amino acid residues 256 to 319 is shown with dotted lines as an example of the PKD domain, and the region at amino acid residues 172 to 246 is shown with solid lines as an example of the PMEL-CAF-like domain.
[Figure 2] Figure 2 shows immunostained photographs indicating the effect of administration of anti-gpNMB antibodies (GPN05-1 and GPN06-1) on reducing gpNMB-positive microglia in Alzheimer's disease model mice. (A) Control, (B) GPN05-1-treated group, and (C) GPN06-1-treated group. L: left brain, R: right brain, DG: dentate gyrus, CA: cornu ammonis sectors, CA3: hippocampal CA3 region, CA 23: hippocampal CA2-CA3 areas, Ent: Entorhinal Cortex, PPtA: cortical somatosensory cortex.
[Figure 3] Figure 3 is a graph showing the statistical analysis of the immunostained photographs in Figure 2.
[Figure 4] Figure 4 shows immunostained photographs indicating the effect of administration of anti-gpNMB antibodies (GPN05-1 and GPN06-1) on removing of Aβ oligomers in Alzheimer's disease model mice. (A) Control, (B) GPN05-1-treated group, and (C) GPN06-1-treated group.
[Figure 5] Figure 5 shows stained photographs indicating the effect of administration of anti-gpNMB antibodies (GPN05-1 and GPN06-1) on restoring synapses in Alzheimer's disease model mice. (A) Control 1 (Non-Tg mice), (B) Control 2 (APPosk mice), (C) GPN05-1-treated group, and (D) GPN06-1-treated group.
[Figure 6] Figure 6 shows immunostained photographs of brain sections of Alzheimer's disease model mice after antibody administration. (A) Control antibody 11E10-treated group, (B) GPN09-1-treated group, (C) GPN11-10-treated group, (D) GPN15-2-treated group, (E) GPN15-3-treated group, and (F) GPN18 -2 treated group.
[Figure 7] Figure 7 is a graph showing the statistical analysis of the immunostained photographs in Figure 6.
[Figure 8] Figure 8 shows stained photographs indicating the effect of administration of an anti-gpNMB antibody (GPN18-2) on restoring synapses in Alzheimer's disease model mice. (A) Control (Non-Tg mice), and (B) GPN18-2-treated group.
[Figure 9] Figure 9 shows immunostained photographs of brain sections of Alzheimer's disease model mice after antibody administration. (A) Control antibody-treated group, (B) GPN18-2-treated group, (C) GPN06-1-treated group, (D) GPN18-5-treated group, and (E) 1-5E-treated group.
[Figure 10] Figure 10 is a graph showing the statistical analysis of the immunostained photographs in Figure 9.
[Figure 11] Figure 11 is a graph showing the results of Morris water maze test (Acquisition test) on Alzheimer's disease model mice treated with anti-gpNMB antibodies (GPN06-1 and GPN18-2).
[Figure 12] Figure 12 is a graph showing the results of Morris water maze test (Probe test) on Alzheimer's disease model mice treated with anti-gpNMB antibodies (GPN06-1 and GPN18-2).
[Figure 13] Figure 13 shows the alignment of humanized mutant sequences VH1 to VH5 to mouse VH0.
[Figure 14] Figure 14 shows the alignment of humanized mutant sequences VL1 to VL5 to mouse VL0.

### DESCRIPTION OF EMBODIMENTS

The present invention will now be described based on specific embodiments. These embodiments should not be construed to limit the scope of the present invention. All references, including patent publications, unexamined patent publications, and non-patent publications cited in this specification, can be incorporated by reference in their entirety for all purposes.

### [Anti-gpNMB antibody]

An embodiment of the present invention relates to an anti-gpNMB antibody or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B) (hereinafter also referred to as "the anti-gpNMB antibody of the present invention" or "the antibody of the present invention"). The statement "at least one site in a region from a PMEL-CAF-like domain to a PKD domain of gpNMB" herein refers to at least one site in a region with, e.g., the amino acid residues at positions 172 to 319 in human gpNMB having the amino acid sequence defined in SEQ ID NO 209.

### *Antibody:

The term "antibody" herein refers to a glycoprotein containing at least two heavy (H) chains and two light (E) chains interconnected through disulfide bonds. Each heavy chain contains a heavy chain variable region (abbreviated as VH) and a heavy chain constant region, and the heavy chain constant region contains three domains, CH1, CH2 and CH3. Each light chain contains a light chain variable region (abbreviated as VL) and a light chain constant region. The light chain constant region contains one domain, CL. The light chain constant region has two chains called a λ chain and a κ chain. The heavy chain constant region has a γ chain, a µ chain, an α chain, a δ chain, and an ε chain, and has isotypes of antibodies called IgG, IgM, IgA, IgD and IgE, respectively, depending on the difference in the heavy chain. The VH and VL regions are further divided into four regions (FR-1, FR-2, FR-3, FR-4) having higher preservability called framework regions (FR) and three regions (CDR-1, CDR-2, CDR-3) called complementarity determining regions (CDR). The VH region comprises three CDRs and four FRs, in detail, a sequence of FR-1, CDR-1 (CDR-H1), FR-2, CDR-2 (CDR-H2), FR-3, CDR-3 (CDR-H3), and FR4 from the amino terminal to the carboxy terminal. The VL region comprises three CDRs and four FRs, in detail, a sequence of FR-1, CDR-1 (CDR-L1), FR-2, CDR-2 (CDR-L2), FR-3, CDR-3 (CDR-L3), and FR4 from the amino terminal to the carboxy terminal. The variable regions of the heavy and light chains contain binding domains that interact with an antigen.

The antibody of the present invention may be a fragment (e.g., an antigen-binding fragment) and/or a derivative of an antibody, as long as it has the activity to bind specifically to at least one site in a region from a PMEL-CAF-like domain to a PKD domain of gpNMB. Examples of fragments of antibodies include F (ab') 2, Fab, and Fv. Examples of derivatives of antibodies include an antibody in which an amino acid mutation is artificially incorporated in the constant region, an antibody in which the domain configuration of the constant region is modified, an antibody in the form having two or more Fc fragments per molecule, an antibody composed of only a heavy chain or only a light chain, a sugar chain modified antibody, a bispecific antibody, an antibody conjugate combined with an antibody or a fragment compound of an antibody or a protein other than antibodies, an antibody enzyme, a nanobody, a tandem scFv fragment, a bispecific tandem scFv fragment, a diabody, and a VHH body. In the present invention, the term "antibody" simply includes a fragment and/or a derivative of an antibody unless otherwise specified.

The antibody of the present invention may be either a monoclonal antibody or a polyclonal antibody, but may preferably be a monoclonal antibody. The term monoclonal antibody classically refers to an antibody molecule obtained from a clone derived from a single antibody producing cell, and is a single type of antibody molecule containing a combination of VH and VL each consisting of a specific amino acid sequence. It is possible to obtain from a monoclonal antibody a nucleic acid molecule having a gene sequence encoding the amino acids of each of the proteins constituting the antibody, and it is also possible to produce the antibody using such nucleic acid molecules using genetic engineering methods. It is well known to a person skilled in the art that genetic information on the sequences of, e.g., the H and L chains, their variable regions, and their CDRs can be used to modify the antibody to improve its binding and specificity, etc., and that antibodies from animals such as mice can be modified into human-type antibodies to produce antibodies with a structure suitable for use as therapeutic agents. A human monoclonal antibody can be also produced with a transgenic animal into which a human antibody gene has been introduced to be sensitized with an antigen. Other methods that do not require sensitization of animals include the use of phage libraries expressing antigen-binding regions of human antibodies or portions thereof (human antibody phage display) to obtain antibodies that bind specifically to the corresponding antigens or phage clones consisting of specific amino acid sequences, and those skilled in the art can use such information to produce human antibodies as appropriate (see, e.g., the review by Taketo Tanaka et al., in Keio J. Med., (2011), 60:37-46). Those skilled in the art can also design antibodies to be administered to non-human animals using the amino acid sequence information of CDRs and variable regions as appropriate, using a technique similar to the humanization technique.

The specificity of an antibody herein means that the antibody exhibits a high antigen-antibody reaction to a specific antigen. Those skilled in the art can determine the antigen-antibody reaction by appropriately selecting a binding measurement method in a solid phase or liquid phase system. Examples of such methods include, although not limited to, enzyme-linked immunosorbent assay (ELISA), enzyme immunoassay (EIA), surface plasmon resonance (SPR), fluorescence resonance energy transfer (FRET), and luminescence resonance energy transfer (LRET). Before the binding between an antigen and an antibody is measured, the antibody and/or the antigen may be labeled with, e.g., an enzyme, a fluorescent substance, a luminescent substance, or a radioisotope, and then the antigen-antibody reaction may be detected by a method suitable for the physical and/or chemical properties of the labeled substance.

### *Specific binding to at least one site in a region from a PMEL-CAF-like domain to a PKD domain of gpNMB:

The antibody of the present invention specifically binds to at least one site in a region from a PMEL-CAF-like domain to a PKD domain of gpNMB. The term "gpNMB" (glycoprotein nonmetastatic melanoma protein B; also referred to as Osteoactivin or DC-HIL) herein refers to the single transmembrane glycoprotein mentioned above. For more details, see each of the documents mentioned above (especially Non-Patent Literature 20).

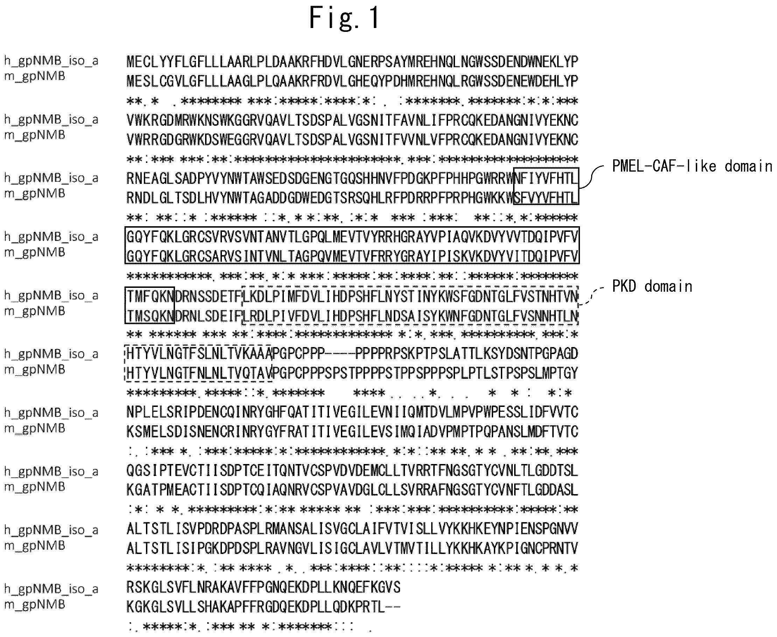

The amino acid sequence of the total length protein of human gpNMB (isotype a) is shown in SEQ ID NO 209, and the nucleic acid sequence of a gene encoding the protein is shown in SEQ ID NO 210. The amino acid sequence of the total length protein of mouse gpNMB is shown in SEQ ID NO 211, and the nucleic acid sequence of a gene encoding the protein is shown in SEQ ID NO 212. The amino acid sequence of the total length protein of rat gpNMB is shown in SEQ ID NO 213, and the nucleic acid sequence of a gene encoding the protein is shown in SEQ ID NO 214. The alignment of the amino acid sequences of human gpNMB (isotype a) and mouse gpNMB is shown in Figure 1.

The PKD (Polycystic Kidney Disease homology) domain of gpNMB is a domain containing a beta sheet consisting of three strands and a beta sheet consisting of four strands folded together to form a beta sandwich structure. It was named the PKD domain because it is a characteristic structure possessed by polycystin-1 (polycystin-1), the gene responsible for polycystic kidney disease (Non-Patent Literature 26). According to an embodiment, in the case of the human gpNMB defined in SEQ ID NO 209 and the mouse gpNMB defined in SEQ ID NO 211, the region containing the amino acid residues at positions 256 to 319 corresponds to the PKD domain. According to an embodiment, the PKD domain may preferably be a region containing the amino acid residues at positions 256 to 316, more preferably a region containing positions 257 to 316, still more preferably a region containing 268 to 316, even more preferably a region containing 270 to 316, particularly preferably a region containing 282 to 316, especially preferably a region containing 287 to 316. According to an embodiment, the PKD domain may preferably be a region containing the amino acid residues at positions 257 to 319, more preferably a region containing positions 257 to 316, still more preferably a region containing positions 257 to 301. According to an embodiment, the PKD domain may preferably be a region containing the amino acid residues at positions 287 to 301. The region at amino acid residues 256 to 319 surrounded by a solid line in Figure 1 is an example of the PDK domain of each of human gpNMB and mouse gpNMB.

The PMEL-CAF-like (PMEL Core Amyloid Fragment-like) domain of gpNMB is a region that is homologous to a CAF region in PMEL of a protein of the same family as described above, which is a region that induces the formation of amyloid-like aggregates in a PMEL protein. The PMEL-CAF-like domain of gpNMB is considered to prevent aggregation formation by having many N-type glycan binding sites in the neighboring PKD region (Non-Patent Literature 67). According to an embodiment, in the case of the human gpNMB defined in SEQ ID NO 209 and the mouse gpNMB defined in SEQ ID NO 211, the region containing the amino acid residues at positions 172 to 246 corresponds to the PMEL-CAF-like domain. The PMEL-CAF-like domain may preferably be a region containing the amino acid residues at positions 178 to 228, more preferably a region containing the amino acid residues at positions 186 to 218. The region at amino acid residues 172 to 246 surrounded by a solid line in Figure 1 is an example of the PMEL-CAF-like domain of each of human gpNMB and mouse gpNMB.

The term "region from a PMEL-CAF-like domain to a PKD domain of gpNMB" herein refers to a region containing not only the PMEL-CAF-like domain and the PKD domain defined above, but also a region intervening between the PMEL-CAF-like domain and the PKD domain. According to an embodiment, in the case of the human gpNMB defined in SEQ ID NO 209 and the mouse gpNMB defined in SEQ ID NO 211, the region containing the amino acid residues at positions 172 to 319 corresponds to a region from a PMEL-CAF-like domain to a PKD domain.

The antibody of the present invention may have the ability to specifically bind to at least one site in a region from a PMEL-CAF-like domain to a PKD domain of gpNMB of any vertebrate. However, in view of human pharmaceutical uses, the antibody of the present invention may preferably have the ability to specifically bind to at least one site in a region from a PMEL-CAF-like domain to a PKD domain of at least the human gpNMB defined in SEQ ID NO 209. From the viewpoint of evaluating the effect on endogenous gpNMB in disease model mice and disease model rats, the antibody of the present invention may more preferably have the ability to specifically bind to at least one site in a region from the PMEL-CAF-like domain to the PKD domain of the mouse gpNMB defined in SEQ ID NO 211, in addition to at least one site in a region from a PMEL-CAF-like domain to a PKD domain of human gpNMB. The statement that an antibody "specifically" binds to gpNMB herein refers to the binding ability of the antibody to gpNMB with a binding strength of 10-fold or more, preferably 100-fold or more, more preferably 1000-fold or more, compared to the binding ability of the antibody to proteins other than gpNMB (e.g. albumin). In addition, the "region from the PMEL-CAF-like domain to the PKD domain" herein refers to a region including the PMEL-CAF-like domain, the PKD domain, and the region intervening between the PMEL-CAF-like domain and the PKD domain.

When the antibody of the present invention specifically binds to at least one site in a region from a PMEL-CAF-like domain to a PKD domain of human gpNMB, the epitope in the region to which the antibody binds is not particularly limited. However, the antibody of the present invention may preferably have the ability to specifically bind to at least one site or more in a region from the PMEL-CAF-like domain to the PKD domain, and may more preferably have the specifically ability to bind to one or more sites selected from a site containing amino acid residue(s) K257 and/or D258 of the PKD domain, a site containing amino acid residue(s) H268 and/or D269 of the PKD domain, a site containing an amino acid residue K282 of the PKD domain, a site containing amino acid residue(s) D287 and/or H301 of the PKD domain, a site containing an amino acid residue K316 of the PKD domain, a site containing an amino acid residue K186 of the PMEL-CAF-like domain, a site containing amino acid residue(s) R214 and/or R215 of the PMEL-CAF-like domain, and a site containing amino acid residue(s) H216 and/or R218 of the PMEL-CAF-like domain of the human gpNMB defined in SEQ ID NO 209. Among them, the antibody of the present invention may preferably have the ability to specifically bind to at least either or both of a site containing amino acid residue(s) D287 and/or H301 of the PKD domain and a site containing amino acid residue(s) R214 and/or R215 of the PMEL-CAF-like domain of the human gpNMB defined in SEQ ID NO 209, and in addition to these sites, it may more preferably have the ability to also specifically bind to one or more sites selected from a site containing amino acid residue(s) K257 and/or D258, a site containing amino acid residue(s) H268 and/or D269, a site containing an amino acid residue K282, a site containing an amino acid residue K316, K186 a region containing an amino acid residue, and a site containing amino acid residue(s) H216 and/or R218 of human gpNMB.

The binding strength of the antibody of the present invention to the region from the PMEL-CAF-like domain to the PKD domain of gpNMB is not particularly limited. However, as measured by antigen ELISA using recombinant gpNMB and/or cell ELISA using gpNMB-expressing cells, the antibody of the present invention may preferably have a 50% effective concentration (EC₅₀) to the region from the PMEL-CAF-like domain to the PKD domain of gpNMB of typically 1×10⁻⁷M or lower, or 1×10⁻⁸M or lower, or 1×10⁻⁹M or lower. The "50% effective concentration" (EC₅₀) of an antibody herein refers to the concentration of the antibody that exhibits 50% of the maximum binding affinity with which that antibody binds to the antigen. Specific conditions for measuring EC₅₀ by antigen ELISA using recombinant gpNMBs and/or cell ELISA using gpNMB-expressing cells include those employed in the Examples described below.

The antibody of the present invention may also bind to one or more sites in any region other than the region from the PMEL-CAF-like domain to the PKD domain of gpNMB. Examples of amino acid sites to which the antibody of the present invention may bind other than the region from the PMEL-CAF-like domain to the PKD domain of gpNMB may preferably include, although not limited to, E385 of the human gpNMB defined in SEQ ID NO 209.

### *Activities of the antibody:

According to an embodiment, the antibody of the present invention may preferably have various activities explained below.

The antibody of the present invention may preferably have the activity to bind to microglia. It has been reported that gpNMB is expressed in mail-functional microglia that have become mal-functional in their ability to degrade toxic or unwanted proteins under stress (Non-Patent Literature 43). The antibody of the present invention may preferably have the activity to bind to at least one site in a region from the PMEL-CAF-like domain to the PKD domain of gpNMB expressed on the plasma membrane of such mal-functional microglia.

The antibody of the present invention may also preferably have various activities against microglia. Specifically, the antibody of the present invention may preferably have at least one or more of:
(a) the activity to reduce or remove mal-functional microglia;
(b) the activity to promote normal microglia's functions to phagocytize mal-functional microglia or the activity to normalize the mal-function of mal-functional microglia; and
(c) the activity to enhance the effect of normal microglia in the vicinity of mal-functional microglia.

The relationship between the aforementioned activities of the antibody of the present invention, i.e., (a) the activity to reduce or remove mal-functional microglia, (b) the activity to promote normal microglia's functions to phagocytize mal-functional microglia or the activity to normalize the mal-function of mal-functional microglia, and (c) the activity to enhance the effects of normal microglia in the vicinity of mal-functional microglia, is unknown. However, although not necessarily bound by the theory, the relationship is inferred as follows. Specifically, it is assumed that binding of the anti-gpNMB antibody to mal-functional microglia may have them recognized by normal microglia with phagocytic activity, which phagocytize and remove the mal-functional microglia to create a space, in which normal microglia proliferate to promote normalization of the brain environment, thereby inducing removal of toxic proteins from neurons and restoration of the number of synapses. Alternatively, it is assumed that the anti-gpNMB antibody may promote phagocytic activation or proliferation of microglia and enhance phagocytosis of mal-functional microglia to promote normalization of the brain environment, thereby inducing removal of toxic proteins from neurons and restoration of the number of synapses.

The antibody of the present invention may also preferably have the activity to improve or restore the brain environment or neuronal functions. Specifically, the antibody of the present invention may preferably have at least either or both of:
(x) the activity to remove amyloid beta oligomers; and
(y) the activity to increase the number of synapses.

The antibody of the present invention may also preferably have (z) the activity to treat or prevent neurodegenerative diseases. Specifically, the antibody of the present invention may preferably have the activity to treat or prevent central neurodegenerative diseases. Specifically, the antibody of the present invention the antibody of the present invention may preferably have the activity to treat or prevent central neurodegenerative diseases explained below.

Diseases classified as neurodegenerative diseases can be divided into tauopathy and other neurodegenerative diseases. Specific examples of each categories are as follows.

Specific examples of tauopathy (diseases in which tau accumulates and causes neurodegeneration) include Alzheimer's disease, frontotemporal lobar degeneration (FTLD-Tau), frontotemporal dementia (FTD), primary age-related tauopathy (PART), chronic traumatic encephalopathy (CTE), Pick's disease, cerebral cortex basal ganglia degeneration (CBD or CBS), progressive supranuclea palsy (PSP), globular glial tauopathy (GGT), argyrophilic grain dementia (AGD) (argyrophilic grain disease), aging-related tau astrogliopathy (ARTAG), familial British dementia (FBD), familial Danish dementia (FDD), FTDP17, multisystem tauopathy with dementia (MSTD), dementia of the neurofibrillary tangle type, diffuse neurofibrillary tangles with calcification (DNTC), and white matter tauopathy with globular glial inclusions (WMT-GGI).

Specific examples of neurodegenerative diseases other than tauopathy include Parkinson's disease (αSyn accumulation), Huntington's disease (mutant huntingtin accumulation), spinal cord cerebellum degeneration (polyglutamic acid protein accumulation), hereditary spinocerebellar ataxia (polyglutamic acid protein accumulation), spinobulbar muscular atrophy (polyglutamic acid protein accumulation), Lewy body dementia (αSyn accumulation), Creutzfeldt-Jakob disease (abnormal prion protein accumulation), amyotrophic lateral sclerosis (ALS) (SOD or TDP43 accumulation), and frontotemporal lobar degeneration (FTLD-TDP, FTLD-FUS:TDP43, or FUS accumulation).

In the Examples described below, the present inventors analyzed the effect of the antibodies of the present invention by administering antibodies according to an embodiment of the present invention to senile Alzheimer's disease (AD) model mice, and evaluating their brain sections by immunohistochemistry. The results showed that administration of their antibodies reduced gpNMB-positive microglia in the brains of AD model mice. More surprisingly, it was found that Aβ oligomers, the most toxic form of amyloid-β in the brains of AD model mice, were removed from neurons. Even more surprisingly, it was found that administration of the antibodies restored the number of neuronal synapses in the brains of AD model mice to normal levels. Still more surprisingly, it was found that administration of the antibodies restored cognitive function in the Morris water maze test using the present inventors' newly constructed APP/Tau-Tg (AD model mice).

The antibody of the present invention may exert the aforementioned various activities either in vivo or ex vivo, or both in vivo and ex vivo. Either in vivo or ex vivo, the subjects in which the aforementioned various activities are exhibited is not limited, but the antibody of the present invention may preferably exert the aforementioned various activities at least in vertebrates, more preferably at least in mammals, especially in humans or non-human animals.

### *Amino acid sequence and nucleic acid sequence of each region of each chain of the antibody:

The amino acid sequence of the antibody of the present invention is not particularly limited, as long as it has the ability to specifically bind to at least one site in a region from a PMEL-CAF-like domain to a PKD domain of gpNMB. However, the antibody of the present invention may preferably have a specific amino acid sequence as each CDR sequence. The details will be explained below. Incidentally, the "identity" of amino acid sequences herein refers to the ratio of matching amino acid residues, and the "homology" of amino acid sequences herein refers to the ratio of matching or similar amino acid residues. The homology and identity of amino acid sequences can be determined by, e.g., BLAST method (default conditions of NCBI's PBLAST). It is also clear that when the phrase "a homology of 80% or more" is used, for example, it includes "an identity of 80% or more."

The term "similar amino acid residues" herein refers to amino acid residues having side chains with similar chemical characteristics (e.g., charge or hydrophobicity). Similar amino acid residues include, for example, the following combinations.
(1) Amino acid residues with aliphatic side chains: glycine (Gly or G), alanine (Ala or A), valine (Val or V), leucine (Leu or L), and isoleucine (Ile or I) residue.
(2) Amino acid residues with aliphatic hydroxyl side chains: serine (Ser or S) and threonine (Thr or T) residue.
(3) Amino acid residues with amide-containing side chains: asparagine(Asn or N)and glutamine(Gln or Q) residue.
(4) Amino acid residues with aromatic side chains: phenylalanine (Phe or F), tyrosine (Tyr or Y), and tryptophan (Trp or W) residue.
(5) Amino acid residues with basic side chains: lysine (Lys or K), arginine(Arg or R)and histidine (His or H) residue.
(6) Amino acid residues with acidic side chains: aspartic acid (Asp or D)and glutamic acid (Glu or E) residue.
(7) Amino acid residues with sulfur-containing side chains: cysteine (Cys or C), and methionine (Met or M) residue.

In addition, the combination of (1) and methionine (Met or M) and the combination of (4) and histidine (His or H) are also treated as similar amino acid residues.

In some cases, substitutions can be made regardless of similarity if the corresponding variants exist with reference to the germline sequence. Germline-referenced variants include substitutions, deletions, or additions. Substitutions include cases where amino acid residues after substitution are presumed to affect the direction of the side chain such as proline (Pro or P) and glycine (Gly or G).

An embodiment of the antibody of the present invention relates to antibodies an antibody containing a heavy chain variable region having any of the combination of the CDR-H1 to -H3 sequences specified in (1) to (12) below.
(1) As a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 1,
   as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 3, and
   as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably identity) of 66.6 % or higher, 75.0 % or higher, 83.3 % or higher, or 91.6 % or higher to SEQ ID NO 5.
(2) As a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 17,
   as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 19, and
   as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 21.
(3) As a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 33,
   as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 35, and
   as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 37.
(4) As a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 49,
   as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 51, and
   as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably identity) of 62.5 % or higher, 68.7 % or higher, 75.0 % or higher, 81.2 % or higher, 87.5 % or higher, or 93.7 % or higher to SEQ ID NO 53.
(5) As a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 65,
   as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 67, and
   as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably identity) of 64.2 % or higher, 71.4 % or higher, 78.5 % or higher, 85.7 % or higher, or 92.8 % or higher to SEQ ID NO 69.
(6) As a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 81,
   as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably identity) of 71.4 % or higher or 85.7 % or higher to SEQ ID NO 83, and
   as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 85.
(7) As a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 97,
   as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 99, and
   as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably identity) of 60.0 % or higher or 80.0 % or higher to SEQ ID NO 101.
(8) As a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 113,
   as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably identity) of 71.4 % or higher or 85.7 % or higher to SEQ ID NO 115, and
   as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 117.
(9) As a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 129,
   as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably identity) of 71.4 % or higher or 85.7 % or higher to SEQ ID NO 131, and
   as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 133.
(10) As a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 145,
   as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 147, and
   as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 149.
(11) As a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 161,
   as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably identity) of 71.4 % or higher or 85.7 % or higher to SEQ ID NO 163, and
   as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 165.
(12) As a CDR-H1 sequence, the amino acid sequence defined in SEQ ID NO 287, or an amino acid sequence derived from SEQ ID NO 287 via substitution, deletion, or insertion of any one, two, or three amino acid residues, or an amino acid sequence having a homology (preferably identity) of 62.5 % or higher, 75.0 % or higher, 87.5 % or higher, or 90.0 % or higher to SEQ ID NO 287,
   as a CDR-H2 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably identity) of 71.4 % or higher or 85.7 % or higher to SEQ ID NO 289, and
   as a CDR-H3 sequence, the amino acid sequence defined in SEQ ID NO 291, or an amino acid sequence derived from SEQ ID NO 291 via substitution, deletion, or insertion of any one or two amino acid residues, or an amino acid sequence having a homology (preferably identity) of 66.6 % or higher or 83.3 % or higher to SEQ ID NO 291.
   As shown in Example 17 below, the present inventors investigated the importance of each amino acid residue of each CDR of humanized anti-gpNMB antibody H1L1 (clone GPN06-1) in maintaining its binding ability by alanine scanning. The results showed that in the heavy chain variable region of the humanized anti-gpNMB antibody H1L1 (SEQ ID NO 29), the following amino acid residues were identified as important for maintaining binding: in CDR-H1 (SEQ ID NO 17), asparagine at position 32 and tryptophan at position 33; in CDR-H2 (SEQ ID NO 19), aspartic acid at position 55, phenylalanine at position 57, and threonine at position 58; and in CDR-H3 (SEQ ID NO 21), arginine at position 98, glycine at position 100, and glycine at position 109. On the other hand, the amino acid residues of each CDR other than those listed above were identified as having little effect on the maintenance of the antibody's binding ability. Based on these findings, one embodiment of the antibody of the present invention relates to an antibody containing a heavy chain variable region with the CDR-H1 to -H3 sequences specified in (13) below (the amino acid positions specified below are indicated with respect to the amino acid sequence of the heavy chain variable region of humanized anti-gpNMB antibody H1L1 (SEQ ID NO 29)).
(13) As a CDR-H1 sequence, the amino acid sequence defined in SEQ ID NO 17, or an amino acid sequence derived from SEQ ID NO 17 via substitution, deletion, or insertion of any one, two, three, four, five, or six amino acid residues other than asparagine at position 32 and tryptophan at position 33,
   as a CDR-H2 sequence, the amino acid sequence defined in SEQ ID NO 19, or an amino acid sequence derived from SEQ ID NO 19 via substitution, deletion, or insertion of any one, two, three, four, or five amino acid residues other than aspartic acid at position 55, phenylalanine at position 57, and threonine at position 58, and
   as a CDR-H3 sequence, the amino acid sequence defined in SEQ ID NO 21, or an amino acid sequence derived from SEQ ID NO 21 via substitution, deletion, or insertion of any one, two, three, four, five, six, seven, eight, nine, ten, eleven, or twelve amino acid residues arginine at position 98, glycine at position 100, and glycine at position 109.

An embodiment of the antibody of the present invention relates to antibodies an antibody containing a light chain variable region having any of the combination of the CDR-L1 to - L3 sequences specified in (1) to (12) below.
(1) As a CDR-L1, the amino acid sequence defined in SEQ ID NO 7, or an amino acid sequence derived from SEQ ID NO 7 via substitution, deletion, or insertion of any one or two amino acid residues, or an amino acid sequence having a homology (preferably identity) of 66.6 % or higher or 83.3 % or higher to SEQ ID NO 7,
   as a CDR-L2, the amino acid sequence defined in SEQ ID NO 9, or an amino acid sequence derived from SEQ ID NO 9 via substitution, deletion, or insertion of any one amino acid residue, or an amino acid sequence having a homology (preferably identity) of 66.6 % or higher to SEQ ID NO 9, and
   as a CDR-L3, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 11.
(2) As a CDR-L1, the amino acid sequence defined in SEQ ID NO 23, or an amino acid sequence derived from SEQ ID NO 23 via substitution, deletion, or insertion of any one or two amino acid residues, or an amino acid sequence having a homology (preferably identity) of 60.0 % or higher or 80.0 % or higher to SEQ ID NO 23,
   as a CDR-L2, the amino acid sequence defined in SEQ ID NO 25, or an amino acid sequence derived from SEQ ID NO 25 via substitution, deletion, or insertion of any one amino acid residue, or an amino acid sequence having a homology (preferably identity) of 66.6 % or higher to SEQ ID NO 25, and
   as a CDR-L3, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 27.
(3) As a CDR-L1, the amino acid sequence defined in SEQ ID NO 39, or an amino acid sequence derived from SEQ ID NO 39 via substitution, deletion, or insertion of any one or two amino acid residues, or an amino acid sequence having a homology (preferably identity) of 60.0 % or higher or 80.0 % or higher to SEQ ID NO 39,
   as a CDR-L2, the amino acid sequence defined in SEQ ID NO 41, or an amino acid sequence derived from SEQ ID NO 41 via substitution, deletion, or insertion of any one amino acid residue, or an amino acid sequence having a homology (preferably identity) of 66.6 % or higher to SEQ ID NO 41, and
   as a CDR-L3, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 43.
(4) As a CDR-L1, the amino acid sequence defined in SEQ ID NO 55, or an amino acid sequence derived from SEQ ID NO 55 via substitution, deletion, or insertion of any one or two amino acid residues, or an amino acid sequence having a homology (preferably identity) of 66.6 % or higher or 83.3 % or higher to SEQ ID NO 55,
   as a CDR-L2, the amino acid sequence defined in SEQ ID NO 57, or an amino acid sequence derived from SEQ ID NO 57 via substitution, deletion, or insertion of any one amino acid residue, or an amino acid sequence having a homology (preferably identity) of 66.6 % or higher to SEQ ID NO 57, and
   as a CDR-L3, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 59.
(5) As a CDR-L1, the amino acid sequence defined in SEQ ID NO 71, or an amino acid sequence derived from SEQ ID NO 71 via substitution, deletion, or insertion of any one or two amino acid residues, or an amino acid sequence having a homology (preferably identity) of 60.0 % or higher or 80.0 % or higher to SEQ ID NO 71,
   as a CDR-L2, the amino acid sequence defined in SEQ ID NO 73, or an amino acid sequence derived from SEQ ID NO 73 via substitution, deletion, or insertion of any one amino acid residue, or an amino acid sequence having a homology (preferably identity) of 66.6 % or higher to SEQ ID NO 73, and
   as a CDR-L3, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 75.
(6) As a CDR-L1, the amino acid sequence defined in SEQ ID NO 87, or an amino acid sequence derived from SEQ ID NO 87 via substitution, deletion, or insertion of any one or two amino acid residues, or an amino acid sequence having a homology (preferably identity) of 66.6 % or higher or 83.3 % or higher to SEQ ID NO 87,
   as a CDR-L2, the amino acid sequence defined in SEQ ID NO 89, or an amino acid sequence derived from SEQ ID NO 89 via substitution, deletion, or insertion of any one amino acid residue, or an amino acid sequence having a homology (preferably identity) of 66.6 % or higher to SEQ ID NO 89, and
   as a CDR-L3, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 91.
(7) As a CDR-L1, the amino acid sequence defined in SEQ ID NO 103, or an amino acid sequence derived from SEQ ID NO 103 via substitution, deletion, or insertion of any one or two amino acid residues, or an amino acid sequence having a homology (preferably identity) of 66.6 % or higher or 83.3 % or higher to SEQ ID NO 103,
   as a CDR-L2, the amino acid sequence defined in SEQ ID NO 105, or an amino acid sequence derived from SEQ ID NO 105 via substitution, deletion, or insertion of any one amino acid residue, or an amino acid sequence having a homology (preferably identity) of 66.6 % or higher to SEQ ID NO 105, and
   as a CDR-L3, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 107.
(8) As a CDR-L1, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 119,
   as a CDR-L2, the amino acid sequence defined in SEQ ID NO 121, or an amino acid sequence derived from SEQ ID NO 121 via substitution, deletion, or insertion of any one amino acid residue, or an amino acid sequence having a homology (preferably identity) of 66.6 % or higher to SEQ ID NO 121, and
   as a CDR-L3, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 123.
(9) As a CDR-L1, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 135,
   as a CDR-L2, the amino acid sequence defined in SEQ ID NO 137, or an amino acid sequence derived from SEQ ID NO 137 via substitution, deletion, or insertion of any one amino acid residue, or an amino acid sequence having a homology (preferably identity) of 66.6 % or higher to SEQ ID NO 137, and
   as a CDR-L3, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 139.
(10) As a CDR-L1, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 151,
   as a CDR-L2, the amino acid sequence defined in SEQ ID NO 153, or an amino acid sequence derived from SEQ ID NO 153 via substitution, deletion, or insertion of any one amino acid residue, or an amino acid sequence having a homology (preferably identity) of 66.6 % or higher to SEQ ID NO 153, and
   as a CDR-L3, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 155.
(11) As a CDR-L1, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 167,
   as a CDR-L2, the amino acid sequence defined in SEQ ID NO 169, or an amino acid sequence derived from SEQ ID NO 169 via substitution, deletion, or insertion of any one amino acid residue, or an amino acid sequence having a homology (preferably identity) of 66.6 % or higher to SEQ ID NO 169, and
   as a CDR-L3, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 171.
(12) As a CDR-L1, the amino acid sequence defined in SEQ ID NO 293, or an amino acid sequence derived from SEQ ID NO 293 via substitution, deletion, or insertion of any one, two, three, or four amino acid residues, or an amino acid sequence having a homology (preferably identity) of 64.2 % or higher, 71.4 % or higher, 81.8 % or higher or 90.9 % or higher to SEQ ID NO 293,
   as a CDR-L2, the amino acid sequence defined in SEQ ID NO 295, or an amino acid sequence derived from SEQ ID NO 295 via substitution, deletion, or insertion of any one amino acid residue, or an amino acid sequence having a homology (preferably identity) of 66.6 % or higher to SEQ ID NO 295, and
   as a CDR-L3, the amino acid sequence defined in SEQ ID NO 297, or an amino acid sequence derived from SEQ ID NO 297 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 297.
   As shown in Example 17 below, the present inventors investigated the importance of each amino acid residue of each CDR of humanized anti-gpNMB antibody H1L1 (clone GPN06-1) in maintaining its binding ability by alanine scanning. The results showed that in the light chain variable region of the humanized anti-gpNMB antibody H1L1 (SEQ ID NO 31), the following amino acid residues were identified as important for maintaining binding: in CDR-L1 (SEQ ID NO 23), isoleucine at position 29 and tyrosine at position 31; in CDR-L2 (SEQ ID NO 25), threonine at position 50; and in CDR-L3 (SEQ ID NO 27), histidine at position 88, glutamine at position 89, tryptophan at position 90, serine at position 92, and tyrosine at position 93. On the other hand, the amino acid residues of each CDR other than those listed above were identified as having little effect on the maintenance of the antibody's binding ability. Based on these findings, one embodiment of the antibody of the present invention relates to an antibody containing a light chain variable region with the CDR-L1 to -L3 sequences specified in (13) below (the amino acid positions specified below are indicated with respect to the amino acid sequence of the light chain variable region of humanized anti-gpNMB antibody H1L1 (SEQ ID NO 31)).
(13) As a CDR-L1, the amino acid sequence defined in SEQ ID NO 23, or an amino acid sequence derived from SEQ ID NO 23 via substitution, deletion, or insertion of any one, two, or three amino acid residues other than isoleucine at position 29 and tyrosine at position 31,
   as a CDR-L2, the amino acid sequence defined in SEQ ID NO 25, or an amino acid sequence derived from SEQ ID NO 25 via substitution, deletion, or insertion of any one or two amino acid residues other than threonine at No. 50, and
   as a CDR-L3, the amino acid sequence defined in SEQ ID NO 27, or an amino acid sequence derived from SEQ ID NO 27 via substitution, deletion, or insertion of any one, two, three, or four amino acid residues other than histidine at position 88, glutamine at position 89, tryptophan at position 90, serine at position 92, and tyrosine at position 93.

An embodiment of the antibody of the present invention relates to an antibody having a heavy chain variable region having any of the combinations of the CDR-H1 to H3 sequences described above and a light chain variable region having any of the combinations of the CDR-L1 to L3 sequences described above.

An embodiment of the antibody of the present invention relates to an antibody having any of the following amino acid sequences (a) to (c) as the sequence of the heavy chain variable region.
(a) An amino acid sequence selected from SEQ ID NO 13, SEQ ID NO 29, SEQ ID NO 45, SEQ ID NO 61, SEQ ID NO 77, SEQ ID NO 93, SEQ ID NO 109, SEQ ID NO 125, SEQ ID NO 141, SEQ ID NO 157, SEQ ID NO 173, and SEQ ID NO 299.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody having any of the following amino acid sequences (a) to (c) as the sequence of the light chain variable region.
(a) An amino acid sequence selected from SEQ ID NO 15, SEQ ID NO 31, SEQ ID NO 47, SEQ ID NO 63, SEQ ID NO 79, SEQ ID NO 95, SEQ ID NO 111, SEQ ID NO 127, SEQ ID NO 143, SEQ ID NO 159, SEQ ID NO 175 and SEQ ID NO 301.
(b) An amino acid sequence having a homology (preferably identity) of at least 60 % or higher, or 65 % or higher, or 70 % or higher, or 75 % or higher, or 80 % or higher, or 85 % or higher, or 90 % or higher, or 95 % or higher, or 96 % or higher, or 97 % or higher, or 98 % or higher, or 99 % or higher to any of the amino acid sequences specified in (a) above.
(c) An amino acid sequence derived from any of the amino acid sequences specified in (a) above via substitution, deletion, or addition of 1 to 12 (preferably 1 to 11, more preferably 1 to 10, 1 to 9, 1 to 8, 1 to 7, 1 to 6, 1 to 5, 1 to 4, 1 to 3, 1 to 2, or 1) amino acid residues.

An embodiment of the antibody of the present invention relates to an antibody having any of the heavy chain variable region sequences described above and any of the light chain variable region sequences described above.

The CDR-H1, CDR-H2, CDR-H3, CDR-L1, CDR-L2, and CDR-L3 sequences in the antibody can be identified by the Kabat method (NIH Publication, (1991) No. 91-3242) or the Chothia method (J. Mol. Biol., (1997), 273). These methods are within the technical common knowledge for those skilled in the art, and are also outlined in, e.g., the Dr. Andrew C.R. Martin's Group Internet home page (http://www.bioinf.org.uk/abs/). The CDRs described herein are indicated as Kabat's CDRs.

Framework sequences of each of the heavy chain variable region and the light chain variable region of the immunoglobulin as the antibody of the present invention may preferably be framework sequences in each class of vertebrate immunoglobulin and, more preferably, framework sequences in each class of immunoglobulin of human or a non-human animal such as mouse or rat.

A person skilled in the art would be able to design the anti-gpNMB-specific antibody of the present invention by combining the amino acid sequence of each of the CDRs and/or each of the heavy and light chain variable regions described above with the amino acid sequence of each of the framework regions and/or each of the heavy and light chain constant regions of an antibody derived from human or non-human animals including mouse or rat, as appropriate. In this regard, a humanized anti-gpNMB-specific antibody can be designed by using the amino acid sequence of each of the framework regions and/or each of the heavy and light chain constant regions of a human antibody. The amino acid sequence of each of the framework regions and/or each of the heavy and light chain constant regions of such a humanized antibody can be selected from, e.g., each class of human IgG, IgA, IgM, IgE, and IgD, and their variants.

The antibody of the present invention may preferably be an antibody of the IgG class or its variant, more preferably of the human IgG class or its variant, of the human IgG4 subclass or its variant, or the human IgG1 subclass or its variant. In one example, the stabilized IgG4 constant region contains a proline at position 241 in the hinge region, according to Kabat's system. This position corresponds to position 228 of the hinge region according to the EU numbering scheme (Proc. Natl. Acad. Sci. USA, (1969), 63[1]:78-85), Sequences of proteins of immunological interest (Washington DC United States Department of Health and Human Services, 2001 and NIH Publication, (1991), No. 91-3242). This residue is generally serine in human IgG4, but its stabilization can be induced by substituting the serine with proline. In one example, the N297A mutation can be incorporated into the constant region of IgG1 to suppress its ability to bind to the Fc receptor and/or anchor complement as much as possible.

### Competitive binding:

An embodiment of the present invention relates to an antibody that binds to at least one site in a region from a PMEL-CAF-like domain to a PKD domain of gpNMB competitively wih the binding of the antibody of the present invention to the same site. Such a competitively binding antibody is also included in the scope of the present invention. The term "competitive binding" herein refers to phenomenon where when at least two monoclonal antibodies coexist with an antigen, the binding of one antibody to the antigen is inhibited by the binding of the other antibody to the antigen. Competitive binding ability between two monoclonal antibodies can generally be measured by using a fixed amount (concentration) of a first monoclonal antibody, adding varying amounts (concentrations) of a second monoclonal antibody thereto, and measuring the amount (concentration) of the second monoclonal antibody added that decreases the binding of the fixed amount of the first monoclonal antibody to the antigen. The degree of inhibition can be expressed in terms of IC₅₀ or Ki. A monoclonal antibody that competitively binds to the antibody of the present invention may refer to, e.g., an antibody whose IC₅₀ measured in terms of competitive antigen-antibody binding to at least one site in a region from a PMEL-CAF-like domain to a PKD domain of gpNMB using 10 nM of the antibody of the invention is usually 1000nM or lower, particularly 100nM or lower, more particularly 10nM or lower. Competitive binding ability can be measured by, e.g., labelling the antibody with an enzyme, fluorescent substance, luminescent substance, radioisotope, etc., and carrying out detection using a measurement method suitable for the physical and/or chemical properties of the labeled substance, or by using a biosensor based on, e.g., surface plasmon resonance (SPR) and bio-layer interferometry (BLI).

### [Production method of the anti-gpNMB antibody]

The antibody according to the present invention can be obtained using techniques known to those skilled in the art. The antibody according to the present invention may be either a polyclonal antibody or a monoclonal antibody (Nature, (1983), 305(5934): 537-40). A polyclonal antibody according to the present invention can be produced, e.g., by preparing a human gpNMB protein having the amino acid sequence defined in SEQ ID NO 209 or a gpNMB partial peptide having a part of the amino acid sequence (e.g., a peptide containing a PKD domain with the amino acid residues 256 to 319) as an antigen, or preparing an antigen-expressing polynucleotide which encodes the amino acid sequence of such a human gpNMB protein or gpNMB partial peptide, administering the antigen or antigen-expressing polynucleotide to a mammal intramuscularly or subcutaneously to express the antigen in the animal body and thereby sensitize the animal, and recovering the antibody produced in the animal body from, e.g., serum of the animal. When a peptide is used as an antigen, it may be bound to a carrier protein such as BSA or KLH or a polylysine.

A monoclonal antibody according to the present invention can be produced, e.g., by administering the human gpNMB protein or the gpNMB partial peptide or the antigen-expressing polynucleotide encoding the human gpNMB protein or gpNMB partial peptide mentioned above to a mammal to thereby sensitize the animal, collecting immune cells from the mammal and fusing them with myeloma cells to produce hybridomas, cloning each hybridoma, and recovering the antibody produced by a culture of the cloned hybridoma. Such methods for obtaining monoclonal antibodies (Nature, (1992), 356[6365]:152-4) and cell fusion techniques (Nature, (1975), 256[5517]:495-7) have already been reported and generalized, and the administration of immunostimulants can further enhance the acquisition efficiency (Cancer Gene Ther., (2007), 14[11]:904-17). Monoclonal antibodies obtained by this method include, although not limited to, the following.

*An antibody containing a heavy chain variable region the amino acid sequence defined in SEQ ID NO 13 and a light chain variable region the amino acid sequence defined in SEQ ID NO 15 (GPN05-1).
* An antibody containing a heavy chain variable region the amino acid sequence defined in SEQ ID NO 29 and a light chain variable region the amino acid sequence defined in SEQ ID NO 31 (GPN06-1).
* An antibody containing a heavy chain variable region the amino acid sequence defined in SEQ ID NO 45 and a light chain variable region the amino acid sequence defined in SEQ ID NO 47 (GPN07-1).
* An antibody containing a heavy chain variable region the amino acid sequence defined in SEQ ID NO 61 and a light chain variable region the amino acid sequence defined in SEQ ID NO 63 (GPN11-10).
* An antibody containing a heavy chain variable region the amino acid sequence defined in SEQ ID NO 77 and a light chain variable region the amino acid sequence defined in SEQ ID NO 79 (GPN15-2).
* An antibody containing a heavy chain variable region the amino acid sequence defined in SEQ ID NO 93 and a light chain variable region the amino acid sequence defined in SEQ ID NO 95 (GPN15-3).
* An antibody containing a heavy chain variable region the amino acid sequence defined in SEQ ID NO 109 and a light chain variable region the amino acid sequence defined in SEQ ID NO 111 (GPN18-4).
* An antibody containing a heavy chain variable region the amino acid sequence defined in SEQ ID NO 125 and a light chain variable region the amino acid sequence defined in SEQ ID NO 127 (GPN18-1).
* An antibody containing a heavy chain variable region the amino acid sequence defined in SEQ ID NO 141 and a light chain variable region the amino acid sequence defined in SEQ ID NO 143 (GPN18-2).
* An antibody containing a heavy chain variable region the amino acid sequence defined in SEQ ID NO 157 and a light chain variable region the amino acid sequence defined in SEQ ID NO 159 (GPN18-6).
* An antibody containing a heavy chain variable region the amino acid sequence defined in SEQ ID NO 173 and a light chain variable region the amino acid sequence defined in SEQ ID NO 175 (GPN18-7).
* An antibody containing a heavy chain variable region the amino acid sequence defined in SEQ ID NO 299 and a light chain variable region the amino acid sequence defined in SEQ ID NO 301 (GPN09-1).

Another production method includes producing a gene encoding the heavy chain and/or the light chain of the antibody, or more specifically, a nucleic acid molecule encoding the heavy chain and/or the light chain of immunoglobulin of the antibody, from a hybridoma which produces the antibody to be obtained, or from a phage clone obtained by human antibody phage display. Here, the nucleic acid molecule may be introduced into various vectors or plasmids to create a vector or plasmid containing the nucleic acid molecule. Host cells are then transformed with the nucleic acid molecule, vector, or plasmid described above. The host cells may be selected from eukaryotic cells, such as mammalian, insect, yeast, or plant cells, or from bacterial cells. The transformed host cells are then cultured under appropriate conditions for producing the anti-gpNMB-specific antibody of the present invention. If necessary, the anti-gpNMB-specific antibody of the present invention produced may be isolated from the host cells. The various methods used in these procedures are all well known to those skilled in the art.

A person skilled in the art can also produce antibody chimeric proteins, small molecule antibodies, scaffolded antibodies, etc., using known techniques, by genetic modification of genes encoding heavy and/or light chains of immunoglobulins to introduce desired traits or by using structural information of variable regions or CDR regions of heavy and/or light chains of immunoglobulins. A person skilled in the art can also make any modifications to the structure of the constant regions or to the glycosylation sites of the antibody using techniques well known to those skilled in the art in order to improve the performance of the antibody or to avoid any side effects.

### [Pharmaceutical composition]

The anti-gpNMB-specific antibody of the present invention may preferably be used as an active ingredient of a pharmaceutical composition. Accordingly, an embodiment of the present invention relates to a pharmaceutical composition containing the anti-gpNMB-specific antibody of the present invention as an active ingredient (hereinafter also collectively referred to as "the first pharmaceutical composition of the present invention").

As mentioned above, the anti-gpNMB-specific antibody of the present invention may be expected to express one or more of the activities specified in (a) to (c) and (w) to (z) below when administered to a subject such as human. Therefore, the first pharmaceutical composition of the present invention may preferably be a pharmaceutical composition intended for one or more of the activities specified in (a) to (c) and (w) to (z) below.
(a) The activity to reduce or remove mal-functional microglia.
(b) The activity to promote normal microglia's functions to phagocytize mal-functional microglia or the activity to normalize the mal-function of mal-functional microglia.
(c) The activity to enhance the effect of normal microglia in the vicinity of mal-functional microglia.
(w) The activity to recover cognitive functions.
(x) The activity to remove amyloid β oligomers.
(y) The activity to increase the number of synapses.
(z) The activity to treat or prevent neurodegenerative diseases.

As mentioned above, one embodiment of the anti-gpNMB-specific antibody of the present invention is estimated to exhibit one or more of the activities specified in (w) to (z) above as a result of exhibiting one or more of the activities specified in (a) to (c) above. Therefore, when the anti-gpNMB-specific antibody of the present invention is an antibody that expresses one or more of the activities specified in (a) to (c) above, the first pharmaceutical composition of the present invention may preferably be a pharmaceutical composition intended for one or more of the activities specified in (w) to (z) above. Preferable among the activities specified in (a) to (c) above is the activity specified in (a) above.

Based on the results of the Examples described herein, it is very likely that one or more of the activities specified in (w) to (z) above are exhibited as a result of one or more of the activities specified in (a) to (c) above. This is a novel finding discovered by the present inventors. Based on this finding, it is also possible to prepare a pharmaceutical compositions that exhibit one or more of the activities specified in (w) to (z) above by not using the anti-gpNMB specific antibody of the present invention, but using any other agent that has one or more of the activities specified in (a) to (c) above (e.g., a small molecule compound or a macromolecular drug such as an antibody). Accordingly, an embodiment of the present invention relates to a pharmaceutical composition intended for one or more of the activities specified in (w) to (z) above, containing an agent that has one or more of the activities specified in (a) to (c) above (which may be, e.g., a small molecule compound or a macromolecular drug such as an antibody) as an active ingredient (hereinafter also collectively referred to as "the second pharmaceutical composition of the present invention"). Preferable among the activities specified in (a) to (c) above is the activity specified in (a) above.

The details of the first pharmaceutical composition of the present invention and the second pharmaceutical composition of the present invention (hereinafter also collectively referred to as "the pharmaceutical composition of the present invention") are, without limitation, as follows.

The pharmaceutical composition of the present invention may be formulated in the form of a pharmaceutical composition containing, in addition to the active ingredient (the anti-gpNMB-specific antibody of the present invention in the case of the first pharmaceutical composition of the present invention, or an agent that has one or more of the activities specified in (a) to (c) above in the case of the second pharmaceutical composition of the present invention), a pharmaceutically acceptable carrier and/or other additive. Formulation using a pharmaceutically acceptable carrier and/or other additive may be carried out using, e.g., the methods described in University of the Sciences in Philadelphia, "Remington: The Science and Practice of Pharmacy, 20th EDITION", Lippincott Williams & Wilkins, (2000).

One form of such a therapeutic or prophylactic agent is a liquid or lyophilized form prepared by dissolving, suspending, or emulsifying the ingredients in a sterile aqueous or oily solution. Examples of such solvents or dissolving solutions as aqueous media include distilled water for injection and saline. In addition, osmotics (e.g., D-glucose, D-sorbitol, D-mannitol, sodium chloride, etc.) may be added, as well as suitable dissolution aids, such as alcohols (e.g., ethanol), poly alcohols (e.g., propylene glycol, polyethylene glycol), nonionic surfactants (e.g., polysorbate 80, polyoxyethylene hardened castor oil 50), etc., may be used in combination. Oil-based liquids may also be used as solvents or dissolving solutions. Examples of such oily liquids include sesame oil and soybean oil. Benzyl benzoate, benzyl alcohol, etc., may also be used as a dissolution aid. In such formulations, additives may be used as appropriate. Examples of additives include: buffers (e.g., phosphate buffer and acetate buffer), soothing agents (e.g., benzalkonium chloride and procaine hydrochloride), stabilizers (e.g., human serum albumin and polyethylene glycol), preservatives (e.g., ascorbic acid, erythorbic acid, and their salts), colorants (e.g., copper chlorophyll, β-carotene, red #2, and blue #1), antiseptics (e.g., paraoxybenzoate, phenol, benzethonium chloride, and benzalkonium chloride), thickeners (e.g., hydroxy propyl cellulose, carboxyl methyl cellulose, and their salts), stabilizing agents (e.g., human serum albumin, mannitol, and sorbitol), and deodorants (e.g., menthol and citrus flavoring).

Other forms of therapeutic or prophylactic agents include solid dosage forms such as dispersions, tablets, granules, capsules, pills, suppositories, and lozenges. Solid dosage forms administered in the form of oral preparations may contain additives such as excipients (e.g., crystalline cellulose, lactose, and starch), lubricants (e.g., magnesium stearate and talc), binding agents (e.g., hydroxypropyl cellulose, hydroxypropyl methylcellulose, and macrogol), and disintegrants (e.g., starch and calcium carboxymethylcellulose). In addition, additives such as preservatives (e.g., benzyl alcohol, chlorobutanol, methyl paraoxibenzoate, propyl paraoxibenzoate, etc.), antioxidants, colorants, and sweetening agents can be used as needed. Another form of the therapeutic or prophylactic agent is intended for mucosal application. This type of formulation may contain adhesives, adhesion enhancers, viscosity agents, and viscosity enhancers (e.g., mucin, canten, gelatin, pectin, carrageenan, sodium alginate, locust bean gum, xanthan gum, tragacanth gum, gum arabic, chitosan, pullulan, waxy starch, sucralfate, cellulose, and their derivatives) are contained as additives mainly to provide absorption and retention on the mucosa. gum arabic, chitosan, pullulan, waxy starches, sucralfate, cellulose, and their derivatives). However, the form of the therapeutic or prophylactic agent to be provided to the living body and the solvent and additives used therein are not limited to these, and can be selected by a person skilled in the art as appropriate.

The pharmaceutical composition of the present invention may contain, in addition to the active ingredient (the anti-gpNMB-specific antibody of the present invention in the case of the first pharmaceutical composition of the present invention, or an agent that has one or more of the activities specified in (a) to (c) above in the case of the second pharmaceutical composition of the present invention), another known drug (active ingredient). Examples of such other known drugs (active ingredients) include, although not limited to, various therapeutic drugs for central neurological diseases, therapeutic drugs for neurodegenerative diseases, nerve function recovery drugs, and therapeutic drugs for Alzheimer's disease. Specific examples include anti-Tau antibodies, anti-amyloid β antibodies, anti-CD33 antibodies, anti-semaphorin 4D antibodies, anti-TNFα antibodies, anti-sortilin antibodies, anti-galactose-specific-lectin (galectin) 3 antibodies, and anti-TREM2 (Triggering receptor expressed on myeloid cells 2) antibodies. They may be used singly or in combination of any two or more.

The pharmaceutical composition of the present invention may also be combined with another known drug in the form of a kit. Examples of other drugs (active ingredients) that can be combined with the anti-gpNMB-specific antibody of the present invention include the known drugs (active ingredients) described above for combination drugs. The dose of the drugs other than the anti-gpNMB-specific antibody used in the form of the combination drug or kit can be determined based on doses normally used for treatment, but can be increased or decreased depending on the circumstances.

The pharmaceutical compositions of the present invention may be administered parenterally for the purpose of improving symptoms. For parenteral administration, it can be administered as, e.g., an intranasal formulation, and liquid, turbid, or solid formulations can be selected. Another parenteral dosage form may be an injectable form, which may be administered by subcutaneous, intravenous, intravenous, intramuscular, intracerebroventricular, or intraperitoneal injection. Other parenteral formulations include those for transmucosal administration other than suppositories, sublingual, transdermal, and other than nasal administration. In addition, the antibody can be locally administered intravascularly as contained in or applied to a stent or intravascular occlusive agent. In some cases, it is also possible to administer the antibody in the form of DNA or RNA encoding the antibody, or in the form of cells or commensal bacteria producing the antibody.

The dosage of the pharmaceutical composition of the present invention varies depending on the age, sex, weight, symptoms, therapeutic effect, method of administration, treatment time, or type of active ingredient contained in the pharmaceutical composition, etc. of the patient, but may usually be from 0.1 mg to 1 g, preferably from 0.5 mg to 300 mg, of the active ingredient per adult per dose. It may be administered once every week to every four weeks or once every month to every six months. However, the dosage and frequency of administration may vary depending on various conditions, and a lower dosage and/or frequency of administration than the aforementioned dosage and/or frequency may be sufficient, or a higher dosage and/or frequency of administration than the aforementioned dosage and/or frequency may be necessary. In addition, the therapeutic or prophylactic agent according to the present invention can be effective in a short period of administration or can be administered for a long period of time by reducing side effects.

### [Others]

As explained above, the anti-gpNMB-specific antibody of the present invention may be expected to express one or more of the activities specified in (a) to (c) and (w) to (z) below when administered to a subject such as human.
(a) The activity to reduce or remove mal-functional microglia.
(b) The activity to promote normal microglia's functions to phagocytize mal-functional microglia or the activity to normalize the mal-function of mal-functional microglia.
(c) The activity to enhance the effect of normal microglia in the vicinity of mal-functional microglia.
(w) The activity to recover cognitive functions.
(x) The activity to remove amyloid β oligomers.
(y) The activity to increase the number of synapses.
(z) The activity to treat or prevent neurodegenerative diseases.

Accordingly, in addition to the pharmaceutical composition of the present invention, the subject of the present invention may include the following embodiments.

Specifically, an embodiment of the present invention relates to a method for achieving one or more of the activities specified in (a) to (c) and (w) to (z) above in a subject, comprising administering an effective amount of the anti-gpNMB-specific antibody of the present invention to a subject in need thereof. When the anti-gpNMB-specific antibody of the present invention is an antibody expressing one or more of the activities specified in (a) to (c) above, the method may preferably be intended to achieve one or more of the activities in (w) to (z) above in the subject. Other details are as explained above. Preferable among the activities specified in (a) to (c) above is the activity specified in (a) above.

An embodiment of the present invention relates to a method for achieving one or more of the activities in (w) to (z) above in a subject, comprising administering an effective amount of an agent that has one or more of the activities specified in (a) to (c) above (which may be, e.g., a small molecule compound or a macromolecular drug such as an antibody) to a subject in need thereof. Other details are as explained above. Preferable among the activities specified in (a) to (c) above is the activity specified in (a) above.

An embodiment of the present invention relates to the anti-gpNMB-specific antibody of the present invention for use in achieving one or more of the activities specified in (a) to (c) and (w) to (z) above in a subject. When the anti-gpNMB-specific antibody of the present invention is an antibody expressing one or more of the activities specified in (a) to (c) above, the antibody may preferably be for use in achieving one or more of the activities in (w) to (z) above in the subject. Other details are as explained above. Preferable among the activities specified in (a) to (c) above is the activity specified in (a) above.

An embodiment of the present invention relates to an agent that has one or more of the activities specified in (a) to (c) above (which may be, e.g., a small molecule compound or a macromolecular drug such as an antibody) for use in achieving one or more of the activities in (w) to (z) above in a subject. Other details are as explained above. Preferable among the activities specified in (a) to (c) above is the activity specified in (a) above.

An embodiment of the present invention relates to the use of the anti-gpNMB-specific antibody of the present invention in the manufacture of a medicament for achieving one or more of the activities specified in (a) to (c) and (w) to (z) above in a subject. When the anti-gpNMB-specific antibody of the present invention is an antibody expressing one or more of the activities specified in (a) to (c) above, the medicament may preferably be for use in achieving one or more of the activities in (w) to (z) above in the subject. Other details are as explained above. Preferable among the activities specified in (a) to (c) above is the activity specified in (a) above.

An embodiment of the present invention relates to the use of an agent that has one or more of the activities specified in (a) to (c) above (which may be, e.g., a small molecule compound or a macromolecular drug such as an antibody) in the manufacture of a medicament for achieving one or more of the activities in (w) to (z) above in a subject. Other details are as explained above. Preferable among the activities specified in (a) to (c) above is the activity specified in (a) above.

### EXAMPLES

The present invention will now be described in further detail by way of Examples. These examples are shown merely for convenience of the description, and should not be construed as limitations to the present invention in any sense.

### [Common procedures]

### *Antigen ELISA (Enzyme-Linked Immunosorbent Assay):

Recombinant soluble human or mouse gpNMB was diluted by PBS to 2 µg/mL, added to a 96-well plate (Nunc, MaxiSorp) at 50 µL/well, and allowed to stand overnight at 4°C. The 96-well plate was blocked with 3% BSA/PBS and used for ELISA as recombinant soluble gpNMB-fixed 96-well plate.

Hybridoma culture supernatant containing an anti-gpNMB antibody or 3% BSA/PBS solution was added at 30 µL/well to the above recombinant soluble gpNMB-fixed 96-well plate and allowed to react for 1 hour at room temperature. After washing with washing buffer (TBS-T: Tris Buffered Saline, 0.025% Tween 20), anti-mouse IgG antibody-ALP conjugate (SBA, 1050 -04), diluted 1000-fold in 3% BSA/PBS, was added at 30 µL/well and allowed to react for 1 hour at room temperature. Substrate (PNPP (p-nitrophenyl phosphate), 1 mg/mL) solution was added at 100 µL/well and allowed to react for 1 hour at room temperature, and absorbance at 405-550 nm was calculated. The absorbance values obtained were evaluated as binding activity.

### *Cell-based ELISA:

The gpNMB-expressing cells were seeded into a polylysine-coated 96-well plate and allowed to adhere, and the culture supernatant was removed. The cells were then fixed with a 10% formalin-containing solution for 10 minutes at room temperature, and the supernatant was removed. The cells were then blocked with 3% BSA/PBS solution, and stored under refrigeration.

After the blocking solution was removed from the cell-fixed plate obtained above, and antibody-containing culture medium or solution was added at 30 µL/well, and incubated at room temperature for about 1 hour to 1 hour 30 minutes. The cells were washed 2 to 4 times with washing buffer (TBS-T), and a 1000-fold dilution of anti-mouse IgG-HRP labeled secondary antibody solution was added to the plate, and incubated at room temperature for 1 hour. The cells were washed 2 to 4 times with washing buffer (TBS-T), and substrate TMB solution was then added at 50 µL/well, and incubated at room temperature for 30 minutes. The reaction was then stopped by adding 1N sulfuric acid at 50 µL/well, and the absorbance at 450-650 nm was calculated. The absorbance value obtained was evaluated as binding activity.

### [Example 11 Production and screening of mouse or rat monoclonal antibodies (antigen ELISA and cell-based ELISA):

Mouse or rat monoclonal antibodies can be produced by the hybridoma method described in, e.g., Koehler et al., Nature, (1975), 256:495-497. Anti-gpNMB antibodies were produced by inducing immunity in mice or rats using nucleic acids, proteins, or peptides containing the full length or part of the amino acid sequence (SEQ ID NO 209) or the nucleic acid sequence (SEQ ID NO 210) of human gpNMB (isotype a), or the amino acid sequence (SEQ ID NO 211) or the nucleic acid sequence (SEQ ID NO 212) of mouse gpNMB, and collecting lymphocyte cells from the mice or rats with elevated antibody titer. All animal experiments were performed according to institutional regulations. Hybridoma generation by fusion of lymphocyte cells from mice or rats with mouse myeloma cell lines (P3U1 (P3X63Ag8U.1) or SP2/0 (SP2/0-Ag14)) was performed using standard hybridoma techniques. Hybridomas were selected using so-called HAT medium, which contains hypoxanthine, aminopterin, and thymidine. An amino acid sequence alignment of human gpNMB (isotype a) (SEQ ID NO 209) and mouse gpNMB (SEQ ID NO 211) is shown in Figure 1, in which the region at amino acid residues 256 to 319 is shown with dotted lines as an example of the PKD domain, and the region at amino acid residues 172 to 246 is shown with solid lines as an example of the PMEL-CAF-like domain.

The resulting hybridoma cultures were subjected to antigen ELISA with fixed recombinant human soluble gpNMB (SEQ ID NO 221) or mouse soluble gpNMB (SEQ ID NO 223) or cell-based ELISA with cells expressing proteins containing human gpNMB (SEQ ID NO 209) to evaluate binding ability, and the wells containing positive hybridomas were selected. Hybridomas in these wells were single-cloned by limiting dilution. The single-cloned positive hybridomas were cultured, and monoclonal antibodies were purified from the culture media using Protein A column (Ab-Capture, ProteNova) to obtain multiple anti-gpNMB antibodies.

### [Example 21 Sequencing of the antibodies:

Each anti-gpNMB antibody clone obtained in Example 1 was subjected to the SMARTer^{(™)} RACE method to determine the gene sequences of its light and heavy chains. RNA derived from the hybridoma producing the antibody was subjected to the SMARTer^{(™)} RACE method to thereby obtain gene fragments of the heavy and light chains of the antibody, including the start and end codons, and their nucleotide sequences were determined. Specifically, the 1st strand cDNA was synthesized with the SMARTer (registered trademark) RACE 5'/3' Kit (634859, Clontech) using total RNA from the hybridoma as a template, and the cDNA was amplified by PCR reaction. Using the cDNA as a template, PCR reactions were performed using primers for the universal sequence provided in the kit and primers specific for each of the heavy and light chains of the antibody. The resulting PCR products were used as 5'RACE PCR products for TA cloning.

When sequencing a rat monoclonal antibody gene, PCR reactions were performed on the variable regions of the heavy and light chains of the rat antibody, and the resulting PCR fragments were bound to the mouse constant regions to produce a mouse chimeric antibody, which was then subjected to sequencing.

For TA cloning, the 5' RACE PCR products were electrophoresed, and cDNA fragments having desired molecular weights were purified using the QIAEX II Gel Extraction Kit (20021, Qiagen). The purified cDNA fragments were subjected to reaction using TaKaRa-Taq (R001A, Takara) at 72°C for 5 minutes to add adenine at the 3' end. The cDNA fragments were then cloned into the pMD20-T vector (hereafter referred to as MD20 vector) using the Mighty TA-cloning Kit (6028, Takara) according to the attached protocol. The MD20 vector in which the target cDNA fragments were cloned was transformed into E. coli TOP10, and cultured on agar medium containing 100 µg/mL ampicillin. Insertion of the target cDNA fragments into the MD20 vector was confirmed by colony PCR. The nucleotide sequences of the cloned cDNA fragments were determined. Similarly, the nucleotide sequences of the 3' RACE PCR products were determined, whereby the full-length sequence of the gene for each antibody was determined.

Of the anti-gpNMB antibody clones obtained in Example 1, GPN05-1, GPN06-1, GPN07-1, GPN09-1, GPN11-10, GPN15-2, GPN15-3, GPN18-1, GPN18-2, GPN18-4, GPN18-5, GPN18-6, GPN18-7, and 1-5E are shown in Tables 1-1 to 1-5, along with the Sequence Identification Nos. of the amino acid sequences and nucleotide sequences of their heavy chain and light chain variable regions (hereinafter also referred to as VH and VL, respectively) and the complementarity determining regions included therein (hereinafter also referred to as CDR-H1 to H3 and CDR-L1 to L3, respectively), as determined by the methods explained above.

**[Table 1-1]**

| Antibody clone | Description | Amino acid sequence | | Nucleic acid sequence | |
|---|---|---|---|---|---|
| GPN05-1 | CDR-H1 | SEQ ID NO | 1 | SEQ ID NO | 2 |
| | CDR-H2 | SEQ ID NO | 3 | SEQ ID NO | 4 |
| | CDR-H3 | SEQ ID NO | 5 | SEQ ID NO | 6 |
| | CDR-L1 | SEQ ID NO | 7 | SEQ ID NO | 8 |
| | CDR-L2 | SEQ ID NO | 9 | SEQ ID NO | 10 |
| | CDR-L3 | SEQ ID NO | 11 | SEQ ID NO | 12 |
| | Heavy chain variable region (VH) | SEQ ID NO | 13 | SEQ ID NO | 14 |
| | Light chain variable region (VL) | SEQ ID NO | 15 | SEQ ID NO | 16 |
| GPN06-1 | CDR-H1 | SEQ ID NO | 17 | SEQ ID NO | 18 |
| | CDR-H2 | SEQ ID NO | 19 | SEQ ID NO | 20 |
| | CDR-H3 | SEQ ID NO | 21 | SEQ ID NO | 22 |
| | CDR-L1 | SEQ ID NO | 23 | SEQ ID NO | 24 |
| | CDR-L2 | SEQ ID NO | 25 | SEQ ID NO | 26 |
| | CDR-L3 | SEQ ID NO | 27 | SEQ ID NO | 28 |
| | Heavy chain variable region (VH) | SEQ ID NO | 29 | SEQ ID NO | 30 |
| | Light chain variable region (VL) | SEQ ID NO | 31 | SEQ ID NO | 32 |
| GPN07-1 | CDR-H1 | SEQ ID NO | 33 | SEQ ID NO | 34 |
| | CDR-H2 | SEQ ID NO | 35 | SEQ ID NO | 36 |
| | CDR-H3 | SEQ ID NO | 37 | SEQ ID NO | 38 |
| | CDR-L1 | SEQ ID NO | 39 | SEQ ID NO | 40 |
| | CDR-L2 | SEQ ID NO | 41 | SEQ ID NO | 42 |
| | CDR-L3 | SEQ ID NO | 43 | SEQ ID NO | 44 |
| | Heavy chain variable region (VH) | SEQ ID NO | 45 | SEQ ID NO | 46 |
| | Light chain variable region (VL) | SEQ ID NO | 47 | SEQ ID NO | 48 |

**[Table 1-2]**

| Antibody clone | Description | Amino acid sequence | | Nucleic acid sequence | |
|---|---|---|---|---|---|
| GPN11-10 | CDR-H1 | SEQ ID NO | 49 | SEQ ID NO | 50 |
| | CDR-H2 | SEQ ID NO | 51 | SEQ ID NO | 52 |
| | CDR-H3 | SEQ ID NO | 53 | SEQ ID NO | 54 |
| | CDR-L1 | SEQ ID NO | 55 | SEQ ID NO | 56 |
| | CDR-L2 | SEQ ID NO | 57 | SEQ ID NO | 58 |
| | CDR-L3 | SEQ ID NO | 59 | SEQ ID NO | 60 |
| | Heavy chain variable region (VH) | SEQ ID NO | 61 | SEQ ID NO | 62 |
| | Light chain variable region (VL) | SEQ ID NO | 63 | SEQ ID NO | 64 |
| GPN15-2 | CDR-H1 | SEQ ID NO | 65 | SEQ ID NO | 66 |
| | CDR-H2 | SEQ ID NO | 67 | SEQ ID NO | 68 |
| | CDR-H3 | SEQ ID NO | 69 | SEQ ID NO | 70 |
| | CDR-L1 | SEQ ID NO | 71 | SEQ ID NO | 72 |
| | CDR-L2 | SEQ ID NO | 73 | SEQ ID NO | 74 |
| | CDR-L3 | SEQ ID NO | 75 | SEQ ID NO | 76 |
| | Heavy chain variable region (VH) | SEQ ID NO | 77 | SEQ ID NO | 78 |
| | Light chain variable region (VL) | SEQ ID NO | 79 | SEQ ID NO | 80 |
| GPN15-3 | CDR-H1 | SEQ ID NO | 81 | SEQ ID NO | 82 |
| | CDR-H2 | SEQ ID NO | 83 | SEQ ID NO | 84 |
| | CDR-H3 | SEQ ID NO | 85 | SEQ ID NO | 86 |
| | CDR-L1 | SEQ ID NO | 87 | SEQ ID NO | 88 |
| | CDR-L2 | SEQ ID NO | 89 | SEQ ID NO | 90 |
| | CDR-L3 | SEQ ID NO | 91 | SEQ ID NO | 92 |
| | Heavy chain variable region (VH) | SEQ ID NO | 93 | SEQ ID NO | 94 |
| | Light chain variable region (VL) | SEQ ID NO | 95 | SEQ ID NO | 96 |

**[Table 1-3]**

| Antibody clone | Description | Amino acid sequence | | Nucleic acid sequence | |
|---|---|---|---|---|---|
| GPN18-4 | CDR-H1 | SEQ ID NO | 97 | SEQ ID NO | 98 |
| | CDR-H2 | SEQ ID NO | 99 | SEQ ID NO | 100 |
| | CDR-H3 | SEQ ID NO | 101 | SEQ ID NO | 102 |
| | CDR-L1 | SEQ ID NO | 103 | SEQ ID NO | 104 |
| | CDR-L2 | SEQ ID NO | 105 | SEQ ID NO | 106 |
| | CDR-L3 | SEQ ID NO | 107 | SEQ ID NO | 108 |
| | Heavy chain variable region (VH) | SEQ ID NO | 109 | SEQ ID NO | 110 |
| | Light chain variable region (VL) | SEQ ID NO | 111 | SEQ ID NO | 112 |
| GPN18-1 | CDR-H1 | SEQ ID NO | 113 | SEQ ID NO | 114 |
| | CDR-H2 | SEQ ID NO | 115 | SEQ ID NO | 116 |
| | CDR-H3 | SEQ ID NO | 117 | SEQ ID NO | 118 |
| | CDR-L1 | SEQ ID NO | 119 | SEQ ID NO | 120 |
| | CDR-L2 | SEQ ID NO | 121 | SEQ ID NO | 122 |
| | CDR-L3 | SEQ ID NO | 123 | SEQ ID NO | 124 |
| | Heavy chain variable region (VH) | SEQ ID NO | 125 | SEQ ID NO | 126 |
| | Light chain variable region (VL) | SEQ ID NO | 127 | SEQ ID NO | 128 |
| GPN18-2 | CDR-H1 | SEQ ID NO | 129 | SEQ ID NO | 130 |
| | CDR-H2 | SEQ ID NO | 131 | SEQ ID NO | 132 |
| | CDR-H3 | SEQ ID NO | 133 | SEQ ID NO | 134 |
| | CDR-L1 | SEQ ID NO | 135 | SEQ ID NO | 136 |
| | CDR-L2 | SEQ ID NO | 137 | SEQ ID NO | 138 |
| | CDR-L3 | SEQ ID NO | 139 | SEQ ID NO | 140 |
| | Heavy chain variable region (VH) | SEQ ID NO | 141 | SEQ ID NO | 142 |
| | Light chain variable region (VL) | SEQ ID NO | 143 | SEQ ID NO | 144 |

**[Table 1-4]**

| Antibody clone | Description | Amino acid sequence | | Nucleic acid sequence | |
|---|---|---|---|---|---|
| GPN18-6 | CDR-H1 | SEQ ID NO | 145 | SEQ ID NO | 146 |
| | CDR-H2 | SEQ ID NO | 147 | SEQ ID NO | 148 |
| | CDR-H3 | SEQ ID NO | 149 | SEQ ID NO | 150 |
| | CDR-L1 | SEQ ID NO | 151 | SEQ ID NO | 152 |
| | CDR-L2 | SEQ ID NO | 153 | SEQ ID NO | 154 |
| | CDR-L3 | SEQ ID NO | 155 | SEQ ID NO | 156 |
| | Heavy chain variable region (VH) | SEQ ID NO | 157 | SEQ ID NO | 158 |
| | Light chain variable region (VL) | SEQ ID NO | 159 | SEQ ID NO | 160 |
| GPN18-7 | CDR-H1 | SEQ ID NO | 161 | SEQ ID NO | 162 |
| | CDR-H2 | SEQ ID NO | 163 | SEQ ID NO | 164 |
| | CDR-H3 | SEQ ID NO | 165 | SEQ ID NO | 166 |
| | CDR-L1 | SEQ ID NO | 167 | SEQ ID NO | 168 |
| | CDR-L2 | SEQ ID NO | 169 | SEQ ID NO | 170 |
| | CDR-L3 | SEQ ID NO | 171 | SEQ ID NO | 172 |
| | Heavy chain variable region (VH) | SEQ ID NO | 173 | SEQ ID NO | 174 |
| | Light chain variable region (VL) | SEQ ID NO | 175 | SEQ ID NO | 176 |
| GPN18-5 | CDR-H1 | SEQ ID NO | 177 | SEQ ID NO | 178 |
| | CDR-H2 | SEQ ID NO | 179 | SEQ ID NO | 180 |
| | CDR-H3 | SEQ ID NO | 181 | SEQ ID NO | 182 |
| | CDR-L1 | SEQ ID NO | 183 | SEQ ID NO | 184 |
| | CDR-L2 | SEQ ID NO | 185 | SEQ ID NO | 186 |
| | CDR-L3 | SEQ ID NO | 187 | SEQ ID NO | 188 |
| | Heavy chain variable region (VH) | SEQ ID NO | 189 | SEQ ID NO | 190 |
| | Light chain variable region (VL) | SEQ ID NO | 191 | SEQ ID NO | 192 |

**[Table 1-5]**

| Antibody clone | Description | Amino acid sequence | | Nucleic acid sequence | |
|---|---|---|---|---|---|
| 1-5E | CDR-H1 | SEQ ID NO | 193 | SEQ ID NO | 194 |
| | CDR-H2 | SEQ ID NO | 195 | SEQ ID NO | 196 |
| | CDR-H3 | SEQ ID NO | 197 | SEQ ID NO | 198 |
| | CDR-L1 | SEQ ID NO | 199 | SEQ ID NO | 200 |
| | CDR-L2 | SEQ ID NO | 201 | SEQ ID NO | 202 |
| | CDR-L3 | SEQ ID NO | 203 | SEQ ID NO | 204 |
| | Heavy chain variable region (VH) | SEQ ID NO | 205 | SEQ ID NO | 206 |
| | Light chain variable region (VL) | SEQ ID NO | 207 | SEQ ID NO | 208 |
| GPN09-1 | CDR-H1 | SEQ ID NO | 287 | SEQ ID NO | 288 |
| | CDR-H2 | SEQ ID NO | 289 | SEQ ID NO | 290 |
| | CDR-H3 | SEQ ID NO | 291 | SEQ ID NO | 292 |
| | CDR-L1 | SEQ ID NO | 293 | SEQ ID NO | 294 |
| | CDR-L2 | SEQ ID NO | 295 | SEQ ID NO | 296 |
| | CDR-L3 | SEQ ID NO | 297 | SEQ ID NO | 298 |
| | Heavy chain variable region (VH) | SEQ ID NO | 299 | SEQ ID NO | 300 |
| | Light chain variable region (VL) | SEQ ID NO | 301 | SEQ ID NO | 302 |

### [Example 31 Analysis of the binding activity of the anti-gpNMB antibodies to microglia (cell ELISA):

To evaluate the binding ability of each anti-gpNMB antibody obtained in Example 1 to microglia, brain cells were harvested from 1- to 2-day-old mouse or rat brains, cultured for about 1 week, and mouse or rat microglia were collected by shaking the adherent cells for 1 hour to collect detached cells. The collected mouse or rat microglia were suspended in 20% FBS/DMEM-GlutaMax, seeded into 96-well plates at 2×10⁴cell /well, cultured in a CO₂ incubator at 37°C for 1 to 2 days for attachment, fixed with Mild form, and then blocked with 3% BSA/PBS to prepare a cell ELISA plate.

After the blocking solution was removed from the mouse or rat microglia-fixed ELISA plate, a diluent of the anti-gpNMB antibody (3% BSA/PBS solution) was added at 30 µL/well, and allowed to react for 1 hour at room temperature. Then, after washing with washing buffer, an antibody that specifically reacts with mouse IgG, i.e., either anti-mouse IgG antibody-HRP conjugate (MBL, Code No. 330) at 50 µL/well or an anti-mouse IgG antibody-ALP conjugate (SBA (SBA, Code No. 1050-04) at 30 µL/well, was added and allowed to react for 1 hour at room temperature. Substrate for HRP (ELISA-Star^{™} peroxidase chemiluminescent substrate, FUJIFILM Wako Pure Chemicals Corporation) was added at 100 µL/well, and the luminescence intensity was immediately measured for 1 to 10 seconds, and the value of luminescence intensity obtained was evaluated as binding activity. Alternatively, substrate for ALP (PNPP) was added at 100 µL/well and reacted at room temperature for 1 hour, and the absorbance at 405 to 550 nm was measured and evaluated as binding activity. The results confirmed the binding activity of the anti-gpNMB antibodies to mouse and rat microglia.

### [Example 41 Analysis of the binding sites of the anti-gpNMB antibodies (antigen ELISA):

To analyze the binding sites of each anti-gpNMB antibody obtained in Example 1 to the human gpNMB protein, the following experiment was performed.

First, based on the amino acid sequence of the human gpNMB extracellular domain (SEQ ID NO 215, in which the amino acid residues at positions 1 to 21 constitute a signal peptide, while the amino acid residues at positions 22 and onward constitute an isolated peptide of the human gpNMB extracellular domain), an N-terminal deletion variant (hgpNMB_d07_76-498) and C-terminal deletion variants (hgpNMB_d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB_d19_1-234) of the human gpNMB extracellular domain were produced as shown in Table 2 below. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein.

**[Table 2]**

| Table 2: N-terminal or C-terminal deletion variants of human gpNMB extracellular domain | |
|---|---|
| Variant No. | Description |
| hgpNMB_d03_1-251 | C-terminal deletion variant consisting of 1 to 251 of hgpNMB |
| hgpNMB_d04_1-321 | C-terminal deletion variant consisting of 1 to 321 of hgpNMB |
| hgpNMB_d05_1-375 | C-terminal deletion variant consisting of 1 to 375 of hgpNMB |
| hgpNMB_d06_1-418 | C-terminal deletion variant consisting of 1 to 418 of hgpNMB |
| hgpNMB_d07_76-498 | N-terminal deletion variant consisting of 76 to 498 of hgpNMB |
| hgpNMB_d13_1-412 | C-terminal deletion variant consisting of 1 to 412 of hgpNMB |
| hgpNMB_d14_1-406 | C-terminal deletion variant consisting of 1 to 406 of hgpNMB |
| hgpNMB_d15_1-400 | C-terminal deletion variant consisting of 1 to 400 of hgpNMB |
| hgpNMB_d16_1-394 | C-terminal deletion variant consisting of 1 to 394 of hgpNMB |
| hgpNMB_d17_1-388 | C-terminal deletion variant consisting of 1 to 388 of hgpNMB |
| hgpNMB_d18_1-382 | C-terminal deletion variant consisting of 1 to 382 of hgpNMB |
| hgpNMB_d19_1-234 | C-terminal deletion variant consisting of 1 to 234 of hgpNMB |

Next, 17 nmol/L of each of purified recombinant human soluble gpNMB (SEQ ID NO 221), the N-terminal deletion variant (hgpNMB_d07_76-498) and the C-terminal deletion variants (hgpNMB d03_1-251, hgpNMB_d04_1-321, hgpNMB_d05_1-375, hgpNMB_d06_1-418, hgpNMB_d13_1-412, hgpNMB_d14_1-406, hgpNMB_d15_1-400, hgpNMB_d16_1-394, hgpNMB_d17_1-388, hgpNMB_d18_1-382, and hgpNMB_d19_1-234) of the human gpNMB extracellular domain shown in Table 2 above, and purified recombinant mouse soluble gpNMB (SEQ ID NO 223) was added to a 96-well plate for ELISA at 100 µL/well, and allowed to stand at room temperature for 90 minutes. The plate was then blocked with 3% BSA/PBS to thereby obtain an antigen ELISA plate.

After the blocking solution was removed from the antigen ELISA plate, a diluent of the anti-gpNMB antibody (3% BSA/PBS solution) was added at 50 µL/well, and allowed to react for 1 hour at room temperature. Then, after washing with washing buffer, an antibody that specifically reacts with mouse IgG, anti-mouse IgG antibody-ALP conjugate (SBA, 1050-04), was added at 50 µL/well and allowed to react for 1 hour at room temperature. Substrate (PNPP) was added at 100 µL/well and allowed to react for 1 hour at room temperature, and the absorbance at 405 to 550 nm was measured and evaluated as binding activity.

### [Example 51 Determination of the epitopes of the anti-gpNMB antibodies (antigen ELISA):

To determine the epitope of each anti-gpNMB antibody obtained in Example 1 in the human gpNMB protein, the following experiment was performed.

First, a C-terminal deletion variant (hgpNMB_d19_1-234) and point mutants (hgpNMB_d22_K245A, hgpNMB_d23_D252A, hgpNMB_d24_E253A, hgpNMB_d25_D264A, hgpNMB_d26_H272A, hgpNMB_d28_H297A, hgpNMB_d30_H376A, hgpNMBd31_D247A_R248A, hgpNMB_d34_D287A, hgpNMB_d35_H301A, hgpNMB_d36_R331A_K334A, hgpNMB_d37_K344A_D347A, hgpNMB_d38_D356A, hgpNMB_d39_E360A, hgpNMB_d40_E367A, and hgpNMB_d41_R373A) of human gpNMB extracellular domain as shown in Table 3 below were produced. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 3.

**[Table 3]**

| Table 3: C-terminal deletion variants and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d22_K245A | K245A point mutant of hgpNMB |
| hgpNMB_d23_D252A | D252A point mutant of hgpNMB |
| hgpNMB_d24_E253A | E253A point mutant of hgpNMB |
| hgpNMB_d25_D264A | D264A point mutant of hgpNMB |
| hgpNMB_d26_H272A | H272A point mutant of hgpNMB |
| hgpNMB_d28_H297A | H297A point mutant of hgpNMB |
| hgpNMB_d30_H376A | H376A point mutant of hgpNMB |
| hgpNMBd31_D247A_R248A | D247A and R248A point mutant of hgpNMB |
| hgpNMB_d34_D287A | D287A point mutant of hgpNMB |
| hgpNMB_d35_H301A | H301A point mutant of hgpNMB |
| hgpNMB_d36R331A_K334A | R33 1A and K334A point mutant of hgpNMB |
| hgpNMB_d37_K344A_D347A | K344A and D347A point mutant of hgpNMB |
| hgpNMB_d38_D356A | D356A point mutant of hgpNMB |
| hgpNMB_d39_E360A | E360A point mutant of hgpNMB |
| hgpNMB_d40_E367A | E367A point mutant of hgpNMB |
| hgpNMB_d41_R373A | R373A point mutant of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 2 above was added to a 96-well plate for ELISA at a concentration of 17 nmol/L at 100 µL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 µL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 µL/well and allowed to react for 1 hour at room temperature. Substrate (PNPP) was added at 100 µL/well and reacted at room temperature for 1 hour, and the absorbance at 405 to 550 nm was calculated and evaluated as binding activity. Compared to the mutant that showed the maximum absorbance at 2 nM concentration, mutants that showed a decrease in absorbance of 50% or more are marked as +, and those that showed an absorbance of 50% or more are marked as -. The results are shown in Table 4.

**[Table 4]**

| Table 4: Results of ELISA binding analysis of each anti-gpNMB antibody clone to human gpNMB extracellular domain variants | | | | | | | | |
|---|---|---|---|---|---|---|---|---|
| | GPN 05-1 | GPN 06-1 | GPN 07-1 | GPN 09-1 | GPN 11-10 | GPN 15-2 | GPN 15-3 | GPN 18-4 |
| 22-234_N terminal | - | - | - | - | - | - | - | - |
| K245A | - | - | - | - | - | - | - | - |
| D252A | - | - | - | - | - | - | - | - |
| E253A | - | - | - | - | - | - | - | - |
| K257A,D258A | - | - | - | + | + | - | - | - |
| D264A | - | - | - | - | - | - | - | - |
| H268A,D269A | - | - | + | - | - | - | - | - |
| H272A | - | - | - | - | - | - | - | - |
| K282A | - | + | + | - | - | - | - | - |
| H297A | - | - | - | - | - | - | - | - |
| H376A | - | - | - | - | - | - | - | - |
| D247A,R248A | - | - | - | - | - | - | - | - |
| **D287A** | + | + | + | + | + | + | + | + |
| **H301A** | + | + | + | + | + | + | + | + |
| K316A | - | + | + | - | - | - | - | - |
| R331A,K334A | - | - | - | - | - | - | - | - |
| K344A,D347A | - | - | - | - | - | - | - | - |
| D356A | - | - | - | - | - | - | - | - |
| E360A | - | - | - | - | - | - | - | - |
| E367A | - | - | - | - | - | - | - | - |
| R373A | - | - | - | - | - | - | - | - |
| E385A | - | - | - | + | + | - | - | - |

In addition, the anti-gpNMB antibodies that were bound to a C-terminal deletion mutant of the human gpNMB extracellular domain (hgpNMB d19_1-234) were subjected to analysis for binding ability to alanine point mutants of N-terminal human gpNMB extracellular domain using antigen ELISA in a similar manner.

First, a C-terminal deletion variant (hgpNMB_d19_1-234) and point mutants (hgpNMB d19_1-234, hgpNMB d43_1-234 R77A, hgpNMB d44_1-234 D85A, hgpNMB_d45_1-234_R104A, hgpNMB_d47_1-234_E117A,K118A, hgpNMB_d48_1-234 R121A,E123A, hgpNMB d49_1-234 D129A, hgpNMB d50_1-234 E140A,D141A, hgpNMB_d51_1-234_D143A,E145A, hgpNMB_d52_1-234_H152A,H153A, hgpNMB_d53_1 -234_D158A,K160A, hgpNMB_d54_1 -234_H164A,H165A, hgpNMB_d55_1-234_R169A, hgpNMB_d58_1-234_R189A, hgpNMB_d59_1-234_R193A, hgpNMB_d61_1-234_R214A,R215A, and hgpNMB_d64_1-234_D109A) of human gpNMB extracellular domain shown in Table 5 below were generated. Each purified gpNMB protein was obtained by constructing a transient animal cell expression vector into which each gpNMB gene had been inserted, and having it expressed in a medium using the Expi293 Expression System (A14635, Thermo Fisher Scientific). The expressed protein was then purified based on affinity with Ni-NTA agarose (143-09763, FUJIFILM Wako) using a histidine tag added to the C-terminus to thereby obtain the purified protein. The C-terminal deletion variant and the point mutants of human gpNMB extracellular domain are summarized in Table 5.

**[Table 5]**

| Table 5: C-terminal deletion variant and point mutants of human gpNMB extracellular domain | |
|---|---|
| Variant No. | Description |
| hgpNMB_d19_1-234 | C-terminal deletion variant consisting of 1 to 234 of hgpNMB |
| hgpNMB_d43_1-234_R77A | R77A point mutant of 1 to 234 of hgpNMB |
| hgpNMB_d44_1-234_D85A | D85A point mutant of 1 to 234 of hgpNMB |
| hgpNMB_d45_1-234_R104A | R104A point mutant of 1 to 234 of hgpNMB |
| hgpNMB_d47_1-234_E117A,K118A | E117A and K118A point mutant of 1 to 234 of hgpNMB |
| hgpNMB_d48_1-234_R121A,E123A | R121A and E123A point mutant of 1 to 234 of hgpNMB |
| hgpNMB_d49_1-234_D129A | D129A point mutant of 1 to 234 of hgpNMB |
| hgpNMB_d50_1-234_E140A,D141A | E140A and D141A point mutant of 1 to 234 of hgpNMB |
| hgpNMB_d51_1-234_D143A,E145A | D143A and E145A point mutant of 1 to 234 of hgpNMB |
| hgpNMB_d52_1-234_H152A,H153A | H152A and H153A point mutant of 1 to 234 of hgpNMB |
| hgpNMB_d53_1-234_D158A,K160A | D158A and K160A point mutant of 1 to 234 of hgpNMB |
| hgpNMB_d54_1-234_H164A,H165A | H164A and H165A point mutant of 1 to 234 of hgpNMB |
| hgpNMB_d55_1-234_R169A | R169A point mutant of 1 to 234 of hgpNMB |
| hgpNMB_d58_1-234_R189A | R189A point mutant of 1 to 234 of hgpNMB |
| hgpNMB_d59_1-234_R193A | R193A point mutant of 1 to 234 of hgpNMB |
| hgpNMB_d61_1-234_R214A,R215A | R214A and R215A point mutant of 1 to 234 of hgpNMB |
| hgpNMB_d64_1 -234_D109A | D109A point mutant of 1 to 234 of hgpNMB |

These alanine point mutants of human gpNMB extracellular domain were subjected to antigen ELISA analysis using the same procedure explained in Example 4 to evaluate the binding ability of each anti-gpNMB antibody. Specifically, each of the C-terminal deletion variant and the alanine point mutants of human gpNMB extracellular domain shown in Table 5 above was added to a 96-well plate for ELISA at a concentration of 17 nmol/L at 100 µL/well and allowed to stand at room temperature for 90 minutes. The antigen ELISA plate was then blocked with 3% BSA/PBS to obtain an antigen ELISA plate.

After the blocking solution was removed from the antigen ELISA plate, a diluent of the anti-gpNMB antibody (3% BSA/PBS solution) was added at 50 µL/well and allowed to react for 1 hour at room temperature. Then, after washing with washing buffer, an antibody that specifically reacts with mouse IgG, i.e., an anti-mouse IgG antibody-ALP conjugate (SBA, 1050-04), was added at 50 µL/well and allowed to react for 1 hour at room temperature. Substrate (PNPP) was added at 100 µL/well and reacted at room temperature for 1 hour, and the absorbance at 405 to 550 nm was calculated and evaluated as binding activity. The results are shown in Table 6 (As mentioned above, in the amino acid sequence shown in SEQ ID NO 215, the amino acid residues at positions 1 to 21 constitute a signal peptide, while the amino acid residues at positions 22 and onward constitute an isolated peptide of the human gpNMB extracellular domain. Accordingly, each mutant protein shown in Table 6 starts at amino acid residue at position 22.). The binding activity to the C-terminal deletion mutant (hgpNMB_d19_22-234) is set as 100%, mutants that showed an absorbance of 50% or less are marked as +, and those that showed an absorbance of 50% or more are marked as -.

**[Table 6]**

| Table 6: Results of ELISA binding analysis of each anti-gpNMB antibody clone to human gpNMB extracellular domain variants | | | | | |
|---|---|---|---|---|---|
| gpNMB variant protein | GPN 18-1 | GPN 18-2 | GPN 18-5 | GPN 18-6 | GPN 18-7 |
| 22-234_N terminal | - | - | - | - | - |
| 22-234_R77A | - | - | - | - | - |
| 22-234_D85A | - | - | + | - | - |
| 22-234_R104A | - | - | - | - | - |
| 22-234_D109A | - | - | - | - | - |
| 22-234_E117A,K118A | - | - | - | - | - |
| 22-234_R121A,E123A | - | - | - | - | - |
| 22-234_D129A | - | - | - | - | - |
| 22-234_E140A,D141A | - | - | - | - | - |
| 22-234_D143A,E145A | - | - | - | - | - |
| 22-234_H152A,H153A | - | - | - | - | - |
| 22-234_D158A,K160A | - | - | - | - | - |
| 22-234_H164A,H165A | - | - | - | - | - |
| 22-234_R169A | - | - | - | - | - |
| 22-234_K186A | - | - | - | + | - |
| 22-234_R189A | - | - | - | - | - |
| 22-234_R193A | - | - | - | - | - |
| 22-234_R214A,R215A | + | + | - | + | + |
| 22-234_H216A, R218A | + | + | - | - | + |

The epitope of each anti-gpNMB antibody was determined by considering the stability of the purified gpNMB mutant and the mutation sites that affected the binding.

The results showed that antibody clones GPN18-1, GPN18-2, GPN18-6, and GPN18-7 interact with amino acids present in the PMEL-CAF-like region (R214 and/or R215). On the other hand, antibody clone GPN18-5 was found to further recognize the N-terminal portion. Antibody clone 1-5E is an anti-gpNMB antibody obtained by immunization with the vaccine peptide identified in a previous report (Non-Patent Literature 36), and the epitope recognized by this antibody is "RRGDGRWKD" at amino acid residues 63 to 71 at the N-terminus.

### [Example 61 Production of recombinant anti-gpNMB antibodies:

Plasmids were prepared by adding a gene sequence encoding a signal sequence to the 5' end of each of a gene sequence encoding the H chain and a gene sequence encoding the L chain of the anti-gpNMB antibody obtained in Example 2, and inserting each of the gene sequences into the animal cell expression vector pcDNA3.4. The prepared plasmids were then transiently expressed using the ExpiCHO Expression System (A29133, Thermo Fisher Scientific) such that the antibodies were secreted into the medium. The culture supernatant was then collected and purified using a Protein A column (Ab-Capture, Protenova) and a gel filtration column (Superdex 200 Increase, Cytiva).

### [Example 7(1)1 Interaction analysis of the anti-gpNMB antibodies by surface plasmon resonance method - 1

To compare the binding properties (binding and dissociation kinetics) of each anti-gpNMB antibody clone obtained in Example 1 to gpNMB, surface plasmon resonance (SPR) measurement was performed. Specifically, the following conditions were used.

The BIACORE T200 system was used as the measurement system. An anti-His tag monoclonal antibody was fixed at around 5000 RU in all flow cells of the sensor chip CM5 (BR-1005-30, Cytiva) with the Amine Coupling Kit (BR-1000-50, GE) and His Capture Kit (28 -9950-56, Cytiva). The running buffer used was HBS-EP+ (BR-1006-69, Cytiva).

Recombinant human gpNMB-FLAG-His (SEQ ID NO 221) or recombinant mouse gpNMB-FLAG-His (SEQ ID NO 223) was captured in the measurement system and used as the ligand. Each anti-gpNMB antibody clone was used at a concentration of 100 nmol/L as an analyte. Shiga toxin 2 (Shiga toxin 2) antibody (11E10) or control mouse IgG1 (leinco: Pro# M1411) was used as an analyte negative control.

The temperature of the measurement system was set at 25°C. As the ligand, recombinant human gpNMB-FLAG-His was reacted with the anti-His monoclonal antibody in the flow cell (2) so as to achieve 100 RU or lower, and recombinant mouse gpNMB-FLAG-His was reacted with the anti-His monoclonal antibody in the flow cell (4) so as to achieve 100 RU or lower. The flow rate was set to 20 µL/min, and 10 nmol/L of purified mouse IgG2a, κ, Isotype Ctrl, Clone: MG2a-53 (401502, BioLegend, hereinafter ctrl IgG2a) was reacted for 1 min, and HBS-EP+ was flowed for at least 10 minutes. The analyte was diluted with HBS-EP+ (100 nmol/L) and reacted with all flow cells for 600 seconds each to obtain a binding curve, and then reacted with HBS-EP+ for 600 seconds to obtain a dissociation curve.

After the reaction, regeneration buffer 1 (0.2% SDS), regeneration buffer 2 (100 mmol/L Tris-HCl (pH 8.5), 1 mol/L NaCl, 15 mmol/L MgCl₂), and regeneration buffer 3 (10 mmol/L glycine -HCl (pH 1.5)) were reacted for 1 minute each to remove and wash gpNMB-FLAG-His in the measurement system. Biacore T200 Evaluation software (ver. 2.0) was used to analyze the data with the 1:1 Binding Model, and the binding rate value (Ass: 1/Ms), binding stability value (Diss: 1/s) and equilibrium dissociation value (Diss/Ass: M) were quantified as ordinal numbers. The results are shown in Table 7.

**[Table 7]**

| Table 7: Results of SPR interaction analysis of each anti-gpNMB antibody clone against human gpNMB and mouse gpNMB - 1 | | | | | | | |
|---|---|---|---|---|---|---|---|
| Antibody clone | | Human gpNMB | | | Mouse gpNMB | | |
| Sample | Recognized domain | Ass. (1/Ms) | Diss (1/s) | Diss/Ass (M) | Ass (1/Ms) | Diss (1/s) | Diss/Ass (M) |
| GPN05-1 | PKD | 4.8.E+04 | 1.6.E-04 | 3.3.E-09 | 6.1.E+04 | 1.3.E-03 | 2.0.E-08 |
| GPN06-1 | PKD | 3.4.E+05 | 4.9.E-04 | 1.4.E-09 | 1.5.E+05 | 1.8.E-03 | 1.2.E-08 |
| GPN09-1 | PKD | - | - | - | 1.6.E+05 | 4.2.E-04 | 2.6.E-09 |
| GPN11-10 | PKD | 8.1.E+04 | 1.0.E-03 | 1.3.E-08 | 7.5.E+04 | 3.7.E-04 | 4.9.E-09 |
| GPN15-2 | PKD | 1.7.E+05 | 6.0.E-07 | 3.5.E-12 | 1.4.E+05 | 2.8.E-04 | 2.0.E-09 |
| GPN15-3 | PKD | 1.3.E+05 | 3.7.E-04 | 2.9.E-09 | 1.3.E+05 | 3.6.E-04 | 2.9.E-09 |
| GPN18-4 | PKD | 4.7.E+04 | 1.5.E-05 | 3.2.E-10 | 3.0.E+04 | 1.2.E-04 | 3.8.E-09 |
| GPN18-1 | PMEL -CAF-like | 3.8.E+04 | 5.4.E-04 | 1.4.E-08 | 3.8.E+04 | 9.1.E-04 | 2.4.E-08 |
| GPN18-2 | PMEL -CAF-like | 3.1.E+04 | 6.8.E-05 | 2.2.E-09 | 2.4.E+04 | 7.9.E-05 | 3.3.E-09 |
| GPN18-6 | PMEL -CAF-like | 2.7.E+04 | 6.5.E-04 | 2.4.E-08 | 3.2.E+04 | 9.8.E-04 | 3.1.E-08 |
| GPN18-7 | PMEL -CAF-like | 2.6.E+04 | 5.9.E-04 | 2.3.E-08 | 2.0.E+04 | 9.9.E-04 | 4.9.E-08 |
| GPN18-5 | N-frg. | 1.5.E+05 | 3.0.E-04 | 2.0.E-09 | 9.1.E+04 | 7.0.E-04 | 7.7.E-09 |
| 1-5E | N-terminal vaccine | - | - | - | 6.7.E+05 | 6.1.E-03 | 9.1.E-09 |

### [Example 7(2)1 Interaction analysis of the anti-gpNMB antibodies by surface plasmon resonance method - 2

Two of the anti-gpNMB antibody clones obtained in Example 1, GPN05-1 and GPN06-1, were subjected to measurement for concentration-dependent binding properties (binding and dissociation kinetics) to gpNMB by surface plasmon resonance (SPR) for investigation. Specifically, the same conditions as in Example 7(1) were used except for the conditions described below.

The sensor chip CM3 (BR-1005-36, Cytiva) was used, and anti-gpNMB antibody clones GPN05-1 and GPN06-1 were used as analytes at various concentrations each. The measurement conditions were determined in accordance with the single-cycle kinetics method. The analyte at each concentration was reacted for 450 seconds to obtain a binding curve, and HBS-EP+ was reacted for 600 seconds to obtain a dissociation curve. After the reaction, regeneration buffer 1 (0.2% SDS), regeneration buffer 2 (100 mmol/L Tris-HCl (pH 8.5), 1 mol/L NaCl, 15 mmol/L MgCl₂), and regeneration buffer 3 (10 mmol/L glycine -HCl (pH 1.5)) were reacted for 1 minute each to remove and wash gpNMB-FLAG-His in the measurement system. Biacore T200 Evaluation software (ver. 2.0) was used to analyze the data with the 1:1 Binding Model, and the binding rate value (Ass: 1/Ms), binding stability value (Diss: 1/s) and equilibrium dissociation value (Diss/Ass: M) were calculated. The results are shown in Table 8.

**[Table 8]**

| Table 8: Results of SPR interaction analysis of each anti-gpNMB antibody clone against human gpNMB and mouse gpNMB - 2 | | | | |
|---|---|---|---|---|
| Ligand | Clone | ka (1/Ms) | kd (1/s) | KD (M) |
| Human gpNMB | GPN05-1 | 9.69E+04 | 3.55E-05 | 3.66E-10 |
| | GPN06-1 | 5.19E+05 | 2.53E-04 | 4.88E-10 |
| Mouse gpNMB | GPN05-1 | 3.61E+04 | 3.07E-04 | 8.50E-09 |
| | GPN06-1 | 1.74E+05 | 8.79E-04 | 5.05E-09 |

### [Example 81 Binding analysis of anti-gpNMB antibody (commercial polyclonal antibody) in Alzheimer's disease model mice

APPosk mice aged 21 to 25 months were reflux-fixed, brains were excised, and 5-µm-thick paraffin sections were prepared. After deparaffinization, the brain sections were boiled in citrate buffer (pH 6) for 30 minutes to activate the antigen, and then double-stained with commercially available anti-gpNMB polyclonal antibody AF2330 (2 µg/mL) + FITC-donkey anti-goat IgG antibody (Jackson Lab) and Iba1 antibody (WAKO, 5 µg/mL) + Rhodamine-donkey anti-rabbit IgG antibody (Jackson Lab). After treatment with lipofuscin quencher TrueBlackplus (Biotium) for 10 minutes at room temperature, the sections were encapsulated with DAPI-containing encapsulant (Vector).

The results showed that the brain sections from the APPosk mice aged 21 to 25 months included cells stained with the anti-gpNMB antibody (AF2330, R&D) among Iba1-positive microglia, and that almost all gpNMB-positive cells were Iba1-positive microglia. The results also showed that the microglia that underwent morphological changes were gpNMB-positive microglia. These findings confirm that gpNMB-positive microglia appear in the Alzheimer's disease model mice (APPosk mice).

### [Example 91 Reduction of gpNMB-positive microglia by administration of anti-gpNMB antibodies (GPN05-1 and GPN06-1) to Alzheimer's disease model mice:

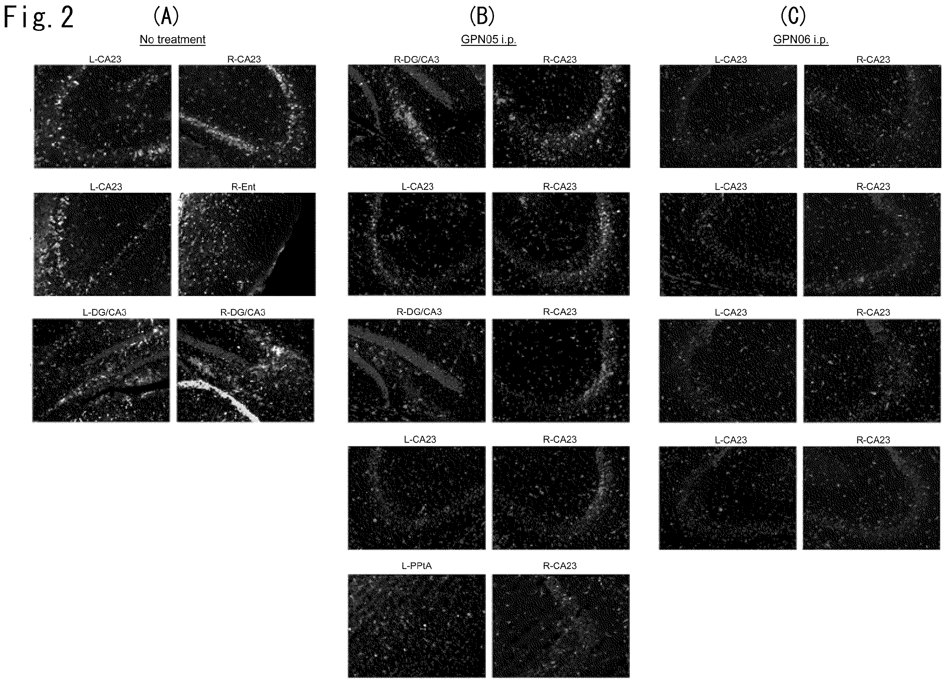

APPosk mice aged 19 to 20 months were divided into two groups (two males and three females in each group), and each group was intraperitoneally injected with a different anti-gpNMB antibody clone obtained in Example 1 (GPN05-1 or GPN06-1) once a week for a total of five times (1 mg/400 µL in PBS each time). Four days after the last dose, mice were reflux-fixed, brains were excised, and 5-µm-thick paraffin sections were prepared. After deparaffinization, the brain sections were boiled in citrate buffer (pH 6) for 30 minutes to activate the antigen, and then double-stained with anti-mouse gpNMB goat polyclonal antibody (R&D systems, #AF2330, 2 µg/mL) + FITC-donkey anti-goat IgG antibody (Jackson Lab) and Iba1 antibody (WAKO, 5 µg/mL) + Rhodamine-donkey anti-rabbit IgG antibody (Jackson Lab). After treatment with lipofuscin quencher TrueBlackplus (Biotium) for 10 minutes at room temperature, the sections were encapsulated with DAPI-containing encapsulant (Vector). One male in the GPN06-1 group died the week after the fourth dose. As controls, brain sections from APPosk mice aged 21 to 25 months (3 mice) were stained in the same manner. The resulting immunostained photographs are shown in Figure 2. Figure 2(A) shows the control, Figure 2(B) shows the GPN05-1-treated group, and Figure 2(C) shows the GPN06-1-treated group.

The results showed that the control APPosk mice had numerous gpNMB-positive microglia (Iba1-positive) detected with the anti-mouse gpNMB goat polyclonal antibody in the hippocampus (CA2-CA3), entorhinal cortex (Ent) and dentate gyrus (DG). On the other hand, gpNMB-positive microglia were reduced in the GPN05-1-treated group, and were almost absent in the GPN06-1-treated group. These results confirm that administration of the anti-gpNMB monoclonal antibodies GPN05-1 and GPN06-1 reduce gpNMB-positive microglia in the brain.

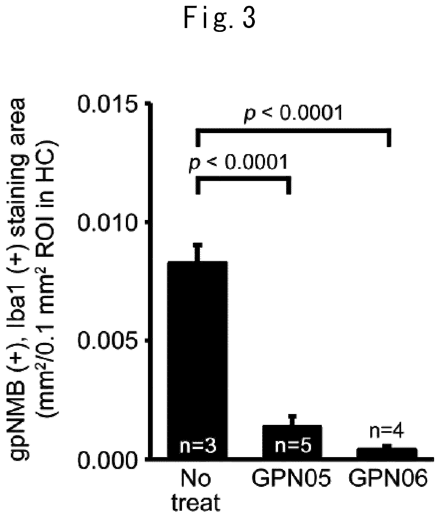

In addition, statistical analysis was performed on the above immunostaining results. Specifically, the staining signal of Iba1 and the co-staining signal of gpNMB and Iba1 were extracted, and the area of each staining region was quantified using ImageJ image processing software. The values obtained were made into a graph as shown in Figure 3. The significant difference test was performed by ANOVA between the three groups using StatView, and only those results that were below the p-value of 0.05 by Fisher's PLSD were considered "significantly different," and their p-values are indicated in the graph.

These analysis results confirm that administration of the anti-gpNMB monoclonal antibodies GPN05-1 and GPN06-1 reduced gpNMB-positive microglia in the brain with statistically significant differences. This means that mal-functional microglia, which account for at least part of gpNMB-positive microglia, were reduced. Such reduction of mal-functional microglia is also supported by the removal of Aβ oligomers as shown in Example 10 and the restoration of the number of synapses as shown in Example 11.

### [Example 101 Removal of Aβ oligomers by administration of anti-gpNMB antibodies (GPN05-1 and GPN06-1) to Alzheimer's disease model mice:

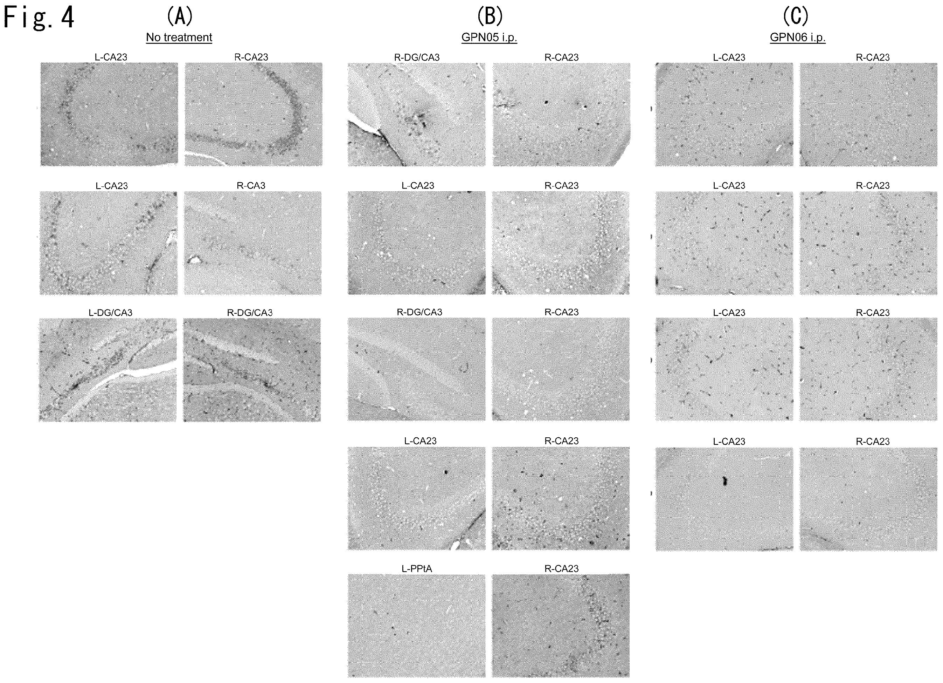

APPosk mice aged 19 to 20 months were divided into two groups (two males and three females in each group), and each group was intraperitoneally administered a different anti-gpNMB antibody obtained in Example 1 (GPN05-1 or GPN06-1) once a week for a total of five times (1 mg/400 µL in PBS each time). Four days after the last dose, the mice were reflux-fixed, brains were excised, and 5-µm-thick paraffin sections were prepared. After deparaffinization, the brain sections were boiled in hydrochloric acid (pH 2) for 10 minutes, and stained with Aβ oligomeric antibody 11A1 (IBL, 1 µg/mL) + Biotin-horse anti-mouse IgG antibody (Vector) + ABC elite (Vector) + DAB. As controls, brain sections from APPosk mice aged 21 to 25 months (3 mice) were stained in the same manner. The resulting immunostained photographs are shown in Figure 4. Figure 4(A) shows the control, Figure 4(B) shows the GPN05-1-treated group, and Figure 4(C) shows the GPN06-1-treated group.

The results showed that Aβ oligomer-positive cells were found in the hippocampus and dentate gyrus in the control APPosk mice. On the other hand, 11A1-positive cells were reduced in the GPN05-1-treated group, and were almost absent in the GPN06-1-treated group. These results confirm that the administration of the anti-gpNMB antibodies removed Aβ oligomers in the brain.

### [Example 111 Restoration of synapses by administration of anti-gpNMB antibodies (GPN05-1 and GPN06-1) to Alzheimer's disease model mice:

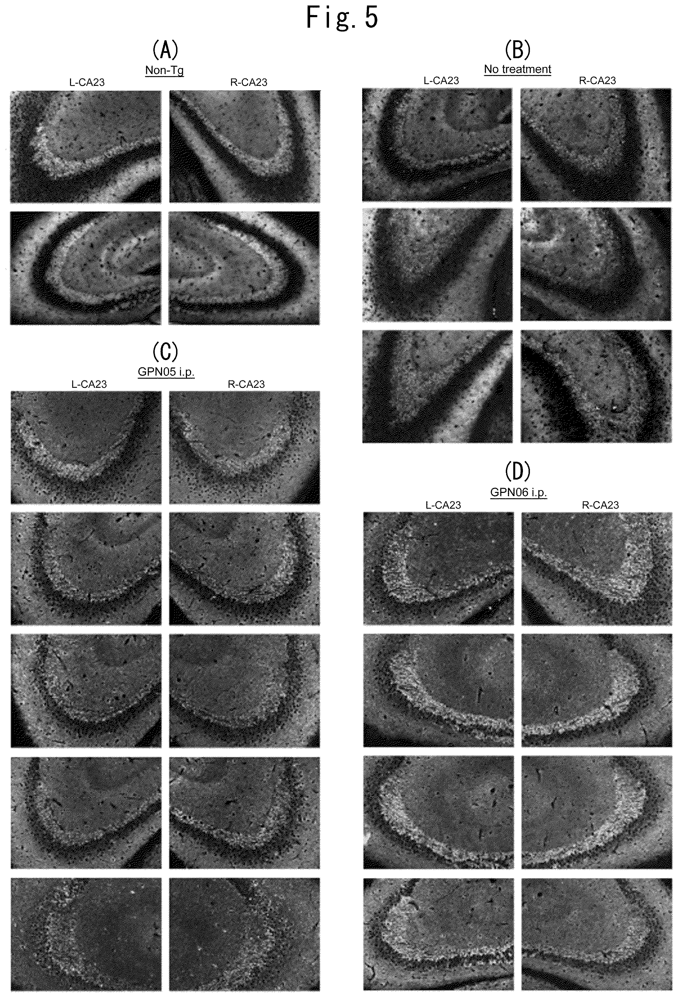

APPosk mice aged 19 to 20 months were divided into two groups (two males and three females in each group), and each group was intraperitoneally administered a different anti-gpNMB antibody obtained in Example 1 (GPN05-1 or GPN06-1) once a week for a total of five times (1 mg/400 µL in PBS each time). Four days after the last dose, the mice were reflux-fixed, brains were excised, and 5-µm-thick paraffin sections were prepared. After deparaffinization, the brain sections were boiled in citrate buffer (pH 6) for 30 minutes, and stained with synaptophysin antibody SVP-38 (Sigma, 200x dilution) + FITC-goat anti-mouse IgG antibody (Jackson Lab). Brain sections from 20-month-old non-transgenic (Non-Tg) mice (2 mice) as Control 1 and brain sections from APPosk mice aged 21 to 25 months (3 mice) as Control 2 were stained in the same manner. The resulting immunostained photographs are shown in Figure 5. Figure 5(A) shows the Control 1 group (Non-Tg mice), Figure 5(B) shows the Control 2 group (APPosk mice), Figure 5(C) shows the GPN05-1-treated group, and Figure 5(D) shows the GPN06-1-treated group.

The results showed that synaptophysin in hippocampal mossy fibers in the Control 2 APPosk mice decreased compared to the Control 1 non-transgenic (Non-Tg) mice. The synaptophysin level recovered slightly in the GPN05-1-treated group and markedly in the GPN06-1-treated group. The recovery of synaptophysin appeared to correlate with the removal of Aβ oligomers. These results suggest that the administration of the anti-gpNMB antibodies increased the number of synapses and restored neural functions in the brains of Alzheimer's disease model mice.

### [Example 12-11 Evaluation of drug efficacy of anti-gpNMB antibodies (GPN09-1, GPN11-10, GPN15-2, GPN15-3, GPN18-2, and 11E10) in Alzheimer's disease model mice (removal of Aβ oligomers)

APPosk mice aged 19-20 months were divided into 6 groups (4 mice in each group), and each group was intraperitoneally administered a different anti-gpNMB antibody obtained in Example 1 (GPN09-1, GPN11-10, GPN15-2, GPN15-3, or GPN18-2) or control antibody 11E10 (anti-Shiga toxin mouse monoclonal antibody), once weekly for a total of 5 times (1 mg/400 µL in PBS for each dose). Four days after the last dose, the mice were reflux-fixed, brains were excised, and 5-µm-thick paraffin sections were prepared. After deparaffinization, the brain sections were boiled in hydrochloric acid (pH 2) for 10 minutes, and stained with Aβ oligomeric antibody 11A1 (IBL, 1 µg/mL) + Biotin-horse anti-mouse IgG antibody (Vector) + ABC elite (Vector) + DAB.

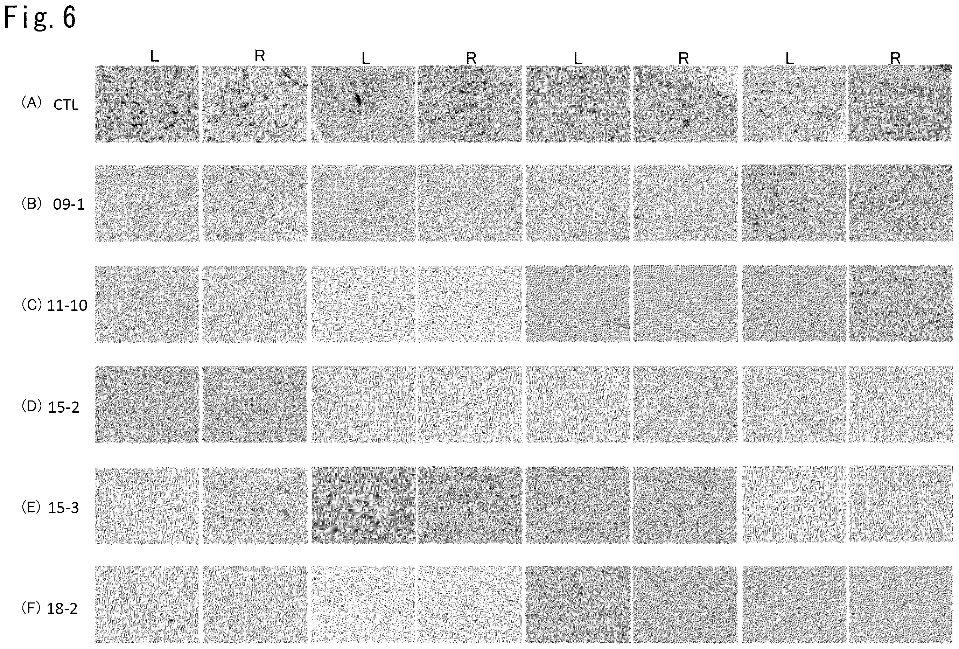

The obtained immunostained photographs are shown in Figure 6. Figure 6(A) shows the Control antibody 11E10-treated group, Figure 6(B) shows the GPN09-1-treated group, Figure 6(C) shows the GPN11-10-treated group, Figure 6(D) shows the GPN15-2-treated group, Figure 6(E) shows the GPN15-3-treated group, and Figure 6(F) shows the GPN18 -2 treated group.

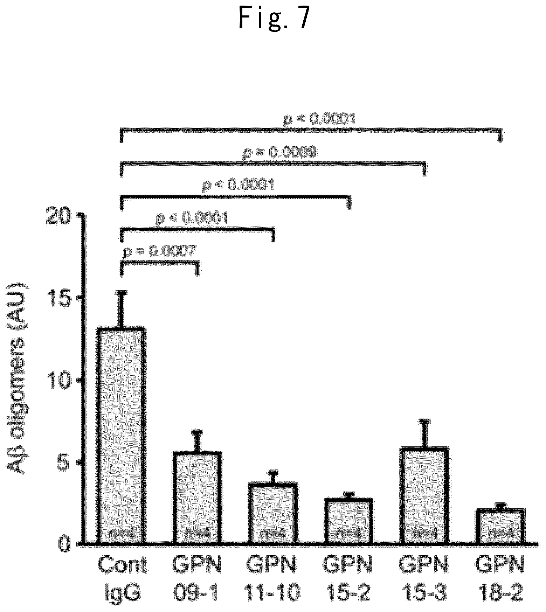

In addition, the intracellular Aβ oligomer staining was quantified for the immunostained photographs in Figure 6, and significance difference test was carried out by ANOVA for the three groups using StatView. The results are shown in the graph in Figure 7. Only the data with p-values below 0.05 by Fisher's PLSD were considered "significantly different," and their p-values are indicated in the graph. The results showed that the APPosk mice in the control antibody-treated group showed 11A1 staining-positive cells in the cerebral cortex. On the other hand, the number of cells positive for 11A1 staining decreased in the GPN09-1-, GPN11-10-, GPN15-2-, and GPN15-3-treated groups, and was almost absent in the GPN18-2-treated group. These results confirm that the administration of the anti-gpNMB antibodies removed Aβ oligomers in the brain.

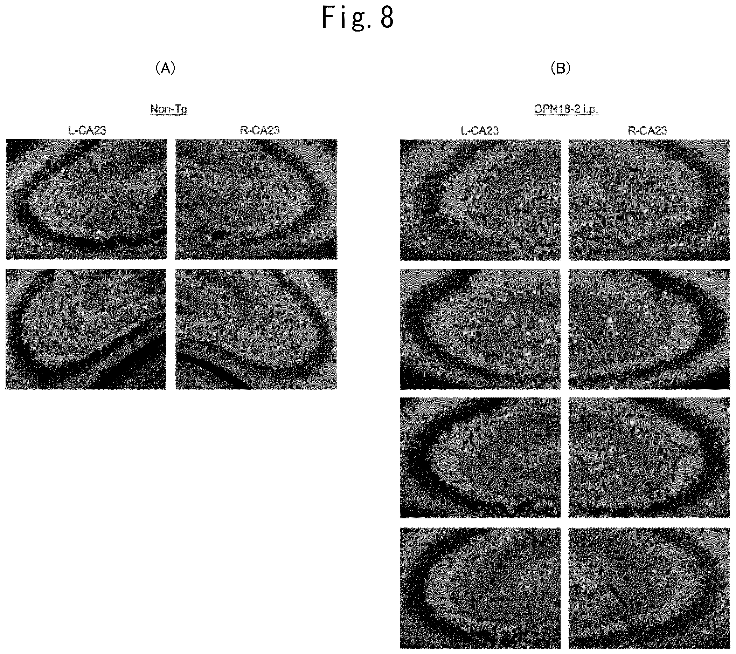

In addition, hippocampal sections from the GPN18-2-treated APPosk mice and those from Non-Tg mice around the same age were subjected to synaptophysin staining according to the method of Example 11. The resulting stained photographs are shown in Figure 8. Figure 8(A) and Figure 8(B) show the control (Non-Tg mice) group and the GPN18-2-treated group, respectively. The results show that the number of synapses in the hippocampus of the GPN18-2-treated APPosk mice was restored to the Non-Tg level.

### [Example 12-2] Evaluation of drug efficacy of anti-gpNMB antibodies (GPN18-2, GPN06-1, GPN18-5, 1-5E, and anti-BTV mouse monoclonal antibody) in Alzheimer's disease model mice (removal of Aβ oligomers)

APPosk mice aged 18 to 20 months were divided into 5 groups (5 mice per group), and each group was treated with a different anti-gpNMB antibody obtained in Example 1 (GPN18-2, GPN06-1, GPN18-5, or 1 -5E) or a control antibody (anti-BTV mouse monoclonal antibody, Leinco Technologies, Inc.) intraperitoneally once a week for a total of five times (1 mg/400 µL in PBS for each dose). Four days after the last dose, mice were reflux-fixed, brains were excised, and 5-µm-thick paraffin sections were prepared. After deparaffinization, the brain sections were boiled in hydrochloric acid (pH 2) for 10 minutes and stained with Aβ oligomeric antibody 11A1 (IBL, 1 µg/mL) + Biotin-horse anti-mouse IgG antibody (Vector) + ABC elite (Vector) + DAB.

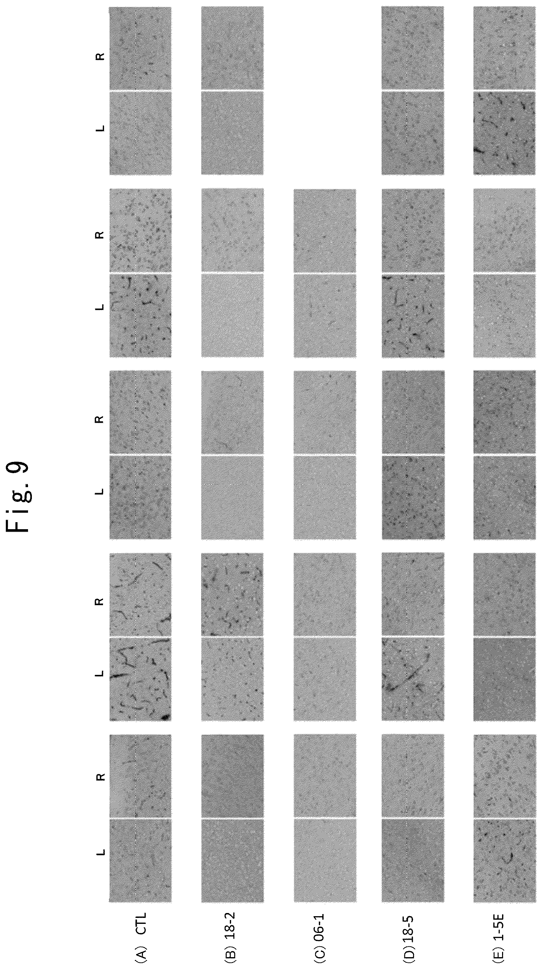

The obtained immunostained photographs are shown in Figure 9. Figure 9(A) shows the Control antibody-treated group, Figure 9(B) shows the GPN18-2-treated group, Figure 9(C) shows the GPN06-1-treated group, Figure 9(D) shows the GPN18-5-treated group, and Figure 9(E) shows the 1-SE-treated group.

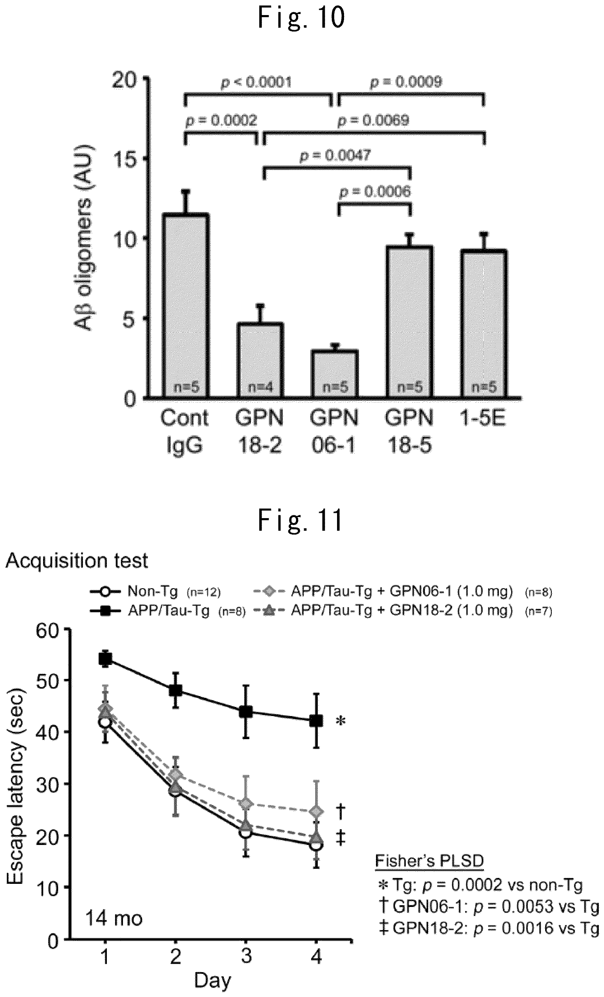

In addition, intracellular Aβ oligomer staining was quantified for the immunostained photographs in Figure 9, and significance difference test was carried out by ANOVA for the three groups using StatView. The results are shown in the graph in Figure 10. Only the data with p-values below 0.05 by Fisher's PLSD were considered "significantly different," and their p-values are indicated in the graph.

The results show that a large number of 11A1 staining-positive cells were observed in the cerebral cortex of the APPosk mice in the control antibody-treated group. On the other hand, the GPN18-2- and GPN06-1-treated groups showed a statistically significant decrease in 11A1 staining-positive cells. The decrease was statistically significant even compared to the GPN18-5- and 1-5E-treated groups. These results indicate that the GPN18-2 antibody, which recognizes the PMEL-CAF region, and the GPN06-1 antibody, which recognizes the PKD region, have stronger medicinal effects in removing Aβ oligomers in the brain than the GPN18-5 antibody, which recognizes the N-terminal region, and the 1-5E antibody, which recognizes the vaccine peptide in the N-terminal region.

These comparative experiments showed that the antibodies recognizing the PMEL-CAF and PKD regions were more effective in removing Aβ oligomers in the brain than the antibodies recognizing the N-terminal region.

### [Reference Example] Production of novel Alzheimer's disease model mice:

A novel mouse model of Alzheimer's disease (Tg2576/Tau264 mice (APP/Tau-Tg)) was produced by crossing Tg2576 mice (Swedish mutant APP transgenic mice), which exhibit amyloid plaques, with Tau264 mice, which expresses human tau (wild type).

Pathological analysis in 9-month-old APP/Tau-Tg showed amyloid plaque formation as detected by an anti-Aβ antibody and accumulation of phosphorylated tau as detected by an AT8 antibody. Appearance of gpNMB-positive microglia was also confirmed by an anti-gpNMB antibody (R & D, goat polyclonal antibody) and an Iba1 antibody, which detects microglia.

### [Example 13] Evaluation of cognitive function improvement by the anti-gpNMB antibody in Alzheimer's disease model mice:

The Alzheimer's disease model mice APP/Tau-Tg aged 14 months described in Reference Example were used for analyzing the cognitive function-improving effect of the anti-gpNMB antibody by Morris water maze test. 14-month-old Non-Tg mice (wild-type mice) born of the same mothers were used as controls for comparison.

### <Group configuration>

*Evaluation antibody group 1: Anti-gpNMB mouse monoclonal antibody GPN06-1, 2.5 mg/mL in 150 mM NaCl buffer (pH 6.5) containing 0.2 M histidine (n = 8)
*Evaluation antibody group 2: Anti-gpNMB mouse monoclonal antibody GPN18-2, 2.5 mg/mL in 150 mM NaCl buffer (pH 6.5) containing 0.2 M histidine (n = 8); the study was started with n = 8, but one animal dropped out before the Morris water maze test, so the data was obtained from n = 7.
*Control antibody group: Anti-BTV mouse monoclonal antibody (Leinco Technologies, Inc.), 2.5 mg/mL in 150 mM NaCl buffer (pH 6.5) containing 0.2 M histidine (n = 9); the study was started with n = 9, but one animal dropped out before the Morris water maze test, so the data was obtained from n = 8.
*Non-Tg group: 150mM NaCl buffer (pH6.5) containing 0.2 M histidine (n=12)

### <Water maze test>

*Preparation: A black pool with an inner diameter of 100 cm and a height of 45 cm was filled with water 16 cm deep. The water temperature was adjusted to 21 to 23°C, and the water was kept clear and colorless. After each trial day, feces were removed, and approximately 10 liters of water was replaced.

*Acquisition test: A 15 cm high transparent platform was sunk 20 cm from the wall (30 cm from the center). The pool was divided into four quadrants, including the area where the platform was sunk, and mice were randomly placed only in one of the three quadrants without the platform. Each trial was limited to 60 seconds and was conducted at a rate of 5 trials/day, with an interval of approximately 5 minutes between trials. The escape time to reach the platform (escape time) was recorded as data. Mice that did not escape within 60 seconds were guided to the platform by an operator, and the escape time was recorded as 60 seconds. Mice on the platform were removed from the pool after 10 seconds, and allowed to act freely and dry their bodies until the next trial. The average seconds of escape time for five trials was used for the daily mouse performance. Trials were conducted for four days.

The acquisition test was finished when the performance of the control group (Non-Tg) stabilized, and a probe test was conducted the next day.
*Probe test: The day after the final day of the acquisition test, the platform was removed from the pool, and a 60-second free swim of each mouse was captured by video camera. The time that the free-swimming mouse spent swimming in the target quadrant of the four quadrants was measured and expressed as a percentage of the 60-second period. The same analysis was performed for swimming up to 30 seconds after entry into the pool.

Significant differences for each test were determined using repeated measure and Fisher's least significant difference test (Fisher's PLSD), and significant differences for the probe test were determined using Fisher's least significant difference test (Fisher's PLSD).

The results of the Acquisition test are shown in the graph in Figure 11. The analysis showed that the control antibody group to APP/Tau-Tg had a significantly longer Escape Latency in the Morris water maze test compared to Non-Tg (wild type). On the other hand, the APP/Tau-Tg group that received anti-gpNMB antibody GPN06-1 or GPN18-2 showed a statistically significant reduction in the time to reach the hidden platform. In the APP/Tau-Tg group treated with the isotype control antibody (Leinco Technologies, Inc.), the time to reach the hidden platform was the same as that in the medium-treated group.

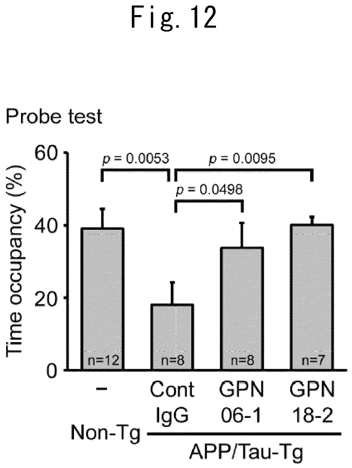

The results of the probe test are shown in the graph in Figure 12. The results of the probe test were similar to those of the acquisition test. Specifically, in the APP/Tau-Tg group treated with the control antibody, the mice spent statistically significantly less time swimming around the quadrant where the hidden platform was present. The mice in the groups treated with GPN06-1 or GPN18-2 spent statistically significantly more time swimming around the quadrant where the hidden platform was located than the control antibody-treated group.

These results indicate that the anti-gpNMB antibodies GPN06-1 and GPN18-2 restore cognitive function in APP/Tau-Tg mice with statistically significant differences.

### [Example 141 Humanization design of mouse anti-gpNMB antibody:

The amino acid sequence of the anti-gpNMB antibody GPN06-1 was analyzed for identifying its CDR regions, in view of IMGT (Lefranc, M.-P., Pommié, C., Ruiz, M., Giudicelli, V, Foulquier, E., Truong, L., Thouvenin-Contet, V. and Lefranc, G. "IMGT unique numbering for immunoglobulin and T cell receptor variable domains and Ig superfamily V-like domains". Dev. Comp. Immunol., 27, 55-77 (2003) PMID: 12477501 LIGM:268.) and Kabat numbering. Combined IMGT/Kabat method was used to keep the CDR loop structure optimal.

### <Heavy chain humanization design>

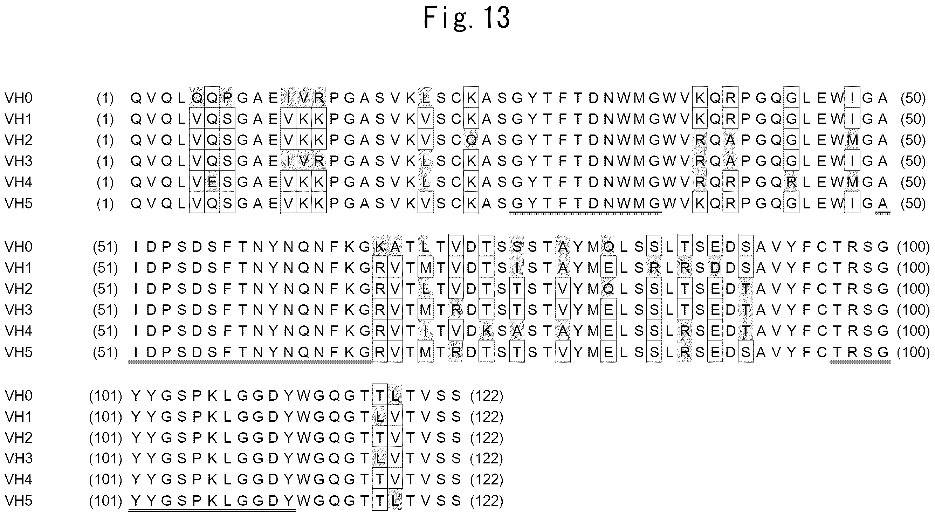

The human germline gene that most closely approximated the amino acid sequence of the mouse heavy chain variable region (VH0) (SEQ ID NO 283) was IGHV1-46*01. BLAST search was used to select 200 candidate human IgG sequences with high homology to VH0. Then, four framework sequences were finally selected in terms of framework homology, retention of key amino acids in the framework, and loop structure. These were subjected to CDR grafting to thereby obtain humanized mutant sequences VH1, VH2, VH3, and VH4. In addition, a sequence designed via CDR-grafting of IGHV1-46*01 as a framework was designated as humanized mutant sequence VH5. The alignment of these humanized mutant sequences VH1 to VH5 to mouse VH0 is shown in Figure 13, in which the identified CDR regions are underlined. Table 9 shows the identity and homology of these humanized mutant sequences VH1 to VH5 to the mouse sequence VH0. The order of identity and homology of the humanized mutant sequences VH1 to VH5 to mouse VH0 was VH3=VH5>VH2>VH1>VH4.

**[Table 9]**

| Table 9: Identity and homology of humanized mutation sequence VH1 to VH5 to mouse sequence VH0 | | |
|---|---|---|
| | Amino acid sequence identity | Amino acid sequence homology |
| VH1 | 86.9% | 92.6% |
| VH2 | 86.9% | 94.3% |
| VH3 | 88.5% | 93.4% |
| VH4 | 85.2% | 92.6% |
| VH5 | 88.5% | 93.4% |

### <Light chain humanized design>

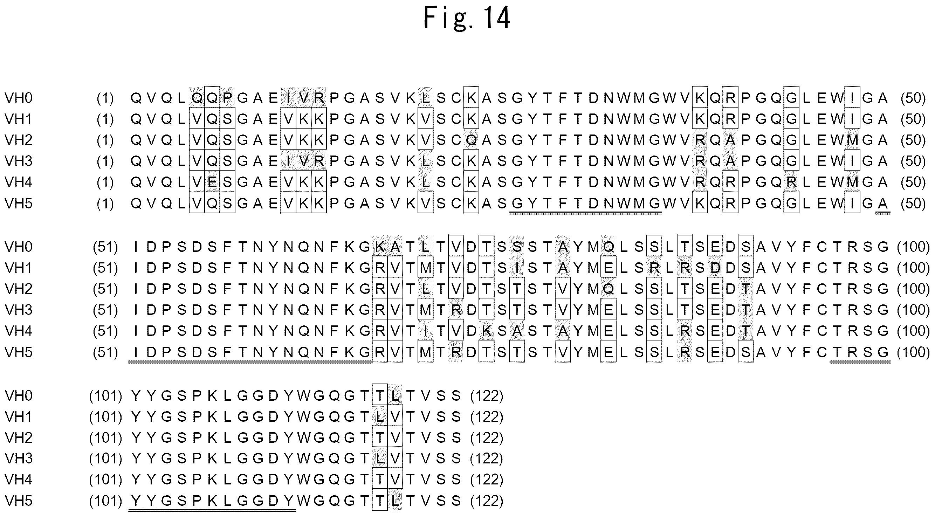

The human germline gene that most closely approximated the amino acid sequence of the mouse light chain variable region (VL0) (SEQ ID NO 285) was IGKV1-9*01. BLAST search was used to select 200 candidate human IgG sequences with high homology to VL0. Then, four framework sequences were finally selected in terms of framework homology, retention of key amino acids in the framework, and loop structure. These were subjected to CDR grafting to thereby obtain humanized mutant sequences VL1, VL2, VL3, and VL4. In addition, a sequence designed via CDR-grafting of IGHV1-9*01 as a framework was designated as humanized mutant sequence VL5. The alignment of these humanized mutant sequences VL1 to VL5 to mouse VL0 is shown in Figure 14, in which the identified CDR regions are underlined. Table 10 shows the identity and homology of these humanized mutant sequences VL1 to VL5 to the mouse sequence VL0. The order of identity and homology of the humanized mutant sequences VL1 to VL5 to mouse VL0 was VL5>VL4>VL1>VL3>VL2.

**[Table 10]**

| Table 10: Identity and homology of humanized mutation sequence VL1 to VL5 to mouse sequence VL0 | | |
|---|---|---|
| | Amino acid sequence identity | Amino acid sequence homology |
| VL1 | 83.0% | 92.5% |
| VL2 | 82.1% | 90.6% |
| VL3 | 82.1% | 91.5% |
| VL4 | 83.0% | 93.4% |
| VL5 | 84.9% | 93.4% |

### [Example 15] Selection of humanized antibodies

The five humanized heavy chain sequences and the five humanized light chain sequences derived from the anti-gpNMB antibody GPN06-1 designed in Example 14 were combined to prepare twenty-five humanized antibodies. These humanized antibodies were evaluated for binding by antigen ELISA to select humanized antibodies whose binding activity was similar to that of human chimeric antibodies.

Specifically, a gene sequence encoding a signal sequence was added to the 5' end of each of the gene sequences encoding the H chains and the L chains of the humanized anti-gpNMB antibodies designed in Example 14, and the resulting sequence was inserted into the animal cell expression vector pcDNA3.4 to form a plasmid, which was transiently expressed using the ExpiCHO Expression System (A29133, Thermo Fisher Scientific) to secrete antibodies into the medium. The culture supernatant was then collected and purified using a Protein A column (Ab-Capture, Protenova) and a gel filtration column (Superdex 200 Increase, Cytiva).

Antigen ELISA was performed by adding each of serially-diluted solutions of the purified humanized antibody to a plate fixed with recombinant human soluble gpNMB (sequence no. 221). The results are shown in Table 11. The results showed that H1L1, H1L2, H2L1, H2L2, H3L1, H3L2, H4L1, H5L2, H5L4, and H5L5 had similar levels of binding ability (i.e., EC₅₀ values were within 3-fold compared to chimeric antibody HOLO).

**[Table 11]**

| Antibody name | EC₅₀ (M) | Relative binding activity |
|---|---|---|
| H0L0 | 3.83E-10 | 1 |
| **H1L1** | **5.75E-10** | **0.92** |
| H1L2 | **5.18E-10** | 0.88 |
| H1L3 | 1.58E-09 | 0.83 |
| H1L4 | 1.64E-09 | 0.86 |
| H1L5 | 2.32E-09 | 0.81 |
| H2L1 | **5.57E-10** | 0.91 |
| H2L2 | **4.44E-10** | 0.89 |
| H2L3 | 3.50E-09 | 0.68 |
| H2L4 | 1.38E-09 | 0.82 |
| H2L5 | 1.15E-09 | 0.79 |
| H3L1 | **6.74E-10** | 0.85 |
| H3L2 | **7.99E-10** | 0.85 |
| H3L3 | 1.48E-09 | 0.7 |
| H3L4 | 1.45E-09 | 0.69 |
| H3L5 | 1.32E-08 | 0.68 |
| H4L1 | **4.31E-10** | 0.87 |
| H4L2 | 5.04E-09 | 0.71 |
| H4L3 | 1.42E-08 | 0.37 |
| H4L4 | 1.69E-08 | 0.49 |
| H4L5 | 1.90E-09 | 0.57 |
| H5L1 | 1.76E-09 | 0.65 |
| H5L2 | **5.94E-10** | 0.84 |
| H5L3 | 1.87E-09 | 0.7 |
| H5L4 | **8.60E-10** | 0.77 |
| H5L5 | **5.00E-10** | 0.78 |

### [Example 161 Interaction analysis of the humanized anti-gpNMB antibodies by surface plasmon resonance method - 3:

Among the humanized GPN06 antibody variants obtained in Example 15, H1L1, H1L2, H1L4, H2L1, H2L2, H3L1, H3L2, H4L1, and H5L2 were compared in terms of the binding properties (binding and dissociation kinetics) to human gpNMB by means of surface plasmon resonance (SPR). Specifically, the same measurement and analysis conditions as in Example 7 were used, except for the conditions described below.

The humanized GPN06 antibody variants H0L0, H1L1, H1L2, H1L4, H2L1, H2L2, H3L1, H3L2, H4L1, and H5L2 were used as analytes. Each analyte at each concentration was reacted for 600 seconds to obtain a binding curve, and HBS-EP+ was reacted for 1200 seconds to obtain a dissociation curve. After the reaction finished, regeneration buffer 1 (0.2% SDS), regeneration buffer 2 (100 mmol/L Tris-HCl (pH 8.5), 1 mol/L NaCl, 15 mmol/L MgCl₂) and regeneration buffer 3 (10 mmol/L glycine -HCl (pH 1.5)) were reacted for 1 minute each to remove and wash gpNMB-FLAG-His in the measurement system. The dissociation rate constant (ka, 1/Ms), binding rate constant (kd, 1/s), and dissociation constant (kd, M) were calculated using BIACORE T200 Evaluation software (ver 2.0) with a Model of 1:1 Binding. The results are shown in Table 12.

**[Table 12]**

| Table 12: Results of SPR kinetics analysis of each anti-gpNMB antibody clone against human gpNMB | | | |
|---|---|---|---|
| Clone | ka (1/Ms) | kd (1/s) | KD (M) |
| H0L0 | 7.89E+05 | 2.09E-04 | 2.65E-10 |
| H1L1 | 9.16E+05 | 5.67E-04 | 6.20E-10 |
| H1L2 | 3.24E+06 | 8.08E-04 | 2.49E-10 |
| H1L4 | 4.98E+05 | 2.13E-04 | 4.28E-10 |
| H2L1 | 4.79E+05 | 3.30E-04 | 6.89E-10 |
| H2L2 | 5.06E+05 | 3.54E-04 | 7.00E-10 |
| H3L1 | 3.51E+06 | 1.54E-03 | 4.38E-10 |
| H3L2 | 9.83E+05 | 6.36E-04 | 6.46E-10 |
| H4L1 | 7.29E+05 | 4.15E-04 | 5.70E-10 |
| H5L2 | 6.02E+05 | 3.54E-04 | 5.87E-10 |

### [Example 17] Identification of amino acids in the CDR regions in humanized antibodies critical for binding by alanine substitution:

Each amino acid in the CDR regions of humanized anti-gpNMB antibody H1L1 produced in Example 15 was substituted with alanine to produce forty-eight CDR substitution mutants of humanized anti-gpNMB antibody H1L1. The binding of these alanine-substituted antibodies to recombinant human soluble gpNMB (SEQ ID NO 221) was evaluated by antigen ELISA to determine which amino acids in the CDR regions are critical for binding. The CDR substitution mutants of the humanized anti-gpNMB antibody H1L1 and their binding activity results are shown in Tables 13, 14-1 and 14-2 below.

The results show that alanine-substitution of the following amino acids in each CDR region caused reduced binding activity and are therefore critical for maintaining the binding ability: in the light chain variable region of the humanized anti-gpNMB antibody H1L1 (SEQ ID NO 31), in CDR-L1 (SEQ ID NO 23), isoleucine at position 29 and tyrosine at position 31; in CDR-L2 (SEQ ID NO 25), threonine at position 50; and in CDR-L3 (SEQ ID NO 27), histidine at position 88, glutamine at position 89, tryptophan at position 90, serine at position 92, and tyrosine at position 93; and in the heavy chain variable region of the humanized anti-gpNMB antibody H1L1 (SEQ ID NO 29), in CDR-H1 (SEQ ID NO 17), asparagine at position 32 and tryptophan at position 33; in CDR-H2 (SEQ ID NO 19), aspartic acid at position 55, phenylalanine at position 57, and threonine at position 58; and in CDR-H3 (SEQ ID NO 21), arginine at position 98, glycine at position 100, and glycine at position 109.

**[Table 13]**

| Table 13: Evaluation results of binding activity of alanine substitutions in the light chain CDR regions | | | | |
|---|---|---|---|---|
| | Alanine substitution position | Amino acid before substitution | EC₅₀ (M) | Relative binding activity |
| H1L1 | - | - | 1.98E-09 | 1.00 |
| CDR-L1 | 27 | S | 1.56E-09 | 0.99 |
| | 28 | S | 1.27E-09 | 1.18 |
| | **29** | **I** | **2.30E-08** | **0.27** |
| | 30 | S | 8.63E-09 | 0.58 |
| | **31** | **Y** | **1.49E-08** | **0.36** |
| CDR-L2 | 49 | S | 7.00E-10 | 0.79 |
| | **50** | **T** | **3.19E-09** | **0.30** |
| | 51 | S | 9.27E-10 | 0.82 |
| | **88** | **H** | **1.47E-07** | **0.08** |
| | **89** | **Q** | **2.75E-08** | **0.06** |
| | **90** | **W** | **1.22E-07** | **0.13** |
| | 91 | N | 1.84E-09 | 0.58 |
| CDR-L3 | **92** | **S** | **1.86E-09** | **0.19** |
| | **93** | **Y** | **2.53E-08** | **0.00** |
| | **94** | **P** | **2.64E-08** | **0.05** |
| | **95** | **C** | **3.15E-09** | **0.45** |
| | 96 | T | 1.03E-09 | 0.83 |

**[Table 14-1]**

| Table 14-1: Evaluation results of binding activity of alanine substitutions in the heavy chain CDR regions | | | | |
|---|---|---|---|---|
| | Alanine substitution position | Amino acid before substitution | EC₅₀ (M) | Relative binding activity |
| H1L1 | - | - | 1.98E-09 | 1.00 |
| CDR-H1 | 26 | G | 1.21E-09 | 1.08 |
| | **27** | **Y** | **6.99E-08** | **0.02** |
| | 28 | T | 1.98E-09 | 1.49 |
| | 29 | F | 4.80E-09 | 0.54 |
| | 30 | T | 2.02E-09 | 1.23 |
| | 31 | D | 5.15E-10 | 1.26 |
| | **32** | **N** | **7.83E-09** | **0.35** |
| | **33** | **W** | **2.81E-08** | **0.06** |
| CDR-H2 | 51 | I | 9.93E-10 | 0.89 |
| | 52 | D | 2.79E-09 | 0.51 |
| | 53 | P | 8.01E-10 | 0.85 |
| | 54 | S | 6.52E-10 | 0.92 |
| | **55** | **D** | **3.15E-08** | **0.00** |
| | 56 | S | 4.29E-10 | 1.14 |
| | **57** | **F** | **3.25E-08** | **0.09** |
| | **58** | **T** | **2.80E-08** | **0.04** |

**[Table 14-2]**

| Table 14-2: Evaluation results of binding activity of alanine substitutions in the heavy chain CDR regions | | | | |
|---|---|---|---|---|
| | Alanine substitution position | Amino acid before substitution | EC₅₀ (M) | Relative binding activity |
| H1L1 | - | - | 1.98E-09 | 1.00 |
| | 97 | T | 3.60E-10 | 0.98 |
| | **98** | **R** | **5.32E-08** | **0.05** |
| | 99 | S | 5.87E-10 | 0.92 |
| | **100** | **G** | **6.58E-08** | **0.06** |
| | 101 | Y | 5.02E-10 | 1.04 |
| | 102 | Y | 2.21E-09 | 0.68 |
| | 103 | G | 7.62E-10 | 1.14 |
| CDR-H3 | 104 | S | 7.12E-10 | 0.88 |
| | 105 | P | 1.18E-09 | 1.09 |
| | 106 | K | 4.41E-10 | 1.00 |
| | 107 | L | 1.05E-09 | 0.71 |
| | 108 | G | 4.76E-10 | 0.96 |
| | **109** | **G** | **4.89E-09** | **0.34** |
| | 110 | D | 9.56E-10 | 0.79 |
| | 111 | Y | 5.84E-10 | 0.99 |

### INDUSTRIAL APPLICABILITY

The present invention provides an anti-gpNMB antibody that can remove mal-functional microglia and/or removing amyloid β oligomers, which are toxic proteins, and/or restore the number of synapses and/or restore cognitive functions, by specifically binding to mammalian microglia expressing gpNMB. Therefore, the present invention can be used for, e.g., the treatment, prevention, or diagnosis of neurodegenerative diseases associated with microglia, such as Alzheimer's disease.

## Claims

1. An anti-gpNMB antibody or its fragment or a derivative thereof which binds specifically to at least one site in a region from a PMEL-CAF-like (PMEL core amyloid fragment-like) domain to a PKD domain of human gpNMB (glycoprotein nonmetastatic melanoma protein B).

2. The anti-gpNMB antibody or its fragment or a derivative thereof according to claim 1, which binds specifically to a region containing amino acid residue(s) D287 and/or H301 of human gpNMB having the amino acid sequence defined in SEQ ID NO 209.

3. The anti-gpNMB antibody or its fragment or a derivative thereof according to claim 1 or 2, which binds specifically to a region containing amino acid residue(s) R214 and/or R215 of human gpNMB having the amino acid sequence defined in SEQ ID NO 209.

4. The anti-gpNMB antibody or its fragment or a derivative thereof according to claim 2 or 3, which also binds specifically to one or more regions selected from of a region containing amino acid residue(s) K257 and/or D258, a region containing amino acid residue(s) H268 and/or D269, a region containing an amino acid residue K282, a region containing an amino acid residue K316, and a region containing amino acid residue(s) H216 and/or R218, of human gpNMB having the amino acid sequence defined in SEQ ID NO 209.

5. The anti-gpNMB antibody or its fragment or a derivative thereof according to any one of claims 1 to 4, which also binds specifically to at least one site in a region from a PMEL-CAF-like domain to a PKD domain of mouse gpNMB.

6. The anti-gpNMB antibody or its fragment or a derivative thereof according to any one of claims 1 to 5, having one or more activities selected from: an activity to reduce the number of mal-functional microglia; an activity to remove amyloid beta oligomers; an activity to increase the number of synapses; and an activity to restore cognitive function.

7. The anti-gpNMB antibody or its fragment or a derivative thereof according to any one of claims 1 to 6, which is a monoclonal antibody or its fragment or a derivative thereof.

8. The anti-gpNMB antibody or its fragment or a derivative thereof according to any one of claims 1 to 7, which comprises at least a heavy chain variable region comprising:
(1) as a CDR-H1 sequence, the amino acid sequence defined in SEQ ID NO 1, or an amino acid sequence derived from SEQ ID NO 1 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 1,
as a CDR-H2 sequence, the amino acid sequence defined in SEQ ID NO 3, or an amino acid sequence derived from SEQ ID NO 3 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 3, and
as a CDR-H3 sequence, the amino acid sequence defined in SEQ ID NO 5, or an amino acid sequence derived from SEQ ID NO 5 via substitution, deletion, or insertion of any one, two, three, or four amino acid residues, or an amino acid sequence having a homology (preferably identity) of 66.6 % or higher, 75.0 % or higher, 83.3 % or higher, or 91.6 % or higher to SEQ ID NO 5, or
(2) as a CDR-H1 sequence, the amino acid sequence defined in SEQ ID NO 17, or an amino acid sequence derived from SEQ ID NO 17 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 17,
as a CDR-H2 sequence, the amino acid sequence defined in SEQ ID NO 19, or an amino acid sequence derived from SEQ ID NO 19 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 19, and
as a CDR-H3 sequence, the amino acid sequence defined in SEQ ID NO 21, or an amino acid sequence derived from SEQ ID NO 21 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 21, or
(3) as a CDR-H1 sequence, the amino acid sequence defined in SEQ ID NO 33, or an amino acid sequence derived from SEQ ID NO 33 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 33,
as a CDR-H2 sequence, the amino acid sequence defined in SEQ ID NO 35, or an amino acid sequence derived from SEQ ID NO 35 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 35, and
as a CDR-H3 sequence, the amino acid sequence defined in SEQ ID NO 37, or an amino acid sequence derived from SEQ ID NO 37 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 37, or
(4) as a CDR-H1 sequence, the amino acid sequence defined in SEQ ID NO 49, or an amino acid sequence derived from SEQ ID NO 49 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 49,
as a CDR-H2 sequence, the amino acid sequence defined in SEQ ID NO 51, or an amino acid sequence derived from SEQ ID NO 51 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 51, and
as a CDR-H3 sequence, the amino acid sequence defined in SEQ ID NO 53, or an amino acid sequence derived from SEQ ID NO 53 via substitution, deletion, or insertion of any one, two, three, four, five, or six amino acid residues, or an amino acid sequence having a homology (preferably identity) of 62.5 % or higher, 68.7 % or higher, 75.0 % or higher, 81.2 % or higher, 87.5 % or higher, or 93.7 % or higher to SEQ ID NO 53, or
(5) as a CDR-H1 sequence, the amino acid sequence defined in SEQ ID NO 65, or an amino acid sequence derived from SEQ ID NO 65 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 65,
as a CDR-H2 sequence, the amino acid sequence defined in SEQ ID NO 67, or an amino acid sequence derived from SEQ ID NO 67 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 67, and
as a CDR-H3 sequence, the amino acid sequence defined in SEQ ID NO 69, or an amino acid sequence derived from SEQ ID NO 69 via substitution, deletion, or insertion of any one, two, three, four, or five amino acid residues, or an amino acid sequence having a homology (preferably identity) of 64.2 % or higher, 71.4 % or higher, 78.5 % or higher, 85.7 % or higher, or 92.8 % or higher to SEQ ID NO 69, or
(6) as a CDR-H1 sequence, the amino acid sequence defined in SEQ ID NO 81, or an amino acid sequence derived from SEQ ID NO 81 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 81,
as a CDR-H2 sequence, the amino acid sequence defined in SEQ ID NO 83, or an amino acid sequence derived from SEQ ID NO 83 via substitution, deletion, or insertion of any one or two amino acid residues, or an amino acid sequence having a homology (preferably identity) of 71.4 % or higher or 85.7 % or higher to SEQ ID NO 83, and
as a CDR-H3 sequence, the amino acid sequence defined in SEQ ID NO 85, or an amino acid sequence derived from SEQ ID NO 85 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 85, or
(7) as a CDR-H1 sequence, the amino acid sequence defined in SEQ ID NO 97, or an amino acid sequence derived from SEQ ID NO 97 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 97,
as a CDR-H2 sequence, the amino acid sequence defined in SEQ ID NO 99, or an amino acid sequence derived from SEQ ID NO 99 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 99, and
as a CDR-H3 sequence, the amino acid sequence defined in SEQ ID NO 101, or an amino acid sequence derived from SEQ ID NO 101 via substitution, deletion, or insertion of any one or two amino acid residues, or an amino acid sequence having a homology (preferably identity) of 60.0 % or higher or 80.0 % or higher to SEQ ID NO 101, or
(8) as a CDR-H1 sequence, the amino acid sequence defined in SEQ ID NO 113, or an amino acid sequence derived from SEQ ID NO 113 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 113,
as a CDR-H2 sequence, the amino acid sequence defined in SEQ ID NO 115, or an amino acid sequence derived from SEQ ID NO 115 via substitution, deletion, or insertion of any one or two amino acid residues, or an amino acid sequence having a homology (preferably identity) of 71.4 % or higher or 85.7 % or higher to SEQ ID NO 115, and
as a CDR-H3 sequence, the amino acid sequence defined in SEQ ID NO 117, or an amino acid sequence derived from SEQ ID NO 117 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 117, or
(9) as a CDR-H1 sequence, the amino acid sequence defined in SEQ ID NO 129, or an amino acid sequence derived from SEQ ID NO 129 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 129,
as a CDR-H2 sequence, the amino acid sequence defined in SEQ ID NO 131, or an amino acid sequence derived from SEQ ID NO 131 via substitution, deletion, or insertion of any one or two amino acid residues, or an amino acid sequence having a homology (preferably identity) of 71.4 % or higher or 85.7 % or higher to SEQ ID NO 131, and
as a CDR-H3 sequence, the amino acid sequence defined in SEQ ID NO 133, or an amino acid sequence derived from SEQ ID NO 133 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 133, or
(10) as a CDR-H1 sequence, the amino acid sequence defined in SEQ ID NO 145, or an amino acid sequence derived from SEQ ID NO 145 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 145,
as a CDR-H2 sequence, the amino acid sequence defined in SEQ ID NO 147, or an amino acid sequence derived from SEQ ID NO 147 via substitution, deletion, or insertion of any one, two, or three amino acid residues, or an amino acid sequence having a homology (preferably identity) of 62.5 % or higher, 75.0 % or higher, or 87.5 % or higher to SEQ ID NO 147, and
as a CDR-H3 sequence, the amino acid sequence defined in SEQ ID NO 149, or an amino acid sequence derived from SEQ ID NO 149 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 149, or
(11) as a CDR-H1 sequence, the amino acid sequence defined in SEQ ID NO 161, or an amino acid sequence derived from SEQ ID NO 161 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 161,
as a CDR-H2 sequence, the amino acid sequence defined in SEQ ID NO 163, or an amino acid sequence derived from SEQ ID NO 163 via substitution, deletion, or insertion of any one or two amino acid residues, or an amino acid sequence having a homology (preferably identity) of 71.4 % or higher or 85.7 % or higher to SEQ ID NO 163, and
as a CDR-H3 sequence, the amino acid sequence defined in SEQ ID NO 165, or an amino acid sequence derived from SEQ ID NO 165 via substitution, deletion, or insertion of any one, two, three, four, five, or six amino acid residues, or an amino acid sequence having a homology (preferably identity) of 60.0 % or higher, 66.6 % or higher, 73.3 % or higher, 80.0 % or higher, 86.6 % or higher, or 93.3 % or higher to SEQ ID NO 165,
(12) as a CDR-H1 sequence, the amino acid sequence defined in SEQ ID NO 287, or an amino acid sequence derived from SEQ ID NO 287 via substitution, deletion, or insertion of any one, two, or three amino acid residues, or an amino acid sequence having a homology (preferably identity) of 62.5 % or higher, 75.0 % or higher, 87.5 % or higher, or 90.0 % or higher to SEQ ID NO 287,
as a CDR-H2 sequence, the amino acid sequence defined in SEQ ID NO 289, or an amino acid sequence derived from SEQ ID NO 289 via substitution, deletion, or insertion of any one or two amino acid residues, or an amino acid sequence having a homology (preferably identity) of 71.4 % or higher or 85.7 % or higher to SEQ ID NO 289, and
as a CDR-H3 sequence, the amino acid sequence defined in SEQ ID NO 291, or an amino acid sequence derived from SEQ ID NO 291 via substitution, deletion, or insertion of any one or two amino acid residues, or an amino acid sequence having a homology (preferably identity) of 66.6 % or higher or 83.3 % or higher to SEQ ID NO 291, or
(13) as a CDR-H1 sequence, the amino acid sequence defined in SEQ ID NO 17, or an amino acid sequence derived from SEQ ID NO 17 via substitution, deletion, or insertion of any one, two, three, four, five, or six amino acid residues other than asparagine at position 32 and tryptophan at position 33,
as a CDR-H2 sequence, the amino acid sequence defined in SEQ ID NO 19, or an amino acid sequence derived from SEQ ID NO 19 via substitution, deletion, or insertion of any one, two, three, four, or five amino acid residues other than aspartic acid at position 55, phenylalanine at position 57, and threonine at position 58, and
as a CDR-H3 sequence, the amino acid sequence defined in SEQ ID NO 21, or an amino acid sequence derived from SEQ ID NO 21 via substitution, deletion, or insertion of any one, two, three, four, five, six, seven, eight, nine, ten, eleven, or twelve amino acid residues arginine at position 98, glycine at position 100, and glycine at position 109.

9. The anti-gpNMB antibody or its fragment or a derivative thereof according to any one of claims 1 to 7, which comprises at least a heavy chain variable region comprising:
(1) the amino acid sequence defined in SEQ ID NO 13, or an amino acid sequence having a homology of 60 % or higher to SEQ ID NO 13, or
(2) the amino acid sequence defined in SEQ ID NO 29, or an amino acid sequence having a homology of 60 % or higher to SEQ ID NO 29, or
(3) the amino acid sequence defined in SEQ ID NO 45, or an amino acid sequence having a homology of 60 % or higher to SEQ ID NO 45, or
(4) the amino acid sequence defined in SEQ ID NO 61, or an amino acid sequence having a homology of 60 % or higher to SEQ ID NO 61, or
(5) the amino acid sequence defined in SEQ ID NO 77, or an amino acid sequence having a homology of 60 % or higher to SEQ ID NO 77, or
(6) the amino acid sequence defined in SEQ ID NO 93, or an amino acid sequence having a homology of 60 % or higher to SEQ ID NO 93, or
(7) the amino acid sequence defined in SEQ ID NO 109, or an amino acid sequence having a homology of 60 % or higher to SEQ ID NO 109, or
(8) the amino acid sequence defined in SEQ ID NO 125, or an amino acid sequence having a homology of 60 % or higher to SEQ ID NO 125, or
(9) the amino acid sequence defined in SEQ ID NO 141, or an amino acid sequence having a homology of 60 % or higher to SEQ ID NO 141, or
(10) the amino acid sequence defined in SEQ ID NO 157, or an amino acid sequence having a homology of 60 % or higher to SEQ ID NO 157, or
(11) the amino acid sequence defined in SEQ ID NO 173, or an amino acid sequence having a homology of 60 % or higher to SEQ ID NO 173, or
(12) the amino acid sequence defined in SEQ ID NO 299, or an amino acid sequence having a homology of 60 % or higher to SEQ ID NO 299.

10. The anti-gpNMB antibody or its fragment or a derivative thereof according to claim 8 or 9, wherein the heavy chain variable region comprises, as a framework sequence, a framework sequence of any class of human immunoglobulin.

11. The anti-gpNMB antibody or its fragment or a derivative thereof according to any one of claims 8 to 10, which further comprises a heavy chain constant region having an amino acid sequence of a heavy chain constant region of any class of human immunoglobulin.

12. The anti-gpNMB antibody or its fragment or a derivative thereof according to any one of claims 1 to 11, which comprises at least a light chain variable region comprising:
(1) as a CDR-L1, the amino acid sequence defined in SEQ ID NO 7, or an amino acid sequence derived from SEQ ID NO 7 via substitution, deletion, or insertion of any one or two amino acid residues, or an amino acid sequence having a homology (preferably identity) of 66.6 % or higher or 83.3 % or higher to SEQ ID NO 7,
as a CDR-L2, the amino acid sequence defined in SEQ ID NO 9, or an amino acid sequence derived from SEQ ID NO 9 via substitution, deletion, or insertion of any one amino acid residue, or an amino acid sequence having a homology (preferably identity) of 66.6 % or higher to SEQ ID NO 9, and
as a CDR-L3, the amino acid sequence defined in SEQ ID NO 11, or an amino acid sequence derived from SEQ ID NO 11 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 11, or
(2) as a CDR-L1, the amino acid sequence defined in SEQ ID NO 23, or an amino acid sequence derived from SEQ ID NO 23 via substitution, deletion, or insertion of any one or two amino acid residues, or an amino acid sequence having a homology (preferably identity) of 60.0 % or higher or 80.0 % or higher to SEQ ID NO 23,
as a CDR-L2, the amino acid sequence defined in SEQ ID NO 25, or an amino acid sequence derived from SEQ ID NO 25 via substitution, deletion, or insertion of any one amino acid residue, or an amino acid sequence having a homology (preferably identity) of 66.6 % or higher to SEQ ID NO 25, and
as a CDR-L3, the amino acid sequence defined in SEQ ID NO 27, or an amino acid sequence derived from SEQ ID NO 27 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 27, or
(3) as a CDR-L1, the amino acid sequence defined in SEQ ID NO 39, or an amino acid sequence derived from SEQ ID NO 39 via substitution, deletion, or insertion of any one or two amino acid residues, or an amino acid sequence having a homology (preferably identity) of 60.0 % or higher or 80.0 % or higher to SEQ ID NO 39,
as a CDR-L2, the amino acid sequence defined in SEQ ID NO 41, or an amino acid sequence derived from SEQ ID NO 41 via substitution, deletion, or insertion of any one amino acid residue, or an amino acid sequence having a homology (preferably identity) of 66.6 % or higher to SEQ ID NO 41, and
as a CDR-L3, the amino acid sequence defined in SEQ ID NO 43, or an amino acid sequence derived from SEQ ID NO 43 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 43, or
(4) as a CDR-L1, the amino acid sequence defined in SEQ ID NO 55, or an amino acid sequence derived from SEQ ID NO 55 via substitution, deletion, or insertion of any one or two amino acid residues, or an amino acid sequence having a homology (preferably identity) of 66.6 % or higher or 83.3 % or higher to SEQ ID NO 55,
as a CDR-L2, the amino acid sequence defined in SEQ ID NO 57, or an amino acid sequence derived from SEQ ID NO 57 via substitution, deletion, or insertion of any one amino acid residue, or an amino acid sequence having a homology (preferably identity) of 66.6 % or higher to SEQ ID NO 57, and
as a CDR-L3, the amino acid sequence defined in SEQ ID NO 59, or an amino acid sequence derived from SEQ ID NO 59 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 59, or
(5) as a CDR-L1, the amino acid sequence defined in SEQ ID NO 71, or an amino acid sequence derived from SEQ ID NO 71 via substitution, deletion, or insertion of any one or two amino acid residues, or an amino acid sequence having a homology (preferably identity) of 60.0 % or higher or 80.0 % or higher to SEQ ID NO 71,
as a CDR-L2, the amino acid sequence defined in SEQ ID NO 73, or an amino acid sequence derived from SEQ ID NO 73 via substitution, deletion, or insertion of any one amino acid residue, or an amino acid sequence having a homology (preferably identity) of 66.6 % or higher to SEQ ID NO 73, and
as a CDR-L3, the amino acid sequence defined in SEQ ID NO 75, or an amino acid sequence derived from SEQ ID NO 75 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 75, or
(6) as a CDR-L1, the amino acid sequence defined in SEQ ID NO 87, or an amino acid sequence derived from SEQ ID NO 87 via substitution, deletion, or insertion of any one or two amino acid residues, or an amino acid sequence having a homology (preferably identity) of 66.6 % or higher or 83.3 % or higher to SEQ ID NO 87,
as a CDR-L2, the amino acid sequence defined in SEQ ID NO 89, or an amino acid sequence derived from SEQ ID NO 89 via substitution, deletion, or insertion of any one amino acid residue, or an amino acid sequence having a homology (preferably identity) of 66.6 % or higher to SEQ ID NO 89, and
as a CDR-L3, the amino acid sequence defined in SEQ ID NO 91, or an amino acid sequence derived from SEQ ID NO 91 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 91, or
(7) as a CDR-L1, the amino acid sequence defined in SEQ ID NO 103, or an amino acid sequence derived from SEQ ID NO 103 via substitution, deletion, or insertion of any one or two amino acid residues, or an amino acid sequence having a homology (preferably identity) of 66.6 % or higher or 83.3 % or higher to SEQ ID NO 103,
as a CDR-L2, the amino acid sequence defined in SEQ ID NO 105, or an amino acid sequence derived from SEQ ID NO 105 via substitution, deletion, or insertion of any one amino acid residue, or an amino acid sequence having a homology (preferably identity) of 66.6 % or higher to SEQ ID NO 105, and
as a CDR-L3, the amino acid sequence defined in SEQ ID NO 107, or an amino acid sequence derived from SEQ ID NO 107 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 107, or
(8) as a CDR-L1, the amino acid sequence defined in SEQ ID NO 119, or an amino acid sequence derived from SEQ ID NO 119 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 119,
as a CDR-L2, the amino acid sequence defined in SEQ ID NO 121, or an amino acid sequence derived from SEQ ID NO 121 via substitution, deletion, or insertion of any one amino acid residue, or an amino acid sequence having a homology (preferably identity) of 66.6 % or higher to SEQ ID NO 121, and
as a CDR-L3, the amino acid sequence defined in SEQ ID NO 123, or an amino acid sequence derived from SEQ ID NO 123 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 123, or
(9) as a CDR-L1, the amino acid sequence defined in SEQ ID NO 135, or an amino acid sequence derived from SEQ ID NO 135 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 135,
as a CDR-L2, the amino acid sequence defined in SEQ ID NO 137, or an amino acid sequence derived from SEQ ID NO 137 via substitution, deletion, or insertion of any one amino acid residue, or an amino acid sequence having a homology (preferably identity) of 66.6 % or higher to SEQ ID NO 137, and
as a CDR-L3, the amino acid sequence defined in SEQ ID NO 139, or an amino acid sequence derived from SEQ ID NO 139 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 139, or
(10) as a CDR-L1, the amino acid sequence defined in SEQ ID NO 151, or an amino acid sequence derived from SEQ ID NO 151 via substitution, deletion, or insertion of any one, two, three, or four amino acid residues, or an amino acid sequence having a homology (preferably identity) of 60.0 % or higher, 70.0 % or higher, 80.0 % or higher, or 90.0 % or higher to SEQ ID NO 151,
as a CDR-L2, the amino acid sequence defined in SEQ ID NO 153, or an amino acid sequence derived from SEQ ID NO 153 via substitution, deletion, or insertion of any one amino acid residue, or an amino acid sequence having a homology (preferably identity) of 66.6 % or higher to SEQ ID NO 153, and
as a CDR-L3, the amino acid sequence defined in SEQ ID NO 155, or an amino acid sequence derived from SEQ ID NO 155 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 155, or
(11) as a CDR-L1, the amino acid sequence defined in SEQ ID NO 167, or an amino acid sequence derived from SEQ ID NO 167 via substitution, deletion, or insertion of any one, two, three, or four amino acid residues, or an amino acid sequence having a homology (preferably identity) of 64.2 % or higher, 71.4 % or higher, 81.8 % or higher, or 90.9 % or higher to SEQ ID NO 167,
as a CDR-L2, the amino acid sequence defined in SEQ ID NO 169, or an amino acid sequence derived from SEQ ID NO 169 via substitution, deletion, or insertion of any one amino acid residue, or an amino acid sequence having a homology (preferably identity) of 66.6 % or higher to SEQ ID NO 169, and
as a CDR-L3, the amino acid sequence defined in SEQ ID NO 171, or an amino acid sequence derived from SEQ ID NO 171 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 171, or
(12) as a CDR-L1, the amino acid sequence defined in SEQ ID NO 293, or an amino acid sequence derived from SEQ ID NO 293 via substitution, deletion, or insertion of any one, two, three, or four amino acid residues, or an amino acid sequence having a homology (preferably identity) of 64.2 % or higher, 71.4 % or higher, 81.8 % or higher or 90.9 % or higher to SEQ ID NO 293,
as a CDR-L2, the amino acid sequence defined in SEQ ID NO 295, or an amino acid sequence derived from SEQ ID NO 295 via substitution, deletion, or insertion of any one amino acid residue, or an amino acid sequence having a homology (preferably identity) of 66.6 % or higher to SEQ ID NO 295, and
as a CDR-L3, the amino acid sequence defined in SEQ ID NO 297, or an amino acid sequence derived from SEQ ID NO 297 via substitution, deletion, or insertion of any one, two, or three amino acid residues, or an amino acid sequence having a homology (preferably identity) of 66.6 % or higher, 77.7 % or higher, or 88.8 % or higher to SEQ ID NO 297, or
(13) as a CDR-L1, the amino acid sequence defined in SEQ ID NO 23, or an amino acid sequence derived from SEQ ID NO 23 via substitution, deletion, or insertion of any one, two, or three amino acid residues other than isoleucine at position 29 and tyrosine at position 31,
as a CDR-L2, the amino acid sequence defined in SEQ ID NO 25, or an amino acid sequence derived from SEQ ID NO 25 via substitution, deletion, or insertion of any one or two amino acid residues other than threonine at No. 50, and
as a CDR-L3, the amino acid sequence defined in SEQ ID NO 27, or an amino acid sequence derived from SEQ ID NO 27 via substitution, deletion, or insertion of any one, two, three, or four amino acid residues other than histidine at position 88, glutamine at position 89, tryptophan at position 90, serine at position 92, and tyrosine at position 93.

13. The anti-gpNMB antibody or its fragment or a derivative thereof according to any one of claims 1 to 11, which comprises at least a light chain variable region comprising:
(1) the amino acid sequence defined in SEQ ID NO 15, or an amino acid sequence having a homology of 60 % or higher to SEQ ID NO 15, or
(2) the amino acid sequence defined in SEQ ID NO 31, or an amino acid sequence having a homology of 60 % or higher to SEQ ID NO 31, or
(3) the amino acid sequence defined in SEQ ID NO 47, or an amino acid sequence having a homology of 60 % or higher to SEQ ID NO 47, or
(4) the amino acid sequence defined in SEQ ID NO 63, or an amino acid sequence having a homology of 60 % or higher to SEQ ID NO 63, or
(5) the amino acid sequence defined in SEQ ID NO 79, or an amino acid sequence having a homology of 60 % or higher to SEQ ID NO 79, or
(6) the amino acid sequence defined in SEQ ID NO 95, or an amino acid sequence having a homology of 60 % or higher to SEQ ID NO 95, or
(7) the amino acid sequence defined in SEQ ID NO 111, or an amino acid sequence having a homology of 60 % or higher to SEQ ID NO 111, or
(8) the amino acid sequence defined in SEQ ID NO 127, or an amino acid sequence having a homology of 60 % or higher to SEQ ID NO 127, or
(9) the amino acid sequence defined in SEQ ID NO 143, or an amino acid sequence having a homology of 60 % or higher to SEQ ID NO 143, or
(10) the amino acid sequence defined in SEQ ID NO 159, or an amino acid sequence having a homology of 60 % or higher to SEQ ID NO 159, or
(11) the amino acid sequence defined in SEQ ID NO 175, or an amino acid sequence having a homology of 60 % or higher to SEQ ID NO 175, or
(12) the amino acid sequence defined in SEQ ID NO 301, or an amino acid sequence having a homology of 60 % or higher to SEQ ID NO 301.

14. The anti-gpNMB antibody or its fragment or a derivative thereof according to claim 12 or 13, wherein the light chain variable region comprises, as a framework sequence, a framework sequence of any class of human immunoglobulin.

15. The anti-gpNMB antibody or its fragment or a derivative thereof according to any one of claims 12 to 14, which further comprises a light chain constant region having an amino acid sequence of a light chain constant region of any class of human immunoglobulin.

16. The anti-gpNMB antibody or its fragment or a derivative thereof according to any one of claims 1 to 15, which is a Fab, scFv, Diabody, Nanobody, VHH, bispecific antibody, or multispecific antibody, or a derivative thereof.

17. An anti-gpNMB antibody or its fragment or a derivative thereof which binds to at least one site in a region from a PMEL-CAF-like domain to a PKD domain competitively with an anti-gpNMB antibody or its fragment or a derivative thereof according to any one of claims 1 to 16.

18. A nucleic acid molecule comprising a polynucleotide sequence encoding an anti-gpNMB antibody or its fragment or a derivative thereof according to any one of claims 1 to 17.

19. A cloning vector or expression vector carrying at least one nucleic acid molecule according to claim 18.

20. A recombinant cell transformed with a vector according to claim 19.

21. A method for producing an anti-gpNMB antibody or its fragment or a derivative thereof according to any one of claims 1 to 17, comprising culturing a recombinant cell according to claim 20.

22. A method for producing an anti-gpNMB antibody or its fragment or a derivative thereof according to any one of claims 1 to 17, comprising: administering to an animal a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209; and collecting an antibody or its fragment or a derivative thereof produced in the body of the animal.

23. A vaccine having an activity to stimulate the production of an anti-gpNMB antibody or its fragment or a derivative thereof according to any one of claims 1 to 17, comprising a polypeptide having the same amino acid sequence as a region containing amino acid residue(s) D287 and/or H301 and /or a region containing amino acid residue(s) R214 and /or R215 of human gpNMB having the amino acid sequence defined in SEQ ID NO 209.

24. A pharmaceutical composition comprising, as an active ingredient, one or more selected from the group consisting of an anti-gpNMB antibody or its fragment or a derivative thereof according to any one of claims 1 to 17, a nucleic acid molecule according to claim 18, a vector according to claim 19, and a recombinant cell according to claim 20.

25. The pharmaceutical composition according to claim 24, for use in decreasing the number of mal-functional microglia in a subject and/or removing amyloid β oligomers in a subject and/or increasing the number of synapses in a subject and/or treating or preventing neurodegenerative disease in a subject.

26. A pharmaceutical composition for use in removing amyloid β oligomers in a subject and/or increasing the number of synapses in a subject and/or treating or preventing neurodegenerative disease in a subject, comprising, as an active ingredient, an agent for decreasing the number of mal-functional microglia.

27. The pharmaceutical composition according to claim 25 or 26, wherein the neurodegenerative disease is Alzheimer's disease.

28. The pharmaceutical composition according to claim 27, further comprising a second active ingredient.

29. The pharmaceutical composition according to claim 28, wherein the second active ingredient is one or more selected from an anti-Tau antibody, anti-amyloid β antibody, anti-CD33 antibody, anti-semaphorin 4D antibody, anti-TNFα antibody, anti-sortilin antibody, anti-galactose-specific lectin (galectin) 3 antibody, and anti-TREM2 (triggering receptor expressed on myeloid cells 2) antibody.

30. The pharmaceutical composition according to claim 28, wherein the second active ingredient is a vaccine comprising a full-length or partial-length polypeptide of one or more proteins selected from Tau, amyloid β, CD33, semaphorin 4D, TNFα, sortilin, galactose-specific lectin (galectin) 3, and TREM2 (triggering receptor expressed on myeloid cells 2), or a nucleic acid encoding the polypeptide.
